# EUROPEAN PATENT APPLICATION

(11) **EP 4 324 478 A2**
(43) Date of publication of application: **21.02.2024**
(21) Application number: 23199107.6
(22) Date of filing: 10.03.2017
(51) Int. Cl.: A61K 39/12

(54) **EPITOPE MIMICS**

(30) Priority: 10.03.2016 US 201662306262 P
(62) Divisional of application: 17764183.4
(71) Applicant: Iogenetics, LLC., Madison, Wisconsin 53704 (US)
(72) Inventor: BREMEL, Robert D., Madison, Wisconsin (US); HOMAN, Jane, Madison, Wisconsin (US)
(74) Representative: Algemeen Octrooi- en Merkenbureau B.V.

(57) **Abstract**

This invention pertains to the identification of antibody mediated epitope mimics and applications of the identification of said mimic peptides in the design of biotherapeutics and vaccines.

## Description

### FIELD OF THE INVENTION

This invention pertains to the identification of antibody mediated epitope mimics and applications of the identification of said mimic peptides in the design of biotherapeutics and vaccines.

### BACKGROUND OF THE INVENTION

Autoimmune disease affects up to 50 million Americans, according to the American Autoimmune Related Diseases Association (AARDA). An autoimmune disease develops when the immune system, which defends the body against disease, decides that healthy self cells are foreign. As a result, the immune system attacks healthy cells. Depending on the type, an autoimmune disease can affect one or many different types of body tissue. It can also cause abnormal organ growth and changes in organ function.

There are as many as 80 types of autoimmune diseases documented. Many of them have similar symptoms, which makes them very difficult to diagnose. It is also possible to have more than one at the same time. Autoimmune diseases usually fluctuate between periods of remission (little or no symptoms) and flare-ups (worsening symptoms). Currently, treatment for autoimmune diseases focuses on relieving symptoms because there is no curative therapy. In some instances, onset of an autoimmune disease may be triggered by exposure of a subject to an infectious microorganism, an allergen, or other exogenous protein.

Autoimmune diseases often run in families, and 75 percent of those affected are women, according to AARDA. African Americans, Hispanics, and Native Americans also have an increased risk of developing an autoimmune disease.

It is also increasingly apparent that autoimmune mechanisms play a significant contributing role in the pathogenesis of many acute diseases, and in particular, infectious diseases, which are not generally thought of or characterized as autoimmune diseases. Indeed, the vast majority of clinical diseases may contain some autoimmune components to their pathogenesis.

As the human proteome differs in sequence from many species which are routinely used as experimental animal models, the occurrence of autoimmune phenomena varies between host species. This may result in disease observed in animal models diverging from that in the human host.

What is needed in the art are improved methods for determining which epitopes may give rise to autoimmune diseases and whether biotherapeutics and vaccines contain epitopes which can trigger autoimmune diseases. Furthermore, the art needs to better understand the autoimmune pathogenesis arising from infectious agents in order to facilitate the design of safe interventions, and in order to select appropriate animal models.

### SUMMARY OF THE INVENTION

This invention pertains to the identification of antibody mediated epitope mimics and applications of the identification of said mimic peptides in the design of biotherapeutics and vaccines.

In some embodiments, the present invention provides methods for identifying epitope mimic peptides which elicit antibodies that bind to a host protein, comprising: assembling a database of all proteins in the host proteome; assigning a curation to each protein based on its reported function; computing the probable B cell epitopes in each protein of the host proteome database wherein the proteins are curated by function; identifying the core peptide of the probable B cell epitopes in each protein of the host proteome; assembling a database of the core peptides of the probable B cell epitopes from each protein of the host proteome in a computer readable medium; entering a sequence of a protein of interest into a computer with access to the database; computing probable B cell epitopes in the protein of interest; identifying the core peptide of the probable B cell epitopes in the protein of interest; comparing the core peptide of the probable B cell epitope in a protein of interest to the core peptides contained in the database of peptides from the host proteome; identifying core peptides in predicted B cell epitopes in the protein of interest which are identical to core peptides in predicted B cell epitopes in one or more proteins of the host proteome; and identifying the function of the host proteome proteins which comprise the identical core peptides matching the core peptides of the protein of interest.

In some embodiments, the host proteome is a human proteome. In other embodiments the host proteome is a murine proteome. In yet other embodiments the host protein is from another species, including but not limited to a non-human primate proteome.

In some embodiments, the probable B cell epitope in the protein of interest is in the top 25% most probable B cell epitopes in the protein of interest. In some embodiments, the probable B cell epitope in the protein of interest is in the top 10% most probable B cell epitopes in the protein of interest. In some embodiments, the probable B cell epitope in the host proteome protein is in the top 40% most probable B cell epitopes in the protein of interest. In some embodiments, the probable B cell epitope in the host proteome protein is in the top 25% most probable B cell epitopes in the protein of interest. In some embodiments, the core peptide in the probable B cell epitope in the protein of interest comprises a sequence of five contiguous amino acids. In some embodiments, the core peptide in the probable B cell epitope in the host proteome protein of interest comprises a sequence of five contiguous amino acids. In some embodiments, the database of core peptides in the data base of host proteome proteins is searched by application of a list of keywords to select to a subset of peptides with functions of interest. In some embodiments, the key words define a group of proteins with neurophysiological function. In some embodiments, the key words define a group of proteins with enzymatic function. In some embodiments, the key words define a group of proteins which function in blood clotting and vascular permeability. In some embodiments, the key words define a group of proteins which function in inflammation. In some embodiments, the key words define a group of proteins which function in arthritis. In some embodiments the core peptide of the probable B cell epitope is matched to the probable B cell epitopes in a dataset of proteins selected based on their known association with a particular disease syndrome. In one particular embodiment, the disease syndrome is Parkinson's disease and related alpha synucleinopathies.

In some embodiments, the methods further comprise identifying those probable B cell epitopes in the protein of interest which are located within 10 to 20 amino acids of a peptide with predicted high binding affinity for one or more MHC II molecule. In some embodiments, the methods further comprise identifying a subpopulation of subjects that is most at risk of adverse effects arising from antibody mediated autoimmunity. In some embodiments, the protein of interest is a microbial protein. In some embodiments, the microbial protein is selected from the group consisting of a virus, a bacteria, a parasite, a fungus, and a microbial toxin. In some embodiments, the protein of interest is an antigen binding protein. In some embodiments, the protein of interest is a biopharmaceutical protein. In some embodiments, the protein of interest is a vaccine. In some embodiments, the protein of interest is a pharmaceutical preparation. In some embodiments, the protein of interest is a food protein. In some embodiments, the protein of interest is an environmental protein. In some embodiments, the methods further comprise the step of synthesizing a mutant version of the protein of interest, wherein the core peptide in the protein of interest is mutated to abrogate the match to a core peptide in the human proteome.

In some embodiments, the present invention provides methods of selecting an animal model to study a disease or to test a vaccine or pharmaceutical product comprising: analyzing a protein of interest by the methods described above both for a human proteome and for a proposed animal model proteome. In some embodiments, said animal model is a mouse. In yet other embodiments the proposed model is a non-human primate. The occurrence of probable epitope mimics in the proposed animal model species is then compared with that of the human, to determine if the model would predict potential autoimmunity in the human subject.

In yet other embodiments, the probable mimics in the human proteome are analyzed by the methods described and then the core peptides of the mimics are compared to determine which other species have identical core peptides in their proteome proteins which are homologous in function to those in the human proteome that carry the core peptides matching the core peptides in the protein of interest.

In some embodiments, the present invention provides methods of producing a vaccine comprising: obtaining one or more gene or amino acid sequences encoding one or more components of vaccine that have been mutated to remove one or more epitope mimics or alter one or more epitope mimics to non-mimics as compared to the corresponding wild type sequences, the epitope mimics identified by a process comprising: assembling a database of all proteins in the human proteome; assigning a curation to each protein based on its reported function; computing the probable B cell epitopes in each protein of the human proteome database wherein the proteins are curated by function; identifying the core peptide of the probable B cell epitopes in each protein of the human proteome; assembling a database of the core peptides of the probable B cell epitopes from each protein of the human proteome in a computer readable medium; entering sequences encoding one or more components of vaccine into a computer with access to the database; computing probable B cell epitopes in the sequences encoding one or more components of vaccine; identifying the core peptide of the probable B cell epitopes in the sequences encoding one or more components of vaccine; comparing the core peptides of the probable B cell epitopes in the sequences encoding one or more components of vaccine to the core peptides contained in the database of peptides from the human proteome; identifying core peptides in predicted B cell epitopes in the sequences encoding one or more components of vaccine which are identical to core peptides in predicted B cell epitopes in one or more proteins of the human proteome; identifying the function of the human proteome proteins which comprise the identical core peptides matching the core peptides of sequences encoding one or more components of vaccine; and synthesizing components for a vaccine by a method selected from the group consisting of a) expressing the one more sequences encoding one or more components of vaccine that have been mutated to remove one or more epitope mimics or alter one or more epitope mimics to non-mimics as compared to the corresponding wild type sequences in a host cell to produce mutated proteins, and b) synthesizing nucleic acid segments encoding the one or more recombinant sequences encoding one or more components of vaccine that have been mutated to remove one or more epitope mimics or alter one or more epitope mimics to non-mimics as compared to the corresponding wild type sequences. In some embodiments, the methods further comprise formulating the mutated proteins or nucleic acid segments with a pharmaceutically acceptable carrier.

In some embodiments, the present invention provides methods of producing a biopharmaceutical protein comprising: obtaining one or more gene or amino acid sequences encoding a biopharmaceutical protein that has been mutated to remove one or more epitope mimics or alter one or more epitope mimics to non-mimics as compared to the corresponding target biopharmaceutical protein sequence, the epitope mimics identified by a process comprising: assembling a database of all proteins in the human proteome; assigning a curation to each protein based on its reported function; computing the probable B cell epitopes in each protein of the human proteome database wherein the proteins are curated by function; identifying the core peptide of the probable B cell epitopes in each protein of the human proteome; assembling a database of the core peptides of the probable B cell epitopes from each protein of the human proteome in a computer readable medium; entering sequences encoding the target biopharmaceutical protein into a computer with access to the database; computing probable B cell epitopes in the sequences encoding the target biopharmaceutical protein; identifying the core peptide of the probable B cell epitopes in the sequences encoding the target biopharmaceutical protein; comparing the core peptides of the probable B cell epitopes in the sequences encoding the target biopharmaceutical protein to the core peptides contained in the database of peptides from the human proteome; identifying core peptides in predicted B cell epitopes in the target biopharmaceutical protein which are identical to core peptides in predicted B cell epitopes in one or more proteins of the human proteome; identifying the function of the human proteome proteins which comprise the identical core peptides matching the core peptides of the target biopharmaceutical protein; and synthesizing the mutated biopharmaceutical protein by expressing the biopharmaceutical that have been mutated to remove one or more epitope mimics or alter one or more epitope mimics to non-mimics as compared to the corresponding target biopharmaceutical protein sequence. In some embodiments, the methods further comprise formulating the mutated biopharmaceutical protein with a pharmaceutically acceptable carrier.

In some embodiments, in the protein of interest is in the top 25% most probable B cell epitopes in the protein of interest (i.e., the vaccine component or biopharmaceutical protein). In some embodiments, the probable B cell epitope in the protein of interest is in the top 10% most probable B cell epitopes in the protein of interest. In some embodiments, the probable B cell epitope in the human proteome protein is in the top 40% most probable B cell epitopes in the protein of interest. In some embodiments, the probable B cell epitope in the human proteome protein is in the top 25% most probable B cell epitopes in the protein of interest. In some embodiments, the core peptide in the probable B cell epitope in the protein of interest comprises a sequence of five contiguous amino acids. In some embodiments, the core peptide in the probable B cell epitope in the human proteome protein of interest comprises a sequence of five contiguous amino acids. In some embodiments, the database of core peptides in the data base of human proteome proteins is searched by application of a list of keywords to select to a subset of peptides with functions of interest. In some embodiments, the key words define a group of proteins with neurophysiological function. In some embodiments, the key words define a group of proteins with enzymatic or endocrine function. In some embodiments, the key words define a group of proteins which function in blood clotting and vascular permeability. In some embodiments, the key words define a group of proteins which function in inflammation. In some embodiments, the methods further comprise identifying those probable B cell epitopes in the protein of interest which are located within 10 to 20 amino acids of a peptide with predicted high binding affinity for one or more MHC II molecule. In some embodiments, the sequences encoding one or more components of vaccine are microbial protein sequences. In some embodiments, the microbial protein sequences are selected from the group consisting of virus, bacteria, parasite, fungus, and microbial toxin sequences. In some embodiments, the target biopharmaceutical protein is selected from the group consisting of an antigen binding protein, a receptor protein and signaling protein. In some embodiments, the methods further comprise administering the one or more components of vaccine that have been mutated to remove one or more epitope mimics or alter one or more epitope mimics to non-mimics as compared to the corresponding wild type sequences to a subject in need thereof. In some embodiments, the methods further comprise administering the biopharmaceutical that have been mutated to remove one or more epitope mimics or alter one or more epitope mimics to non-mimics as compared to the corresponding target biopharmaceutical protein sequence to a subject in need thereof.

In some embodiments, the present invention provides methods of evaluating a biopharmaceutical protein comprising: identifying the presence in the biopharmaceutical protein of probable B cell epitopes and core peptides contained therein; determining which of the core peptides of the probable B cell epitopes match core peptides of probable B cell epitopes in a human proteome; and identifying the function of the proteins thus matched in the human proteome. In some embodiments, the methods further comprise the step of synthesizing a mutant version of the biopharmaceutical protein, wherein the core peptide in the biopharmaceutical protein is mutated to abrogate the match to a core peptide in the human proteome. In some embodiments, the methods further comprise identifying the spectrum of possible side effects arising from the binding of antibody elicited by the vaccine or biopharmaceutical protein to the B cell epitope in a human proteome protein.

In some embodiments, the present invention provides a non-transitory computer readable medium comprising a database of pentamer peptides which are found in human proteins of a defined set of functions and that are the core peptides of a predicted B cell epitope. In some embodiments, the defined set of functions are selected from the group consisting of neurophysiologic, endocrine, cardiovascular, respiratory, hormonal, skin and mucosal health, musculoskeletal functions.

In some embodiments, the present invention provides methods of evaluating potential side effects of a pharmaceutical protein comprising: determining the core peptides located in the probable B cell epitopes of the pharmaceutical proteins; interrogating the database as described above to determine if the core peptides of the pharmaceutical protein are present; and preparing a report identifying a spectrum of possible pathophysiologic interactions of the biopharmaceutical proteins.

In some embodiments, the present invention provides methods of attenuating the pathology of a microorganism comprising: identifying core peptides within probable B cell epitopes of the organism which elicit antibodies that bind to a matching core peptide in a B cell epitope of host protein; and mutating or removing the matching core peptide in the microorganism.

In some embodiments, the present invention provides methods of treating a subject affected by an autoimmune disease comprising: applying the methods described above to identify an epitope mimic peptide; providing the peptide as an antibody binding substrate; and incorporating the antibody binding substrate into an apheresis system.

In some embodiments, the present invention provides methods of diagnosing an autoimmune disease comprising: identifying epitope mimic peptides which elicit antibodies that bind to a human protein by the methods described above; providing a synthetic protein derived from the human protein which comprises the epitope mimic peptides; contacting the synthetic protein with serum harvested from a subject at risk of being affected by an autoimmune disease; and identifying the presence of antibodies with specific binding to mimic epitopes in the synthetic protein.

In some embodiments, the present invention provides methods of diagnosing an autoimmune disease wherein antibody mediated mimicry is suspected, comprising: harvesting a serum sample from a subject suspected of being affected by an autoimmune disease; contacting the serum sample to a microarray of peptides and identifying peptides which bind to antibodies in the serum; and analyzing the peptides thus identified by the methods described above to identify which of the peptides function as epitope mimic peptides.

### DESCRIPTION OF THE FIGURES

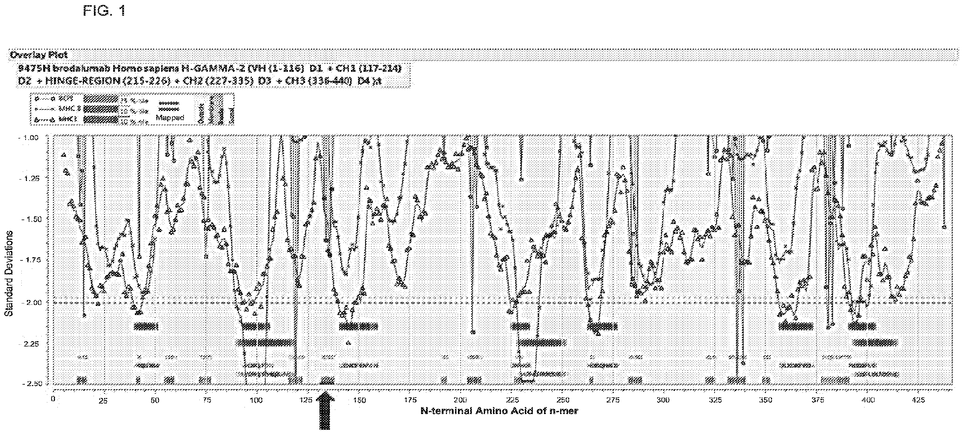

FIG. 1 shows the location of potential mimic epitopes in Brodalumab. X axis shows N>C amino acid positions. Y axis shows standard deviation units of predicted MHC binding. Background shading shows signal peptide (white) and propeptide (yellow). Predicted MHC-I (red line), MHC-II (blue line) binding, and probability of B cell binding (orange lines) for each peptide, arrayed N-C, for a permuted population comprising 63 HLAs. Ribbons (red=MHC-I, blue-MHC-II) indicate the top 25% affinity binding. Orange bars indicate high probability B-cell binding.

### DEFINITIONS

As used herein, the term "genome" refers to the genetic material (e.g., chromosomes) of an organism or a host cell.

As used herein, the term "proteome" refers to the entire set of proteins expressed by a genome, cell, tissue or organism. A "partial proteome" refers to a subset the entire set of proteins expressed by a genome, cell, tissue or organism. Examples of "partial proteomes" include, but are not limited to, transmembrane proteins, secreted proteins, and proteins with a membrane motif. Human proteome refers to all the proteins comprised in a human being. This includes multiple isoforms of many proteins. Multiple such sets of proteins have been sequenced and are accessible at the InterPro international repository (www.ebi.ac.uk/interpro). Another such repository is UniProt (www.uniprot.org) Human proteome is also understood to include those proteins and antigens thereof which may be over-expressed in certain pathologies, or expressed in a different isoforms in certain pathologies. Hence, as used herein, tumor associated antigens are considered part of the human proteome. Murine proteome refers to the proteome of the mouse as catalogued in Uniprot, where a reference proteome is recorded for C57BL/6J mice www.uniprot.org/proteomes/UP000000589.

As used herein the term "host proteome" refers to the proteome of any species of interest in the study of a disease that afflicts said host. Thus for example, the human proteome is a host proteome for a human disease and a mouse proteome is a host proteome for a virus that infects it; and a macaque proteome is a host proteome for a parasite that affects it.

As used herein, the terms "protein," "polypeptide," and "peptide" refer to a molecule comprising amino acids joined via peptide bonds. In general "peptide" is used to refer to a sequence of 20 or less amino acids and "polypeptide" is used to refer to a sequence of greater than 20 amino acids.

As used herein, the term, "synthetic polypeptide," "synthetic peptide" and "synthetic protein" refer to peptides, polypeptides, and proteins that are produced by a recombinant process (i.e., expression of exogenous nucleic acid encoding the peptide, polypeptide or protein in an organism, host cell, or cell-free system) or by chemical synthesis.

As used herein, the term "protein of interest" refers to a protein encoded by a nucleic acid of interest. It may be applied to any protein to which further analysis is applied or the properties of which are tested or examined. Similarly, as used herein, "target protein" may be used to describe a protein of interest that is subject to further analysis.

As used herein "peptidase" refers to an enzyme which cleaves a protein or peptide. The term peptidase may be used interchangeably with protease, proteinases, oligopeptidases, and proteolytic enzymes. Peptidases may be endopeptidases (endoproteases), or exopeptidases (exoproteases). Similarly, the term peptidase inhibitor may be used interchangeably with protease inhibitor or inhibitor of any of the other alternate terms for peptidase.

As used herein, the term "exopeptidase" refers to a peptidase that requires a free N-terminal amino group, C-terminal carboxyl group or both, and hydrolyses a bond not more than three residues from the terminus. The exopeptidases are further divided into aminopeptidases, carboxypeptidases, dipeptidyl-peptidases, peptidyl-dipeptidases, tripeptidyl-peptidases and dipeptidases.

As used herein, the term "endopeptidase" refers to a peptidase that hydrolyses internal, alpha-peptide bonds in a polypeptide chain, tending to act away from the N-terminus or C-terminus. Examples of endopeptidases are chymotrypsin, pepsin, papain and cathepsins. A very few endopeptidases act a fixed distance from one terminus of the substrate, an example being mitochondrial intermediate peptidase. Some endopeptidases act only on substrates smaller than proteins, and these are termed oligopeptidases. An example of an oligopeptidase is thimet oligopeptidase. Endopeptidases initiate the digestion of food proteins, generating new N- and C-termini that are substrates for the exopeptidases that complete the process. Endopeptidases also process proteins by limited proteolysis. Examples are the removal of signal peptides from secreted proteins (e.g. signal peptidase I,) and the maturation of precursor proteins (e.g. enteropeptidase, furin). In the nomenclature of the Nomenclature Committee of the International Union of Biochemistry and Molecular Biology (NC-IUBMB) endopeptidases are allocated to sub-subclasses EC 3.4.21, EC 3.4.22, EC 3.4.23, EC 3.4.24 and EC 3.4.25 for serine-, cysteine-, aspartic-, metallo- and threonine-type endopeptidases, respectively. Endopeptidases of particular interest are the cathepsins, and especially cathepsin B, L and S known to be active in antigen presenting cells.

As used herein, the term "immunogen" refers to a molecule which stimulates a response from the adaptive immune system, which may include responses drawn from the group comprising an antibody response, binding to a B cell epitope, a cytotoxic T cell response, a T helper response, and a T cell memory. An immunogen may stimulate an upregulation of the immune response with a resultant inflammatory response, or may result in down regulation or immunosuppression. Thus the T-cell response may be a T regulatory response. An immunogen also may stimulate a B-cell response and lead to an increase in antibody titer. "Antigen" is a term used to describe one or more immunogens

As used herein, the term "native" (or "wild type") when used in reference to a protein refers to proteins encoded by the genome of a cell, tissue, or organism, other than one manipulated to produce synthetic proteins.

As used herein the term "epitope" refers to a peptide sequence which elicits an immune response, from either T cells or B cells or antibody

As used herein, the term "B-cell epitope" refers to a polypeptide sequence that is recognized and bound by a B-cell receptor. A B-cell epitope may be a linear peptide or may comprise several discontinuous sequences which together are folded to form a structural epitope. Such component sequences which together make up a B-cell epitope are referred to herein as B-cell epitope sequences. Hence, a B-cell epitope may comprise one or more B-cell epitope sequences. Hence, a B cell epitope may comprise one or more B-cell epitope sequences. A linear B-cell epitope may comprise as few as 2-4 amino acids or more amino acids. In some particular instances the B cell epitope is a pentamer of five contiguous amino acids.

As used herein, the term "predicted B-cell epitope" refers to a polypeptide sequence that is predicted to bind to a B-cell receptor by a computer program, for example, as described in PCT US2011/029192, PCT US2012/055038, and US2014/014523, each of which is incorporated herein by reference, and in addition by Bepipred (Larsen, et al., Immunome Research 2:2, 2006.) and others as referenced by Larsen et al (ibid) (Hopp T et al PNAS 78:3824-3828, 1981; Parker J et al, Biochem. 25:5425-5432, 1986). A predicted B-cell epitope may refer to the identification of B-cell epitope sequences forming part of a structural B-cell epitope or to a complete B-cell epitope. In some usages herein B cell epitope is abbreviated to BEPI.

As used herein, the term "T-cell epitope" refers to a polypeptide sequence which when bound to a major histocompatibility protein molecule provides a configuration recognized by a T-cell receptor. Typically, T-cell epitopes are presented bound to a MHC molecule on the surface of an antigen-presenting cell.

As used herein, the term "predicted T-cell epitope" refers to a polypeptide sequence that is predicted to bind to a major histocompatibility protein molecule by the neural network algorithms described herein, by other computerized methods, or as determined experimentally.

As used herein, the term "major histocompatibility complex (MHC)" refers to the MHC Class I and MHC Class II genes and the proteins encoded thereby. Molecules of the MHC bind small peptides and present them on the surface of cells for recognition by T-cell receptor-bearing T-cells. The MHC-Is both polygenic (there are several MHC class I and MHC class II genes) and polyallelic or polymorphic (there are multiple alleles of each gene). The terms MHC-I, MHC-II, MHC-1 and MHC-2 are variously used herein to indicate these classes of molecules. Included are both classical and nonclassical MHC molecules. An MHC molecule is made up of multiple chains (alpha and beta chains) which associate to form a molecule. The MHC molecule contains a cleft or groove which forms a binding site for peptides. Peptides bound in the cleft or groove may then be presented to T-cell receptors. The term "MHC binding region" refers to the groove region of the MHC molecule where peptide binding occurs.

As used herein, a "MHC II binding groove" refers to the structure of an MHC molecule that binds to a peptide. The peptide that binds to the MHC II binding groove may be from about 11 amino acids to about 23 amino acids in length, but typically comprises a 15-mer. The amino acid positions in the peptide that binds to the groove are numbered based on a central core of 9 amino acids numbered 1-9, and positions outside the 9 amino acid core numbered as negative (N terminal) or positive (C terminal). Hence, in a 15mer the amino acid binding positions are numbered from -3 to +3 or as follows: -3, -2, -1, 1, 2, 3, 4, 5, 6, 7, 8, 9, +1, +2, +3.

As used herein, the term "haplotype" refers to the HLA alleles found on one chromosome and the proteins encoded thereby. Haplotype may also refer to the allele present at any one locus within the MHC. Each class of MHC-Is represented by several loci: e.g., HLA-A (Human Leukocyte Antigen-A), HLA-B, HLA-C, HLA-E, HLA-F, HLA-G, HLA-H, HLA-J, HLA-K, HLA-L, HLA-P and HLA-V for class I and HLA-DRA, HLA-DRB1-9, HLA-, HLA-DQA1, HLA-DQB1, HLA-DPA1, HLA-DPB1, HLA-DMA, HLA-DMB, HLA-DOA, and HLA-DOB for class II. The terms "HLA allele" and "MHC allele" are used interchangeably herein. HLA alleles are listed at hla.alleles.org/nomenclature/naming.html, which is incorporated herein by reference.

The MHCs exhibit extreme polymorphism: within the human population there are, at each genetic locus, a great number of haplotypes comprising distinct alleles-the IMGT/HLA database release (February 2010) lists 948 class I and 633 class II molecules, many of which are represented at high frequency (>1%). MHC alleles may differ by as many as 30-aa substitutions. Different polymorphic MHC alleles, of both class I and class II, have different peptide specificities: each allele encodes proteins that bind peptides exhibiting particular sequence patterns.

The naming of new HLA genes and allele sequences and their quality control is the responsibility of the WHO Nomenclature Committee for Factors of the HLA System, which first met in 1968, and laid down the criteria for successive meetings. This committee meets regularly to discuss issues of nomenclature and has published 19 major reports documenting firstly the HLA antigens and more recently the genes and alleles. The standardization of HLA antigenic specifications has been controlled by the exchange of typing reagents and cells in the International Histocompatibility Workshops. The IMGT/HLA Database collects both new and confirmatory sequences, which are then expertly analyzed and curated before been named by the Nomenclature Committee. The resulting sequences are then included in the tools and files made available from both the IMGT/HLA Database and at hla.alleles.org.

Each HLA allele name has a unique number corresponding to up to four sets of digits separated by colons. See e.g., hla.alleles.org/nomenclature/naming.html which provides a description of standard HLA nomenclature and Marsh et al., Nomenclature for Factors of the HLA System, 2010 Tissue Antigens 2010 75:291-455. HLA-DRB1*13:01 and HLA-DRB 1*13:01:01:02 are examples of standard HLA nomenclature. The length of the allele designation is dependent on the sequence of the allele and that of its nearest relative. All alleles receive at least a four digit name, which corresponds to the first two sets of digits, longer names are only assigned when necessary.

The digits before the first colon describe the type, which often corresponds to the serological antigen carried by an allotype, The next set of digits are used to list the subtypes, numbers being assigned in the order in which DNA sequences have been determined. Alleles whose numbers differ in the two sets of digits must differ in one or more nucleotide substitutions that change the amino acid sequence of the encoded protein. Alleles that differ only by synonymous nucleotide substitutions (also called silent or non-coding substitutions) within the coding sequence are distinguished by the use of the third set of digits. Alleles that only differ by sequence polymorphisms in the introns or in the 5' or 3' untranslated regions that flank the exons and introns are distinguished by the use of the fourth set of digits. In addition to the unique allele number there are additional optional suffixes that may be added to an allele to indicate its expression status. Alleles that have been shown not to be expressed, 'Null' alleles have been given the suffix 'N'. Those alleles which have been shown to be alternatively expressed may have the suffix 'L', 'S', 'C', 'A' or 'Q'. The suffix 'L' is used to indicate an allele which has been shown to have 'Low' cell surface expression when compared to normal levels. The 'S' suffix is used to denote an allele specifying a protein which is expressed as a soluble 'Secreted' molecule but is not present on the cell surface. A 'C' suffix to indicate an allele product which is present in the 'Cytoplasm' but not on the cell surface. An 'A' suffix to indicate 'Aberrant' expression where there is some doubt as to whether a protein is expressed. A 'Q' suffix when the expression of an allele is 'Questionable' given that the mutation seen in the allele has previously been shown to affect normal expression levels.

In some instances, the HLA designations used herein may differ from the standard HLA nomenclature just described due to limitations in entering characters in the databases described herein. As an example, DRB1_0104, DRB1*0104, and DRB1-0104 are equivalent to the standard nomenclature of DRB1 *01:04. In most instances, the asterisk is replaced with an underscore or dash and the semicolon between the two digit sets is omitted.

As used herein, the term "polypeptide sequence that binds to at least one major histocompatibility complex (MHC) binding region" refers to a polypeptide sequence that is recognized and bound by one or more particular MHC binding regions as predicted by the neural network algorithms described herein or as determined experimentally.

As used herein the terms "canonical" and "non-canonical" are used to refer to the orientation of an amino acid sequence. Canonical refers to an amino acid sequence presented or read in the N terminal to C terminal order; non-canonical is used to describe an amino acid sequence presented in the inverted or C terminal to N terminal order.

As used herein, the term "affinity" refers to a measure of the strength of binding between two members of a binding pair, for example, an antibody and an epitope and an epitope and a MHC-I or II haplotype. K_{d} is the dissociation constant and has units of molarity. The affinity constant is the inverse of the dissociation constant. An affinity constant is sometimes used as a generic term to describe this chemical entity. It is a direct measure of the energy of binding. The natural logarithm of K is linearly related to the Gibbs free energy of binding through the equation ΔG₀ = -RT LN(K) where R= gas constant and temperature is in degrees Kelvin. Affinity may be determined experimentally, for example by surface plasmon resonance (SPR) using commercially available Biacore SPR units (GE Healthcare) or *in silico* by methods such as those described herein in detail. Affinity may also be expressed as the ic50 or inhibitory concentration 50, that concentration at which 50% of the peptide is displaced. Likewise ln(ic50) refers to the natural log of the ic50.

The term "K_{off}", as used herein, is intended to refer to the off rate constant, for example, for dissociation of an antibody from the antibody/antigen complex, or for dissociation of an epitope from an MHC haplotype.

The term "K_{d}", as used herein, is intended to refer to the dissociation constant (the reciprocal of the affinity constant "Ka"), for example, for a particular antibody-antigen interaction or interaction between an epitope and an MHC haplotype.

As used herein, the terms "strong binder" and "strong binding" and "High binder" and "high binding" or "high affinity" refer to a binding pair or describe a binding pair that have an affinity of greater than 2 ×10⁷M⁻¹ (equivalent to a dissociation constant of 50nM Kd)

As used herein, the term "moderate binder" and "moderate binding" and "moderate affinity" refer to a binding pair or describe a binding pair that have an affinity of from 2 ×10⁷M⁻¹ to 2 ×10⁶M⁻¹.

As used herein, the terms "weak binder" and "weak binding" and "low affinity" refer to a binding pair or describe a binding pair that have an affinity of less than 2 ×10⁶M⁻¹ (equivalent to a dissociation constant of 500nM Kd)

Binding affinity may also be expressed by the standard deviation from the mean binding found in the peptides making up a protein. Hence a binding affinity may be expressed as "-1σ" or <-1σ, where this refers to a binding affinity of 1 or more standard deviations below the mean. A common mathematical transformation used in statistical analysis is a process called standardization wherein the distribution is transformed from its standard units to standard deviation units where the distribution has a mean of zero and a variance (and standard deviation) of 1. Because each protein comprises unique distributions for the different MHC alleles standardization of the affinity data to zero mean and unit variance provides a numerical scale where different alleles and different proteins can be compared. Analysis of a wide range of experimental results suggest that a criterion of standard deviation units can be used to discriminate between potential immunological responses and non-responses. An affinity of 1 standard deviation below the mean was found to be a useful threshold in this regard and thus approximately 15% (16.2% to be exact) of the peptides found in any protein will fall into this category.

The terms "specific binding" or "specifically binding" when used in reference to the interaction of an antibody and a protein or peptide or an epitope and an MHC haplotype means that the interaction is dependent upon the presence of a particular structure (i.e., the antigenic determinant or epitope) on the protein; in other words the antibody is recognizing and binding to a specific protein structure rather than to proteins in general. For example, if an antibody is specific for epitope "A," the presence of a protein containing epitope A (or free, unlabeled A) in a reaction containing labeled "A" and the antibody will reduce the amount of labeled A bound to the antibody.

As used herein, the term "antigen binding protein" refers to proteins that bind to a specific antigen. "Antigen binding proteins" include, but are not limited to, immunoglobulins, including polyclonal, monoclonal, chimeric, single chain, and humanized antibodies, Fab fragments, F(ab')2 fragments, and Fab expression libraries. Various procedures known in the art are used for the production of polyclonal antibodies. For the production of antibody, various host animals can be immunized by injection with the peptide corresponding to the desired epitope including but not limited to rabbits, mice, rats, sheep, goats, *etc.* Various adjuvants are used to increase the immunological response, depending on the host species, including but not limited to Freund's (complete and incomplete), mineral gels such as aluminum hydroxide, surface active substances such as lysolecithin, pluronic polyols, polyanions, peptides, oil emulsions, keyhole limpet hemocyanins, dinitrophenol, and potentially useful human adjuvants such as BCG (Bacille Calmette-Guerin) and *Corynebacterium parvum.*

For preparation of monoclonal antibodies, any technique that provides for the production of antibody molecules by continuous cell lines in culture may be used (*See e.g.,* Harlow and Lane, Antibodies: A Laboratory Manual, Cold Spring Harbor Laboratory Press, Cold Spring Harbor, NY). These include, but are not limited to, the hybridoma technique originally developed by Köhler and Milstein (Köhler and Milstein, Nature, 256:495-497 [1975]), as well as the trioma technique, the human B-cell hybridoma technique (*See e.g.,* Kozbor et al., Immunol. Today, 4:72 [1983]), and the EBV-hybridoma technique to produce human monoclonal antibodies (Cole et al., in Monoclonal Antibodies and Cancer Therapy, Alan R. Liss, Inc., pp. 77-96 [1985]). In other embodiments, suitable monoclonal antibodies, including recombinant chimeric monoclonal antibodies and chimeric monoclonal antibody fusion proteins are prepared as described herein.

According to the invention, techniques described for the production of single chain antibodies (US 4,946,778; herein incorporated by reference) can be adapted to produce specific single chain antibodies as desired. An additional embodiment of the invention utilizes the techniques known in the art for the construction of Fab expression libraries (Huse et al., Science, 246:1275-1281 [1989]) to allow rapid and easy identification of monoclonal Fab fragments with the desired specificity.

Antibody fragments that contain the idiotype (antigen binding region) of the antibody molecule can be generated by known techniques. For example, such fragments include but are not limited to: the F(ab')2 fragment that can be produced by pepsin digestion of an antibody molecule; the Fab' fragments that can be generated by reducing the disulfide bridges of an F(ab')2 fragment, and the Fab fragments that can be generated by treating an antibody molecule with papain and a reducing agent.

Genes encoding antigen-binding proteins can be isolated by methods known in the art. In the production of antibodies, screening for the desired antibody can be accomplished by techniques known in the art (*e.g*., radioimmunoassay, ELISA (enzyme-linked immunosorbant assay), "sandwich" immunoassays, immunoradiometric assays, gel diffusion precipitin reactions, immunodiffusion assays, *in situ* immunoassays (using colloidal gold, enzyme or radioisotope labels, for example), Western Blots, precipitation reactions, agglutination assays (*e.g*., gel agglutination assays, hemagglutination assays, *etc*.), complement fixation assays, immunofluorescence assays, protein A assays, and immunoelectrophoresis assays, *etc.*) *etc.*

As used herein "immunoglobulin" means the distinct antibody molecule secreted by a clonal line of B cells; hence when the term "100 immunoglobulins" is used it conveys the distinct products of 100 different B-cell clones and their lineages.

As used herein, the terms "computer memory" and "computer memory device" refer to any storage media readable by a computer processor. Examples of computer memory include, but are not limited to, RAM, ROM, computer chips, digital video disc (DVDs), compact discs (CDs), hard disk drives (HDD), and magnetic tape.

As used herein, the term "computer readable medium" refers to any device or system for storing and providing information (e.g., data and instructions) to a computer processor. Examples of computer readable media include, but are not limited to, DVDs, CDs, hard disk drives, magnetic tape and servers for streaming media over networks.

As used herein, the terms "processor" and "central processing unit" or "CPU" are used interchangeably and refer to a device that is able to read a program from a computer memory (e.g., ROM or other computer memory) and perform a set of steps according to the program.

As used herein, the term "support vector machine" refers to a set of related supervised learning methods used for classification and regression. Given a set of training examples, each marked as belonging to one of two categories, an SVM training algorithm builds a model that predicts whether a new example falls into one category or the other.

As used herein, the term "classifier" when used in relation to statistical processes refers to processes such as neural nets and support vector machines.

As used herein "neural net", which is used interchangeably with "neural network" and sometimes abbreviated as NN, refers to various configurations of classifiers used in machine learning, including multilayered perceptrons with one or more hidden layer, support vector machines and dynamic Bayesian networks. These methods share in common the ability to be trained, the quality of their training evaluated, and their ability to make either categorical classifications of non numeric data or to generate equations for predictions of continuous numbers in a regression mode. Perceptron as used herein is a classifier which maps its input x to an output value which is a function of x, or a graphical representation thereof.

As used herein, the term "principal component analysis", or as abbreviated PCA, refers to a mathematical process which reduces the dimensionality of a set of data (Wold, S., Sjorstrom,M., and Eriksson,L., Chemometrics and Intelligent Laboratory Systems 2001. 58: 109-130.; Multivariate and Megavariate Data Analysis Basic Principles and Applications (Parts I&II) by L. Eriksson, E. Johansson, N. Kettaneh-Wold, and J. Trygg , 2006 2nd Edit. Umetrics Academy ). Derivation of principal components is a linear transformation that locates directions of maximum variance in the original input data, and rotates the data along these axes. For n original variables, n principal components are formed as follows: The first principal component is the linear combination of the standardized original variables that has the greatest possible variance. Each subsequent principal component is the linear combination of the standardized original variables that has the greatest possible variance and is uncorrelated with all previously defined components. Further, the principal components are scale-independent in that they can be developed from different types of measurements. The application of PCA generates numerical coefficients (descriptors). The coefficients are effectively proxy variables whose numerical values are seen to be related to underlying physical properties of the molecules. A description of the application of PCA to generate descriptors of amino acids and by combination thereof peptides is provided in PCT US2011/029192 incorporated herein by reference, Unlike neural nets PCA do not have any predictive capability. PCA is deductive not inductive.

As used herein, the term "vector" when used in relation to a computer algorithm or the present invention, refers to the mathematical properties of the amino acid sequence.

As used herein, the term "vector," when used in relation to recombinant DNA technology, refers to any genetic element, such as a plasmid, phage, transposon, cosmid, chromosome, retrovirus, virion, *etc.,* which is capable of replication when associated with the proper control elements and which can transfer gene sequences between cells. Thus, the term includes cloning and expression vehicles, as well as viral vectors.

As used herein, the term "vector" when used in relation to transmission of an arbovirus refers to the intermediate host of a virus, such as a mosquito or tick or other arthropod.

As used herein, the term "host cell" refers to any eukaryotic cell (*e.g*., mammalian cells, avian cells, amphibian cells, plant cells, fish cells, insect cells, yeast cells), and bacteria cells, and the like, whether located *in vitro* or *in vivo* (*e.g.,* in a transgenic organism).

As used herein, the term "cell culture" refers to any *in vitro* culture of cells. Included within this term are continuous cell lines (*e.g*., with an immortal phenotype), primary cell cultures, finite cell lines (*e.g*., non-transformed cells), and any other cell population maintained *in vitro,* including oocytes and embryos.

The term "isolated" when used in relation to a nucleic acid, as in "an isolated oligonucleotide" refers to a nucleic acid sequence that is identified and separated from at least one contaminant nucleic acid with which it is ordinarily associated in its natural source. Isolated nucleic acids are nucleic acids present in a form or setting that is different from that in which they are found in nature. In contrast, non-isolated nucleic acids are nucleic acids such as DNA and RNA that are found in the state in which they exist in nature.

The terms "in operable combination," "in operable order," and "operably linked" as used herein refer to the linkage of nucleic acid sequences in such a manner that a nucleic acid molecule capable of directing the transcription of a given gene and/or the synthesis of a desired protein molecule is produced. The term also refers to the linkage of amino acid sequences in such a manner so that a functional protein is produced.

A "subject" is an animal such as vertebrate, preferably a mammal such as a human, or a bird, or a fish. Mammals are understood to include, but are not limited to, murines, simians, humans, bovines, ovines, cervids, equines, porcines, canines, felines *etc.*)*.*

An "effective amount" is an amount sufficient to effect beneficial or desired results. An effective amount can be administered in one or more administrations,

As used herein, the term "purified" or "to purify" refers to the removal of undesired components from a sample. As used herein, the term "substantially purified" refers to molecules, either nucleic or amino acid sequences, that are removed from their natural environment, isolated or separated, and are at least 60% free, preferably 75% free, and most preferably 90% free from other components with which they are naturally associated. An "isolated polynucleotide" is therefore a substantially purified polynucleotide.

"Strain" as used herein in reference to a microorganism describes an isolate of a microorganism (e.g., bacteria, virus, fungus, parasite) considered to be of the same species but with a unique genome and, if nucleotide changes are non-synonymous, a unique proteome differing from other strains of the same organism. Typically strains may be the result of isolation from a different host or at a different location and time but multiple strains of the same organism may be isolated from the same host.

As used herein "Complementarity Determining Regions" (CDRs) are those parts of the immunoglobulin variable chains which determine how these molecules bind to their specific antigen. Each immunoglobulin variable region typically comprises three CDRs and these are the most highly variable regions of the molecule.

As used herein, the term "motif" refers to a characteristic sequence of amino acids forming a distinctive pattern.

The term "Groove Exposed Motif" (GEM) as used herein refers to a subset of amino acids within a peptide that binds to an MHC molecule; the GEM comprises those amino acids which are turned inward towards the groove formed by the MHC molecule and which play a significant role in determining the binding affinity. In the case of human MHC-I the GEM amino acids are typically (1,2,3,9). In the case of MHC-Π molecules two formats of GEM are most common comprising amino acids (-3,2,-1,1,4,6,9,+1,+2,+3) and (-3,2,1,2,4,6,9,+1,+2,+3) based on a 15 -mer peptide with a central core of 9 amino acids numbered 1-9 and positions outside the core numbered as negative (N terminal) or positive (C terminal).

"Immunoglobulin germline" is used herein to refer to the variable region sequences encoded in the inherited germline genes and which have not yet undergone any somatic hypermutation. Each individual carries and expresses multiple copies of germline genes for the variable regions of heavy and light chains. These undergo somatic hypermutation during affinity maturation. Information on the germline sequences of immunoglobulins is collated and referenced by www. imgt.org (*1*). "Germline family" as used herein refers to the 7 main gene groups, catalogued at IMGT, which share similarity in their sequences and which are further subdivided into subfamilies.

"Affinity maturation" is the molecular evolution that occurs during somatic hypermutation during which unique variable region sequences generated that are the best at targeting and neutralizing and antigen become clonally expanded and dominate the responding cell populations.

"Germline motif" as used herein describes the amino acid subsets that are found in germline immunoglobulins. Germline motifs comprise both GEM and TCEM motifs found in the variable regions of immunoglobulins which have not yet undergone somatic hypermutation.

"Immunopathology" when used herein describes an abnormality of the immune system. An immunopathology may affect B-cells and their lineage causing qualitative or quantitative changes in the production of immunoglobulins. Immunopathologies may alternatively affect T-cells and result in abnormal T-cell responses. Immunopathologies may also affect the antigen presenting cells. Immunopathologies may be the result of neoplasias of the cells of the immune system. Immunopathology is also used to describe diseases mediated by the immune system such as autoimmune diseases. Illustrative examples of immunopathologies include, but are not limited to, B-cell lymphoma, T-cell lymphomas, Systemic Lupus Erythematosus (SLE), allergies, hypersensitivities, immunodeficiency syndromes, radiation exposure or chronic fatigue syndrome.

An "autoimmune disease" or "autoimmunity" as used herein refers to any disease or pathology which arises as the result of an immune response directed to a self-antigen. An autoimmune disease may be chronic, lasting over years with periodic flare ups and remissions, or many be acute and transitory, such as when an acute infection generates antibodies directed to a self-protein and the effects of said antibodies wane rapidly in days or weeks.

"Obverse" as used herein describes the outward directed face or the side facing outwards. Hence, in the context of a pMHC complex, the obverse side is that face presented to the T-cell receptor and comprises the space-shape made up of the TCEM and the contiguous and surrounding outward facing components of the MHC molecule that will be different for each different MHC allele.

"pMHC" Is used to describe a complex of a peptide bound to an MHC molecule. In many instances a peptide bound to an MHC-I will be a 9-mer or 10-mer however other sizes of 7-11 amino acids may be thus bound. Similarly MHC-II molecules may form pMHC complexes with peptides of 15 amino acids or with peptides of other sizes from 11-23 amino acids. The term pMHC is thus understood to include any short peptide bound to a corresponding MHC.

"Somatic hypermutation" (SHM), as used herein refers to the process by which variability in the immunoglobulin variable region is generated during the proliferation of individual B-cells responding to an immune stimulus. SHM occurs in the complementarity determining regions.

"T-cell exposed motif" (TCEM), as used herein, refers to the sub set of amino acids in a peptide bound in a MHC molecule which are directed outwards and exposed to a T-cell binding to the pMHC complex. A T-cell binds to a complex molecular space-shape made up of the outer surface MHC of the particular HLA allele and the exposed amino acids of the peptide bound within the MHC. Hence any T-cell recognizes a space shape or receptor which is specific to the combination of HLA and peptide. The amino acids which comprise the TCEM in an MHC-I binding peptide typically comprise positions 4, 5, 6, 7, 8 of a 9-mer. The amino acids which comprise the TCEM in an MHC-II binding peptide typically comprise 2, 3, 5, 7, 8 or -1, 3, 5, 7, 8 based on a 15-mer peptide with a central core of 9 amino acids numbered 1-9 and positions outside the core numbered as negative (N terminal) or positive (C terminal). As indicated under pMHC, the peptide bound to a MHC may be of other lengths and thus the numbering system here is considered a non-exclusive example of the instances of 9-mer and 15 mer peptides.

"Regulatory T-cell" or "Treg" as used herein, refers to a T-cell which has an immunosuppressive or down-regulatory function. Regulatory T-cells were formerly known as suppressor T-cells. Regulatory T-cells come in many forms but typically are characterized by expression CD4+, CD25, and Foxp3. Tregs are involved in shutting down immune responses after they have successfully eliminated invading organisms, and also in preventing immune responses to self-antigens or autoimmunity.

"Tregitope" as used herein describes an epitope to which a Treg or regulatory T-cell binds.

"uTOPE^{™} analysis" as used herein refers to the computer assisted processes for predicting binding of peptides to MHC and predicting cathepsin cleavage, described in PCT US2011/029192, PCT US2012/055038, and US2014/01452, each of which is incorporated herein by reference.

"Framework region" as used herein refers to the amino acid sequences within an immunoglobulin variable region which do not undergo somatic hypermutation.

"Isotype" as used herein refers to the related proteins of particular gene family. Immunoglobulin isotype refers to the distinct forms of heavy and light chains in the immunoglobulins. In heavy chains there are five heavy chain isotypes (alpha, delta, gamma, epsilon, and mu, leading to the formation of IgA, IgD, IgG, IgE and IgM respectively) and light chains have two isotypes (kappa and lambda). Isotype when applied to immunoglobulins herein is used interchangeably with immunoglobulin "class".

"Isoform" as used herein refers to different forms of a protein which differ in a small number of amino acids. The isoform may be a full length protein (i.e., by reference to a reference wild-type protein or isoform) or a modified form of a partial protein, i.e., be shorter in length than a reference wild-type protein or isoform.

"Class switch recombination" (CSR) as used herein refers to the change from one isotype of immunoglobulin to another in an activated B cell, wherein the constant region associated with a specific variable region is changed, typically from IgM to IgG or other isotypes.

"Immunostimulation" as used herein refers to the signaling that leads to activation of an immune response, whether said immune response is characterized by a recruitment of cells or the release of cytokines which lead to suppression of the immune response. Thus immunostimulation refers to both upregulation or down regulation.

"Up-regulation" as used herein refers to an immunostimulation which leads to cytokine release and cell recruitment tending to eliminate a non self or exogenous epitope. Such responses include recruitment of T cells, including effectors such as cytotoxic T cells, and inflammation. In an adverse reaction upregulation may be directed to a self-epitope.

"Down regulation" as used herein refers to an immunostimulation which leads to cytokine release that tends to dampen or eliminate a cell response. In some instances such elimination may include apoptosis of the responding T cells.

"Frequency class" or "frequency classification" as used herein is used to describe the counts of TCEM motifs found in a given dataset of peptides. A logarithmic (log base 2) frequency categorization scheme was developed to describe the distribution of motifs in a dataset. As the cellular interactions between T-cells and antigen presenting cells displaying the motifs in MHC molecules on their surfaces are the ultimate result of the molecular interactions, using a log base 2 system implies that each adjacent frequency class would double or halve the cellular interactions with that motif. Thus using such a frequency categorization scheme makes it possible to characterize subtle differences in motif usage as well as providing a comprehensible way of visualizing the cellular interaction dynamics with the different motifs. Hence a Frequency Class 2, or FC 2 means 1 in 4, a Frequency class 10 or FC 10 means 1 in 2¹⁰ or 1 in 1024.

"40K set" as used herein refers to the database of 40,000 IGHV assembled from Genbank as described in Example 1

"IGHV" as used herein is an abbreviation for immunoglobulin heavy chain variable regions

"IGLV" as used herein is an abbreviation for immunoglobulin light chain variable regions "Adverse immune response" as used herein may refer to (a) the induction of immunosuppression when the appropriate response is an active immune response to eliminate a pathogen or tumor or (b) the induction of an upregulated active immune response to a self-antigen or (c) an excessive up-regulation unbalanced by any suppression, as may occur for instance in an allergic response.

As used herein "epitope mimic" describes a peptide that is present and elicits an immune response in one protein (e.g., source protein) and the humoral and cellular effectors of that immune response then recognize and act upon the same peptide motif where it occurs in a different protein (e.g., target protein). For example, an antibody which is elicited by a B cell epitope in a microorganism and which binds to a B cell epitope peptide derived from a human protein would be said to have found an epitope mimic. In some embodiments, epitope mimics are an important mechanism in autoimmunity.

As used herein "TCEM mimic" is used to describe a peptide which has an identical or overlapping TCEM, but may have a different GEM. Such a mimic occurring in one protein may induce an immune response directed towards another protein which carries the same TCEM motif. This may give rise to autoimmunity or inappropriate responses to the second protein.

"Anchor peptide", as used herein, refers to peptides or polypeptides which allow binding to a substrate to facilitate purification or which facilitate attachment to a solid medium such as a bead or plastic dish or are capable of insertion into a membrane of a cell or liposome or virus like particle or other nanoparticle. Among the examples of anchor peptides are the following, which are considered non-limiting, his tags, immunoglobulins, Fc region of immunoglobulin, G coupled protein, receptor ligand, biotin, and FLAG tags. In some instances an anchor peptide is designed to be cleavable following exposure to an endopeptidase in vitro or in vivo.

"Cytotoxin" or "cytocide" as used herein refers to a peptide or polypeptide which is toxic to cells and which causes cell death. Among the non-limiting examples of such polypeptides are RNAses, phospholipase, membrane active peptides such as cercropin, and diphtheria toxin. Cytotoxin also includes radionuclides which are cytotoxic.

"Cytokine" as used herein refers to a protein which is active in cell signaling and may include, among other examples, chemokines, interferons, interleukins, lymphokines, *granulocyte colony-stimulating factor,* tumor necrosis factor and programmed death proteins.

As used herein the term "Alpha emitter" refers to a radioisotope which emits alpha radiation. Examples of alpha emitters which may be suitable for clinical use include Astatine-211, Bismuth-212, Bismuth-213, Actinium-225 Radium-223, Terbium-149, Fermium-255

As used herein "Auger particles" refers to the low energy electrons emitted by radionuclides such as but not limited to, Gadolinium-67, Technicium-99, Indium-111, Iodine-123, Iodine-125, Tellurium-201. Auger electrons are advantageous as they have a short path of transit through tissue.

As used herein "oncoprotein" means a protein encoded by an oncogene which can cause the transformation of a cell into a tumor cell if introduced into it. Examples of oncoproteins include but are not limited to the early proteins of papillomaviruses, polyomaviruses, adenoviruses and herpes viruses, however oncoproteins are not necessarily of viral origin.

"Label peptide" as used herein refers to a peptide or polypeptide which provides, either directly or by a ligated residue, a colorimetric , fluorescent, radiation emitting, light emitting, metallic or radiopaque signal which can be used to identify the location of said peptide. Among the non-limiting examples of such label peptides are streptavidin, fluorescein, luciferase, gold, ferritin, tritium,

"MHC subunit chain" as used herein refers to the alpha and beta subunits of MHC molecules. A MHC II molecule is made up of an alpha chain which is constant among each of the DR, DP, and DQ variants and a beta chain which varies by allele. The MHC I molecule is made up of a constant beta macroglobulin and a variable MHC A, B or C chain.

As used herein "high frequency T cell exposed motifs" refers to a T cell exposed motif which occurs at high frequency in a reference database of >50000 immunoglobulin variable regions. A motif that occurs more than once in 1024 variable regions is considered to be a high frequency motif which will have a large cognate T cell population and be likely to elicit a Tregulatory response when it is also highly bound by a MHC molecule.

The term "nanoparticle" as used herein refers to a small particle used to array immunogens which may be comprised of protein, lipid, carbohydrate or combination thereof or may be a "virus like particle" which mimics a virus in structure but lacks replicative capability.

As used herein an "immunostimulant" may refer to an adjuvant, including but not limited to Freunds adjuvant, inorganic compounds (e.g., alum, aluminum hydroxide, aluminum phosphate, calcium phosphate hydroxide), mineral oil (e.g., paraffin oil), bacterial products (e.g., killed bacteria, Bordetella pertussis, Mycobacterium bovis, toxoids), nonbacterial organics (e.g., squalene, thimerosal), detergents (e.g., Quil A), plant saponins from quillaja, soybean, polygala senega, cytokines (e.g., IL-1, IL-2, IL-12), and food Based oil (e.g., adjuvant 65).

A used herein the term "domain", when used herein to describe the domains of flavivirus envelopes, refers to structural domains as characterized in crystal structures (e.g., crystal structures for tick borne encephalitis and Japanese encephalitis viruses (2, 3)).

"Neural and neurologic proteins," as used herein, refers to proteins within the human proteome, which have been identified as having a function in the nervous system in development or function. Included among such proteins, but not limited to these examples, are those which have the term neural, neuron, neuronal, neurologic, neurotropic, neurotropin, neuropeptide, neurogenic, glial, synaptic, and neurite in their curation at Uniprot (www.uniprot.org). Proteins are described by their Uniprot identifies in the tables included herein. Glycoprotein M6A and Glial fibrillary acidic protein are also included herein. While described by use of the identifiers for human proteins the defined term is intended to also include close homologues from other species.

"Microencephaly," as used herein describes a condition of fetuses and neonates in which part or all of the brain is absent and the cranium is reduced in size at birth.

"Guillain Barré syndrome," abbreviated as GBS, as used herein refers to a complex of symptoms, which include peripheral neuropathy affecting motor, sensitive and autonomic nerves and spinal roots causing acute, or subacute, progressive motor weakness sometimes advancing to respiratory paralysis. GBS is an autoimmune disease and has been noted following various infections, including influenza, *Campylobacter,* dengue and Zika virus. Although symptomatology is shared, GBS may have various pathogeneses, with different immune responses directed to different self proteins.

"Flaviviruses" as used herein refers to the taxonomic group of viruses of that name (4). Abbreviations are used for several flaviviruses as follows Japanese encephalitis JEV, West Nile Virus WNV, Tick Borne encephalitis TBEV, yellow fever YF, dengue DEN.

"Microbiocide" as used herein refers to a composition which may be a peptide, polypeptide or enzyme or small molecule which acts on a microorganism to inhibit its replication or cause lethal structural damage. Microbiocides include but are not limited to bactericides, virucides, and fungicides.

"Core peptides" or "core pentamer" when used herein refers to the central 5 amino acid peptide in a predicted B cell epitope sequence. Said B cell epitope may be evaluated by predicting the binding of across a series of 9-mer windows, the core pentamer then is the central pentamer of the 9-mer window

"Target biopharmaceutical" as used herein refers to an original biopharmaceutical or a first iteration of a biopharmaceutical product which may be improved to reduce risk and increase safety by removal or mutation of a mimic epitope.

As used herein the term "arthritis" refers to any pathologic process resulting in inflammation, degeneration, pain or stiffness of the joints.

As used here in the term "alpha synucleinopathy", or synucleinopathy, refers to a disease characterized by abnormal processing or accumulation of alphasynuclein protein in neurons. Alphasynucleinopathy includes Parkinson's disease, dementia with Lewy bodies, and multiple system atrophy.

As used herein the term "parasite" refers to both endoparasites and ectoparasites. Endoparasites include protozoa, and multicellular parasites such as helminths; ectoparasites include arthropods such as ticks and lice. Antigens derived from said parasites which elicit antibodies may include both structural and physiologic proteins, and those proteins secreted by the parasites. In one particular instance, this includes the salivary proteins of ectoparasites.

### DESCRIPTION OF THE INVENTION

There is increasing awareness that autoimmune reactions are a major contributor to morbidity and mortality. This includes both autoimmunity mediated by the cellular immune response and autoimmunity mediated by antibody responses.

The present invention provides a method for prediction and identification of antibody mediated epitope mimicry, in which antibodies elicited by an exogenous antigen react with an epitope on a self-protein, i.e., one that is a normal constituent of the human proteome or other host proteome. As the outcome of such interactions may be adverse and may contribute to clinical disease, anticipating such reactions permits avoidance, design away in development of biotherapeutics and vaccines, and interventions to remediate antibody mediated mimic reactions.

In one embodiment therefore the present invention provides a process to identify epitopes on an exogenous antigenic protein which are B cell epitopes and to identify predicted B cell epitopes within proteins of the human proteome which carry the same pentamer amino acid motif. In some particular embodiments, said exogenous protein is present in a microorganism, including but not limited to, a virus, bacteria, fungus, parasite, or a toxin thereof, and said autoimmunity is a sequel to an infection or infestation. In one particular embodiment involving parasites the protein which generates an antibody response is the saliva of an ectoparasite. In yet other embodiments the exogenous antigen is found in the environment as a component of a food product or an allergen, or any other environmental protein to which a subject is exposed. In further embodiments, the exogenous protein is a component of a pharmaceutical product, including but not limited to a vaccine, prophylactic or therapeutic drug, either as the active biopharmaceutical constituent thereof or as an excipient. These examples of antigenic proteins are not considered limiting.

The protein in the human proteome bearing the B cell epitope to which said antibody binds, recognizing it as a mimic of the epitope which elicited the antibody, may have one of many different functions. In some instances, the target protein may have a neurophysiologic function, in other instances it may function in cardiovascular systems, including but not limited to endothelial permeability and clotting. In yet further embodiments, the target protein may have urophysiologic, dermatologic, endocrine, or gastrointestinal functions, may involve a particular group of enzymes, or any one of several other physiologic functions the impairment of which results in disease. In order to classify the potential mimics, a series of filters may be applied which comprise groups of key words used in curation of the proteins pertinent to the organ system or physiologic function of interest.

In yet other embodiments, the proteins known to be associated or affected in a given disease may be examined to identify their B cell epitopes and thus provide a panel against which specific pathogens or exogenous antigens may be filtered. For instance, as non-limiting examples, human proteins known to be associated with arthritis or Parkinson's disease, may be selected and a panel established against which matches in a protein from an infectious agent of interest may be cross checked. The stringency of selection and identification of the antibody targeted mimicry is determined by the percentage of the ranked probability of B cell binding, first in the protein which gives rise to the antibody, i.e. the exogenous protein and secondly in the host self protein. In a preliminary screening such levels of stringency may be set to select the top 25 % of B cell epitopes in the exogenous protein and the top 40% of B cell epitopes in the target protein. Such selection filters may be increased in stringency to select only the top 10% of the B cell epitopes in the exogenous protein and 25% of the target proteins B cell epitopes, or increased or decreased in stringency to whatever the operator deems to be an appropriate level of stringency. In particular embodiments, an additional selection criterion is to identify B cell epitopes in the exogenous protein which have closely juxtaposed peptides with high affinity MHC binding providing good T cell help. This is turn is conducive to generation of high antibody titers, immunoglobulin class switching and a higher chance of epitope mimicry occurring. In some instances, the B cell epitope in the exogenous protein is accompanied by peptides binding to one or more MHC alleles, however in yet other instances the adjacent peptides provide binding to most or all MHC alleles and at high affinity. This relationship will determine whether antibody mimicry affects all subjects, or occurs only sporadically in those subjects carrying a particular MHC allele. The MHC binding may determine the familial associations of an autoimmune disease.

In some embodiments, the process described herein for identifying antibody mediated epitope mimicry may be applied in the design of a vaccine, or a biopharmaceutical, where targeting antibodies to self-proteins is undesirable. Following identification of epitope mimics which may cause such adverse effects, a vaccine may be designed to mutate or delete said mimics and focus the response only on the desirable antibody eliciting epitopes. The approach described in this invention may also be employed to evaluate a novel biopharmaceutical to identify whether it may have epitopes which will elicit self reacting antibodies. Such an application of the methods can reduce risk, and hence cost and time, and increase safety in the design of a biopharmaceutical because multiple iterations can be evaluated *in silico* before a clinical trial.

In some particular embodiments once a target protein of autoimmunity is identified *in silico,* the information can be used to determine if a particular animal species will form a good preclinical disease model. This is by allowing a target protein to be compared in a proposed animal species for its identity and hence determine if it is representative of the protein in humans. This will aid in the selection of an animal model which can best represent the human species. In one particular embodiment, therefore, the proteome of the mouse, based on the C57BL6 inbred strain is used as a comparator to determine which exogenous antigens share B cell epitope mimics with the mouse proteome. In this embodiment, the B cell epitopes of the murine proteome are pre-computed and a set of key word based filters established for the mouse proteome to enable filtering of epitope mimic matches of infectious organisms or environmental or other exogenous antigens with murine proteins that have neurologic, cardiovascular, and other sets of functional groupings. As those skilled in the art will appreciate, as the complete proteomes of other important domestic and laboratory animals are sequenced and annotated, it will become increasingly possible to match epitope mimics in other animal models of interest, such as non-human primates, and thus the example of murine model is not considered limiting.

In some particular embodiments, the comparison of predicted epitope mimics can shed light on the differences in clinical manifestations arising from infections by different strains or isolates of a given infectious organism, whether viral or bacterial or of other taxonomies. In one particular embodiment, identifying the peptide in the exogenous protein which leads to the immune response and antibodies which ultimately are self-reactive, enables the use of said mimic peptide as a component of an apheresis device in which the peptide binds the antibodies which would otherwise bind to the self-protein.

The methods described herein provide a tool for understanding and responding to antibody mediated autoimmune diseases. It will be apparent to those skilled in the art that the applications are not limited to one autoimmune disease and can be applied to a wide variety of autoimmune diseases and thus none of the examples are considered limiting.

Historically, it was generally assumed that the immune system does not recognize self proteins. We are increasingly recognizing there is an active interaction and overlap between the immune recognition of self and exogenous antigens. There are many instances where the cellular immune system fails to differentiate between recognition motifs, comprising a small group of amino acids occurring in a pathogen, from the same small group of amino acids where they occur in a self-protein (see, e.g., PCT/US2015/039969, the entire contents of which is incorporated herein by reference; see also Bremel et al (5)). However, another sphere of interactions occurs between exogenous proteins, including but not limited to pathogens, and the self-proteins of the human proteome; this is antibody mediated epitope mimicry. Antibody mediated epitope mimicry occurs when an antigenic exogenous protein elicits antibodies that also recognize and bind to an epitope on a self-protein. The binding of an antibody to a self-protein may then inhibit or compromise the functionality or processing of the self-protein. In some instances, the spectrum of clinical signs following microbial infection may be as much, or even more, dependent on the effect of the antibodies elicited by the infectious agent binding to the host proteins, as it is due to the primary microbial replication. Antibody mediated autoimmune diseases, in which the antibodies generated in response to one epitope, on a microorganism or other exogenous protein, but which then bind to a self -protein are notoriously difficult to diagnose, and it can be very difficult to pin down the exact mechanism of pathogenesis leading to the clinical signs. The processes described in the present invention apply bioinformatics tools to greatly facilitate understanding of such antibody mediated autoimmune responses and to permit them to be identified and recognized rapidly. When applied to a biotherapeutic or vaccine synthetic protein, the *in silico* screening tools provided herein enable evaluation of potential mimics, thereby reducing the time, costs, and most importantly risks, of waiting for clinical trials. When applied to antibody mediated mimicry arising from natural infection or exposure to an antigenic exogenous proteins, the tools described herein enable diagnosis of the pathways of disease and hence provide information critical to designing interventions.

In a related mechanism, the presence of linear B cell epitopes may also reflect the propensity for a protruding and polarized peptide to bind other ligands. In other words, the presence of matching B cell epitopes is simply an indicator of potential interference or blocking between other ligands. The basic components of antibody mediated autoimmune disease are as follows.

An exogenous protein, which may be from any one of a wide range of sources, as noted below, has a group of amino acids which form a B cell epitope. The epitope binds to a B cell and causes that cell to generate antibodies. The antibodies thus generated recognize a B cell epitope on a self-protein and preferentially bind to it, impeding the function or processing of the protein.

The exogenous protein may be a microorganism, including but not limited to a virus, a bacteria, a parasite, a fungus, or a toxin generated by a microorganism. These taxonomic descriptions are intended to be descriptive examples, and not considered limiting. It may be a synthetic or attenuated microbial protein intended to be introduced into the host as a vaccine. In other embodiments the exogenous protein may be a biopharmaceutical protein, such as a monoclonal antibody or a monoclonal antibody-based product, comprising part or all of an immunoglobulin. In some particular instances an excipient incorporated in a pharmaceutical formulation may be the source of the exogenous protein which elicits antibodies. In some embodiments the exogenous protein may be a toxin. In yet others it may be an allergen or another environmental protein. Such examples provide orientation but are not intended to limit the definition of exogenous protein.

The titer of antibodies elicited by the exogenous protein will in part determine how much of the host protein is bound by antibodies, and to what degree its function is compromised, and hence the degree of clinical effect. If a B cell epitope is immediately flanked by a peptide of high MHC affinity, the chance of a strong T helper effect is increased (6). T cell help is also essential to bring about immunoglobulin class switch. The occurrence of IgG and not just IgM may be a deciding factor in antibody mimicry. For instance IgG will cross the human placental and may bind to proteins in the fetus whereas IgM will not. MHC binding peptides, taken up at the B cell synapse at the time ofB cell epitope binding, will be those most likely to be presented by the B cell to T cells and elicit T cell help (7, 8). Hence those peptides close to the B cell epitope will be those most likely to provide specific help. Therefore, a further consideration in identifying B cell epitopes which may elicit antibodies that bind to antibody mimics is to also determine if there is an adjacent MHC binding peptide. In some cases, such MHC binding may be of high affinity for many alleles of MHC II. In other instances only a few alleles provide such T cell help. Therefore, a further aspect of the process described herein is to identify which alleles may lead to most risk of developing an antibody mediated autoimmunity. In this way a sub population of individual subjects who are most at risk can be identified. Importantly, this relationship is between the host MHC and the exogenous protein. It is unlikely that in the host protein that is the target of the antibody binding that the MHC binding plays any role in determining if the antibody will bind.

At some minimal level, such antibody mediated "off target binding" to mimics on self proteins occurs very frequently, is the norm, and occurs across the diversity of antibodies that a subject generates. This is inevitable given the relatively narrow number of different options in specificity. If a pentamer is considered as the core of the B cell epitope then only 20⁵ or 3.2 million possibilities of different configuration exist. If the recipient epitope on the host protein is also a pentamer, comprising 3.2 million possibilities then the chance of a match is 20⁵×20⁵ or approximately 1 in 10¹³. Whether such binding has any clinical relevance is dependent on the titer of antibody, and thus how much of the host protein gets bound, the isotype of the immunoglobulin, with what affinity binding occurs, and in particular, what is the function of the host protein. Most of the time such binding has no clinical impact whatsoever; it is diverse, it is at low levels and transient, and it impacts proteins which are not on a critical metabolic path. Where high titer antibody and essential host protein function both occur, the clinical signs may become evident. This may be the case following a burst of antibody production after an acute infection or exposure.

There are many examples in which antibody mediated mimicry has been described and is well known to the art. There is rapidly increasing awareness of the role of antibodies in autoimmunity. Among the most recently reported antibody mediated autoimmune interactions are a relationship between seropositivity to West Nile virus and myasthenia gravis (9), interaction between certain antibodies to herpes simplex virus and alphasynuclein, a critical component of the Lowey bodies of Parkinson disease (10) and the demonstration that antibodies to dengue cross react with von Willebrand factor (*11*). Further, enteroviruses have been shown to exert neuropathologic effects through antibody mediated binding (12).

Guillain Barré (GBS) is a clinical syndrome of multiple autoimmune etiologies, which involve idiopathic peripheral neuropathy leading to acute flaccid paralysis. The clinical course of GBS varies; 25% of patients require artificial ventilation (days to months), 20% of patients remain non ambulatory at 6 months and 3-10% of patients die despite standard of care treatment. In medical care environments where ventilatory support is not readily available, GBS mortality is often much higher. Globally, annual GBS incidence is estimated at 1.1 to 1.8/100,000/year, of which approximately 70% appear associated with antecedent infectious disease and the product of antibody mimicry. Other cases of GBS arise from cell mediated autoimmunity. Infections leading to GBS are typically gastrointestinal or respiratory. *Campylobacter jejeuni* infections are among the most common infections which lead to GBS. This is seen as a sequel especially after severe *C. jejeuni* diarrhea (*13*, *14*)*..* As we show in the examples cited below, epitope mimicry may play a wider and under recognized role in pathogenesis.

A particular embodiment in which antibody mediated autoimmunity may cause additional problems is during pregnancy when the fetus is also exposed to the antibodies. The human placenta, unlike that of many species, is very efficient in transfer of IgG to the fetus. Placental transfer of immunoglobulins to a fetus prior to blood brain barrier formation can be detrimental to the fetus. The human placenta facilitates the transfer of IgG, but not IgM, mediated by FcRn and increasing during the second trimester (*15*). IgG1 and IgG4 are most efficiently transferred. Approximately 10% of maternal IgG is thought to pass into the fetal circulation, starting as early as week 13 (*16*). The fetal blood brain barrier (BBB) is not fully developed until the third trimester and indeed may preferentially transfer proteins to the fetal brain (*17, 18*). Thus, the literature suggests that the developing CNS is exposed to maternal antibodies in the first two trimesters. There is clearly precedent for autoimmune diseases caused by the transplacental passage of antibody, including pemphigus, myasthenia gravis, and lupus *(16, 17, 19*). Transplacental antibody has also been implicated in autism spectrum disorders (*20*). In dengue infection maternal antibodies transfer to the fetus, achieving a level determined by maternal antibody titer (*21*). Fetal titer may actually exceed maternal titer suggesting an active transfer process without direct adverse effects on the fetus being reported until ADE following post-natal dengue infection (*22*). In one embodiment, therefore, this invention addresses the understanding of autoimmunity in the fetus arising from maternal antibodies and the detection of immunogens that can result in antibodies in the mother that cross the placenta. Antibody binding proteins critical to fetal development at key time windows in development may result in teratogenic defects. Understanding this antibody transfer pathway is essential to development of products, including vaccines and biotherapeutics, intended to be administered to pregnant women.

Cytomegalovirus and rubella are both viral infections which cause congenital abnormalities, in some cases evident at birth in other cases developing during childhood. While in both cases virus may be isolated from the fetus and there is no question that direct pathology arises from such viral replication, there is still a lack of understanding of the pathogenesis of much of the teratologic effect seen (*23, 24*). In one embodiment of the present invention, the role of antibody mediated epitope mimicry is shown in which antibody to the membrane proteins of cytomegalovirus are predicted to generate antibodies which are reactive with among others the NAV2 neural navigator protein needed for neurite elongation in the early fetal development (*25*, *26*). Notably secondary infections with cytomegalovirus are associated with a rise in antibodies membrane protein glycoprotein B. In another embodiment we show that similar antibodies are generated in response to rubella envelope protein 2. Remarkably it has been noted that babies born with more sever sequelae of rubella *in utero* infection have higher titers of antibody to rubella (*27-29*)

This is similar to the predicted antibody mimicry following Zika virus infection (see, e.g., copending applications 62/292,964; 62/290,616 and 62/286,779, each of which is incorporated by reference herein in its entirety). Zika virus has a pentamer epitope in its envelope protein Domain III that is predicted to generate antibodies which also bind to proNeuropeptide Y and, in Asian Pacific strains also has a Domain I envelope protein epitope, antibodies to which are also predicted to bind NAV2 and affect fetal growth and also impact retinal development, leading to the combination of clinical signs now recognized as Zika fetal syndrome. It will be apparent to those skilled in the art that grossly evident fetal malformation may be the "tip of the iceberg" and that lower titers of antibody transferred transplacentally may compromise fetal development to a lesser degree, leading to signs, such as the deafness, that may appear years after birth of a child exposed to rubella infection in utero, or which may manifest themselves as behavioral changes.

It is evident therefore that there is great need to be able to identify with greater precision and efficiency the exact pathways leading to autoimmunity in order to determine methods of intervention and to avoid off-target adverse responses in the development of biotherapeutics.

In one embodiment therefore, the present invention addresses researching the pathogenesis of autoimmune diseases to identify the epitope mimics leading to antibody mediated autoimmune responses in order to design interventions and avoid safety risks. This information can then be used in the design of vaccines and therapeutics in which key mimic epitopes are mutated out. In a parallel embodiment it then follows that having created a new epitope amino acid motif, by mutation of a known epitope mimic, that the process must be repeated and the replacement pentamer motif must be checked against the proteome to make sure a further new cross reactive epitope mimic motif has not been created in the process.

In a particular embodiment, the present invention addresses screening of a new biotherapeutic to identify potential epitope mimics. The invention provides a rapid way in which many biotherapeutics in early development can be screened *in silico* to anticipate adverse reactions which can arise from antibody mediated autoimmunity, and to identify epitope mimics. A particular reason why this is a major savings in cost and time is that the invention enables screening against the whole proteome of the human, and all isoforms of any protein therein. As not all isoforms occur in any single individual it is possible that early clinical trials would not detect all possible adverse effects from epitope mimics. Further *in silico* analysis by the methods described herein allows evaluation for all MHC alleles, identifying those individuals most likely to generate a high titer of antibody due to the T cell help. A further motive to apply the invention described herein, is that animal models may not detect epitope mimic effects. This is because, in addition to the MHC differences between hosts, where the host protein to which antibodies bind differs by as little as a single amino acid in the animal model species, there may be no antibody mediated mimic effect detected in the animal model. Thus a potential adverse effect could go unnoticed until the biotherapeutic or vaccine enters clinical trials in humans.

Another embodiment of the present invention is to assist in designing therapies for antibody mediated autoimmune diseases. If the peptide that forms the target of the antibody binding the host protein is identified, then this peptide can be deployed to bind the problem antibody. This could be done by administration of the peptide to the subject in a pharmaceutical preparation, or *ex vivo* by inclusion of the peptide in a plasmapheresis system, or similar exchange system, to bind and remove the antibodies of concern.

Given the differences between the proteomes of human and other species the occurrence of epitopes in the host proteome matching that of a given exogenous antigen will be species dependent. There is ongoing concern about the inability of animal models to accurately predict the pathogenesis of diseases in humans. This is a particular concern when animal models are used to assess the safety of therapeutics or vaccines in an animal model, only to find that they do not fully replicate what is seen in human clinical trials. In another embodiment therefore the present invention examines the differences in epitope mimics between human and murine models. As other species may be used as animal models and as the proteomes are fully annotated the example of the murine model can be extended to other species of interest. Furthermore having used the invention described herein to identify potential epitope matches in the human, using this peptide sequence as guidance, the presence or absence of the same epitope mimics in other species of interest such as non-human primates can be assessed by interrogating for the identical peptide in the proteome of that species.

The processes we describe herein utilize the ability to predict probable B cell epitopes and to predict MHC binding affinity, which we have described in copending application PCT US2011/029192, incorporated herein by reference in its entirety. The present invention then provides an appropriate set of selection filters to establish a stringent selection system, and a system for interrogating the large human proteome database for matches. The stringency filters are applied at two levels. On one hand it is necessary to determine which of the antibodies elicited by a linear epitope in an exogenous protein are most likely to generate a strong B cell response, and which are likely to be made at high titer. The algorithms developed permit an initial screen, for instance using the 25% linear epitopes in the exogenous protein most likely to elicit antibodies. This filter can be made less stringent, or more stringent, to select only 10% or only 5% of the probable B cell epitopes. In a preferred embodiment, the initial screen of potential antibody binding sites in the proteome protein would typically define the top 40% most probable antibody binding sites in each protein of the human proteome, but likewise can be set to be more or less stringent. This selection criterion can be changed to the top 30% or 20% as desired. The appropriate cutoff will depend on the circumstances; very low levels of mimic binding antibody may be problematic in the fetus whereas much more stringent cutoffs may be adequate for adults.

The following clauses provide an illustration of the above embodiments.

### CLAUSES

1. A method for identifying epitope mimic peptides which elicit antibodies that bind to a host protein, comprising:
   assembling a database of all proteins in the host proteome;
   assigning a curation to each protein based on its reported function;
   computing the probable B cell epitopes in each protein of said host proteome database that is curated by function;
   identifying the core peptide of said probable B cell epitopes in each protein of the host proteome;
   assembling a database of said core peptides of said probable B cell epitopes from each protein of the host proteome in a computer readable medium;
   entering a sequence of a protein of interest into a computer with access to said database;
   computing probable B cell epitopes in the protein of interest;
   identifying the core peptide of said probable B cell epitopes in said protein of interest;
   comparing said core peptide of said probable B cell epitope in a protein of interest to the core peptides contained in said database of peptides from the host proteome;
   identifying core peptides in predicted B cell epitopes in said protein of interest which are identical to core peptides in predicted B cell epitopes in one or more proteins of the host proteome; and
   identifying the function of the host proteome proteins which comprise the identical core peptides matching the core peptides of the protein of interest.
2. The method of clause 1, wherein said host proteome is selected from the group consisting of a human proteome and a murine proteome.
3. The method of clause 1, wherein said host proteome is a non-human primate proteome.
4. The method of any of clauses 1 to 3, wherein the probable B cell epitope in said protein of interest is in the top 25% most probable B cell epitopes in said protein of interest.
5. The method of any of clauses 1 to 4, wherein said probable B cell epitope in said protein of interest is in the top 10% most probable B cell epitopes in said protein of interest.
6. The method of any of clauses 1 to 5, wherein the probable B cell epitope in said host proteome protein is in the top 40% most probable B cell epitopes in said protein of interest.
7. The method of any of clauses 1 to 6, wherein the probable B cell epitope in said host proteome protein is in the top 25% most probable B cell epitopes in said protein of interest.
8. The method of any of clauses 1 to 7, wherein the core peptide in said probable B cell epitope in said protein of interest comprises a sequence of five contiguous amino acids.
9. The method of any of clauses 1 to 8, wherein the core peptide in said probable B cell epitope in said host proteome protein of interest comprises a sequence of five contiguous amino acids.
10. The method of any of clauses 1 to 9, wherein the database of core peptides in said data base of host proteome proteins is searched by application of a list of keywords to select to a subset of peptides with functions of interest.
11. The method of clause 10, wherein said key words define a group of proteins with neurophysiological function.
12. The method of clause 10, wherein said key words define a group of proteins with enzymatic function.
13. The method of clause 10, wherein said key words define a group of proteins which function in blood clotting and vascular permeability.
14. The method of clause 10, wherein said key words define a group of proteins which function in inflammation.
15. The method of clause 10, wherein said key words define a group of proteins which have a function in arthritis.
16. The method of any of clauses 1 to 9, wherein the database of core peptides in said data base of host proteome proteins is searched by application of a list of keywords to select to a subset of peptides with association with development of a specific disease syndrome.
17. The method of clause 16, wherein said key words define a group of proteins which are associated with Parkinson's disease and related alpha synucleinopathies.
18. The method of any of clauses 1 to 17, which further comprises identifying those probable B cell epitopes in the protein of interest which are located within about 10 to 20 amino acids of a peptide with predicted high binding affinity for one or more MHC II molecule.
19. The method of clause 18, further comprising identifying a subpopulation of host subjects that is most at risk of adverse effects arising from antibody mediated autoimmunity.
20. The method of any of clauses 1 to 19 wherein said protein of interest is a microbial protein.
21. The method of clause 20, wherein said microbial protein is selected from the group consisting of a virus protein, a bacteria protein, a parasite protein, a fungus protein, and a microbial toxin.
22. The method of any of clauses 1 to 19, wherein said protein of interest is an antigen binding protein.
23. The method of any of clauses 1 to 19, wherein said protein of interest is a biopharmaceutical protein.
24. The method of any of clauses 1 to 19, wherein said protein of interest is a vaccine.
25. The method of any of clauses 1 to 19, wherein said protein of interest is a pharmaceutical preparation.
26. The method of any of clauses 1 to 19, wherein said protein of interest is a food protein.
27. The method of any of clauses 1 to 19, wherein said protein of interest is an environmental protein.
28. The method of any of clauses 1 to 27, further comprising the step of synthesizing a mutant version of said protein of interest, wherein said core peptide in said protein of interest is mutated to abrogate said match to a core peptide in the host proteome.
29. A non-transitory computer readable medium comprising a database of pentamer peptides which are found in proteins of a host proteome, wherein said pentamer peptides are curated by function and are the core peptides of a predicted B cell epitope.
30. The non-transitory computer readable medium of clause 29 wherein said function is selected from the group consisting of neurophysiologic, endocrine, cardiovascular, respiratory, hormonal, skin and mucosal health, or musculoskeletal functions.
31. A non-transitory computer readable medium comprising a database of pentamer peptides which are found in proteins of a host proteome, wherein said pentamer peptides are associated with a defined set of disease conditions and that are the core peptides of a predicted B cell epitope.
32. The non-transitory computer readable medium of clause 31, wherein said defined set of disease conditions are selected from the group consisting of alpha synucleopathies.
33. The non-transitory computer readable medium of any of clauses 29 and 31,
   wherein said host proteome is selected from the group comprising a human proteome and a murine proteome.
34. The non-transitory computer readable medium of any of clauses 29 and 31,
   wherein said host proteome is a non-human primate proteome.
35. A method of selecting an animal model to study a disease or to test a vaccine or pharmaceutical product comprising:
   analyzing a protein of interest by the method of any of clauses 1 to 28; and
   comparing the epitope mimics identified in the host proteome of the animal species of interest with those of the human proteome.
36. A method of selecting an animal model to study a disease or to test a vaccine or pharmaceutical product comprising:
   analyzing a protein of interest by the method of any of clauses 1 to 28; and
   determining by comparison with epitope mimic matches identified in the human proteome which other species have identical core peptides in their proteome proteins which are homologous in function to those in the human proteome that carry the core peptides matching said core peptides in said protein of interest.
37. A method of diagnosing an autoimmune disease comprising:
   identifying epitope mimic peptides which elicit antibodies that bind to a human protein by the method of any of clauses 1-2 and 4-28;
   providing a synthetic protein derived from the human protein which comprises said epitope mimic peptides;
   contacting said synthetic protein with serum harvested from a subject at risk of being affected by an autoimmune disease; and
   identifying the presence of antibodies with specific binding to mimic epitopes in said synthetic protein.
38. A method of diagnosing an autoimmune disease wherein antibody mediated mimicry is suspected, comprising:
   harvesting a serum sample from a subject suspected of being affected by an autoimmune disease;
   contacting said serum sample to a microarray of peptides and identifying peptides which bind to antibodies in said serum; and
   analyzing the peptides thus identified by the methods of any of clauses 1-2 and 4-28 to identify which of said peptides function as epitope mimic peptides.
39. A method of producing a vaccine comprising:
   obtaining one or more gene or amino acid sequences encoding one or more components of vaccine that have been mutated to remove one or more epitope mimics or alter one or more epitope mimics to non-mimics as compared to the corresponding wild type sequences, said epitope mimics identified by a process comprising:
   assembling a database of all proteins in the human proteome;
   assigning a curation to each protein based on its reported function;
   computing the probable B cell epitopes in each protein of said human proteome database wherein said proteins are curated by function;
   identifying the core peptide of said probable B cell epitopes in each protein of the human proteome;
   assembling a database of said core peptides of said probable B cell epitopes from each protein of the human proteome in a computer readable medium;
   entering sequences encoding one or more components of vaccine into a computer with access to said database;
   computing probable B cell epitopes in said sequences encoding one or more components of vaccine;
   identifying the core peptide of said probable B cell epitopes in said sequences encoding one or more components of vaccine;
   comparing said core peptides of said probable B cell epitopes in said sequences encoding one or more components of vaccine to the core peptides contained in said database of peptides from the human proteome;
   identifying core peptides in predicted B cell epitopes in said sequences encoding one or more components of vaccine which are identical to core peptides in predicted B cell epitopes in one or more proteins of the human proteome;
   identifying the function of the human proteome proteins which comprise the identical core peptides matching the core peptides of sequences encoding one or more components of vaccine; and
   synthesizing components for a vaccine by a method selected from the group consisting of a) expressing said one more sequences encoding one or more components of vaccine that have been mutated to remove one or more epitope mimics or alter one or more epitope mimics to non-mimics as compared to the corresponding wild type sequences in a host cell to produce mutated proteins, and b) synthesizing nucleic acid segments encoding said one or more recombinant sequences encoding one or more components of vaccine that have been mutated to remove one or more epitope mimics or alter one or more epitope mimics to non-mimics as compared to the corresponding wild type sequences.
40. The method of clause 39, further comprising formulating said mutated proteins or nucleic acid segments with a pharmaceutically acceptable carrier.
41. A method of producing a biopharmaceutical protein comprising:
   obtaining one or more gene or amino acid sequences encoding a biopharmaceutical protein that has been mutated to remove one or more epitope mimics or alter one or more epitope mimics to non-mimics as compared to the corresponding target biopharmaceutical protein sequence, said epitope mimics identified by a process comprising:
   assembling a database of all proteins in the human proteome;
   assigning a curation to each protein based on its reported function;
   computing the probable B cell epitopes in each protein of said human proteome database wherein said proteins are curated by function;
   identifying the core peptide of said probable B cell epitopes in each protein of the human proteome;
   assembling a database of said core peptides of said probable B cell epitopes from each protein of the human proteome in a computer readable medium;
   entering sequences encoding said target biopharmaceutical protein into a computer with access to said database;
   computing probable B cell epitopes in said sequences encoding said target biopharmaceutical protein;
   identifying the core peptide of said probable B cell epitopes in said sequences encoding said target biopharmaceutical protein;
   comparing said core peptides of said probable B cell epitopes in said sequences encoding said target biopharmaceutical protein to the core peptides contained in said database of peptides from the human proteome;
   identifying core peptides in predicted B cell epitopes in said target biopharmaceutical protein which are identical to core peptides in predicted B cell epitopes in one or more proteins of the human proteome;
   identifying the function of the human proteome proteins which comprise the identical core peptides matching the core peptides of said target biopharmaceutical protein; and
   synthesizing said mutated biopharmaceutical protein by expressing said biopharmaceutical that have been mutated to remove one or more epitope mimics or alter one or more epitope mimics to non-mimics as compared to the corresponding target biopharmaceutical protein sequence.
42. The method of clause 41, further comprising formulating said mutated biopharmaceutical protein with a pharmaceutically acceptable carrier.
43. The method of any of clauses 39 to 42, wherein the probable B cell epitope in said vaccine component or biopharmaceutical protein is in the top 25% most probable B cell epitopes in said protein of interest.
44. The method of any of clauses 39 to 43, wherein said probable B cell epitope in said vaccine component or biopharmaceutical protein is in the top 10% most probable B cell epitopes in said protein of interest.
45. The method of any of clauses 39 to 44, wherein the probable B cell epitope in said human proteome protein is in the top 40% most probable B cell epitopes in said vaccine component or biopharmaceutical protein.
46. The method of any of clauses 39 to 45, wherein the probable B cell epitope in said human proteome protein is in the top 25% most probable B cell epitopes in said vaccine component or biopharmaceutical protein.
47. The method of any of clauses 39 to 46, wherein the core peptide in said probable B cell epitope in said vaccine component or biopharmaceutical protein comprises a sequence of five contiguous amino acids.
48. The method of any of clauses 39 to 47, wherein the core peptide in said probable B cell epitope in said human proteome vaccine component or biopharmaceutical protein comprises a sequence of five contiguous amino acids.
49. The method of any of clauses 39 to 48, wherein the database of core peptides in said data base of human proteome proteins is searched by application of a list of keywords to select to a subset of peptides with functions of interest.
50. The method of clause 49, wherein said key words define a group of proteins with neurophysiological function.
51. The method of clause 49, wherein said key words define a group of proteins with enzymatic or endocrine function.
52. The method of clause 49, wherein said key words define a group of proteins which function in blood clotting and vascular permeability.
53. The method of clause 49, wherein said key words define a group of proteins which function in inflammation.
54. The method of clause 53, wherein said key words define a group of proteins which have a function in arthritis.
55. The method of any of clauses 39 to 48, wherein the database of core peptides in said data base of host proteome proteins is searched by application of a list of keywords to select to a subset of peptides with association with development of a specific disease syndrome.
56. The method of any of clauses 39 to 55, further comprising identifying those probable B cell epitopes in the vaccine component or biopharmaceutical protein which are located within 10-20 amino acids of a peptide with predicted high binding affinity for one or more MHC II molecule.
57. The method of any of clauses 39 to 40 and 43 to 56, wherein said sequences encoding one or more components of vaccine are microbial protein sequences.
58. The method of clause 57, wherein said microbial protein sequences are selected from the group consisting of virus, bacteria, parasite, fungus, and microbial toxin sequences.
59. The method of clauses any of 41 to 58, wherein said target biopharmaceutical protein is selected from the group consisting of an antigen binding protein, a receptor protein and a signaling protein.
60. The method of any of clauses 39, 40, and 43 to 58, further comprising administering said one or more components of vaccine that have been mutated to remove one or more epitope mimics or alter one or more epitope mimics to non-mimics as compared to the corresponding wild type sequences to a subject in need thereof.
61. The method of any of clauses 41, 42 to 55 and 59 further comprising administering said biopharmaceutical that have been mutated to remove one or more epitope mimics or alter one or more epitope mimics to non-mimics as compared to the corresponding target biopharmaceutical protein sequence to a subject in need thereof.
62. A method of evaluating a biopharmaceutical protein comprising:
   identifying the presence in said biopharmaceutical protein of probable B cell epitopes and core peptides contained therein;
   determining which of said core peptides of said probable B cell epitopes match core peptides of probable B cell epitopes in a human proteome; and
   identifying the function of the proteins thus matched in the human proteome.
63. The method of clause 62, further comprising the step of synthesizing a mutant version of said biopharmaceutical protein, wherein said core peptide in said biopharmaceutical protein is mutated to abrogate said match to a core peptide in the human proteome.
64. The method of clauses 62 and 63 further comprising identifying the spectrum of possible side effects arising from the binding of antibody elicited by said vaccine or biopharmaceutical protein to the B cell epitope in a human proteome protein.
65. A method of evaluating potential side effects of a pharmaceutical protein comprising:
   determining the core peptides located in the probable B cell epitopes of said pharmaceutical proteins;
   interrogating the database of any of clauses 29 and 31 to determine if the core peptides of said pharmaceutical protein are present; and
   preparing a report identifying a spectrum of possible pathophysiologic interactions of the biopharmaceutical proteins.
66. A method of attenuating the pathology of a microorganism comprising:
   identifying core peptides within probable B cell epitopes of said organism which elicit antibodies that bind to a matching core peptide in a B cell epitope of host protein; and
   mutating or removing said matching core peptide in the microorganism.
67. A method of treating a subject affected by an autoimmune disease comprising:
   applying the method of any of clauses 1 to 28 to identify an epitope mimic peptide;
   providing said peptide as an antibody binding substrate; and
   incorporating said antibody binding substrate into an apheresis system.

The following examples provide illustrations of the above embodiments.

### Examples

### Example 1: A process for detection of antibody mimics

Building on the methods described in PCT US2011/029192, incorporated herein by reference, which enable the prediction of a B cell epitope in a protein of interest we established a work flow for identifying core pentamer peptides in a source protein of interest, for instance a viral protein, and then detecting matches of this peptide in a human protein in which B cell epitope core pentamers have been previously computed. Proteins in the human proteome are curated as to their functions based on information in UniProt (30). This allows a set of search terms to be applied to extract sets of proteins from the overall proteome database based on key words.

In computing the predicted probable B cell epitopes, a sliding 9-mer window is used. For comparative purposes the pentamer central core of the 9-mer is used. A pentamer is chosen because, not only does it provide a very stringent filter, but it corresponds to the area needed to engage the paratope of an antibody (31). While an antibody may engage a smaller number of amino acids, as few as 3 may be sufficient, it was determined by experimentation that using a pentamer as the core peptide provided a filter with sufficient stringency to identify matches to a meaningful number of human proteins. While B cell epitopes may be conformational, comprising amino acids in different strands of a sequence that are juxtaposed by folding, the simplest form ofB cell epitope is a linear sequence. Therefore pentamer motifs analyzed in identification of mimic matches may be linear or comprise conformationally juxtaposed amino acids brought together by folding.

To implement the search for matches between a protein of interest and the human proteome we implemented the following workflow, described here as for a viral protein but identically applicable to any protein of interest.
a. A database was precomputed to identify every sequential pentamer peptide in the human proteome. For this we use all proteins available on UniProt which comprises multiple isoforms of many proteins, in total >88,000 proteins. This generated a set of >34 million individual pentamers identified to source protein.
b. The viral proteins of interest are analyzed using previously described methods (see, e.g., PCT US2011/029192) to compute predicted probability ofB cell epitopes (BEPIs) and predicted MHC binding affinity for all sequential peptides. These predictions are standardized within protein. To compute BEPI probabilities a sliding window of 9-mers is used.
c. The viral and proteome datasets are joined to identify all viral pentamers which have matching pentamers in the proteome (Virus Proteome Match).
d. Three initial selection criteria are then applied to this selection to select:
   a. the top 25% probable BEPIs in the viral protein;
   b. the top 40% probable BEPIs in the proteome; and
   c. the human proteins with UniProt curations comprising certain keywords. In this case we utilized keywords comprising variations on the terms "neur", "glial", "myelin", "opt", and "synapt" (full list in Table A). Pentamers fulfilling all 3 criteria are declared to be predicted Virus Proteome Mimics. The stringency of these criteria can be increased to identify the highest probability mimics.

This process provides a highly selective set of filters. Any pentamer has a 20⁵ chance of occurrence (5 of 20 amino acids, a 1 in 3.2 million chance). When this probability is applied independently to both all the Zika viral proteins (a polyprotein of 3423 amino acids) and to the human proteome sets, there is a 3423/20⁵×20⁵ chance of a match, or 1 in 3.3×10¹⁰. This probability is then further reduced by application of the BEPI and keyword filters, but increases because the proteome comprises multiple similar isoforms of some proteins and some repetitive pentamers may occur in the virus. Progressively greater stringency may be applied to identify B cell epitopes most likely to elicit antibodies and most likely to become host targets of such antibodies.

In a further independent evaluation step of the viral proteins, the adjacency to probable BEPIs of predicted high affinity MHC binding of 15mers which may stimulate T cell help is determined. T cell help will not change antibody binding but may stimulate a higher titer. This selection process is discussed in further detail in the methods.

In the particular work flow described above we were interested in proteins of neurologic function. Therefore a key word list was assembled to identify proteins with these functions as shown in Table 1

**Table 1**

| Key words | |
|---|---|
| fibrinogen | neuromedin-b |
| fibroblast | neuron |
| fibrocystin | neuronal |
| fibrocystin-l | neuropeptide |
| fibronectin | neuropilin-2 |
| glial | neuroserpin |
| myelin | neurotrimin |
| myelin-associated | neurotrophic |
| neural | neurotrophin-4 |
| neural-specific | optineurin |
| neurexin | poliovirus |
| neurexin-1 | pro-neuropeptide |
| neurexin-1-beta | synapsin-2 |
| neurexin-2 | synaptic |
| neurexin-2-beta | synaptogyrin-1 |
| neurexin-3 | synaptonemal |
| neurexin-3-beta | synaptopodin |
| neurexophilin-1 | synaptosomal-associated |
| neurobeachin | synaptotagmin-1 |
| neurobeachin-like | synaptotagmin-10 |
| neuroblast | synaptotagmin-11 |
| neuroblastoma | synaptotagmin-14 |
| neuroblastoma-amplified | synaptotagmin-15 |
| neuro-d4 | synaptotagmin-3 |
| neurofibromin | synaptotagmin-4 |
| neurofilament | synaptotagmin-8 |
| neurogenic | synaptotagmin-like |
| neuroligin-2 | |

Similar lists may be developed to capture matches in proteome proteins with other functions, for instance the blood clotting cascade or pancreatic function. The key word list can be customized according to the circumstances and the protein of interest to focus the search for potential epitope mimics. In some cases the key word list may be selected based on the clinical signs of a particular disease, thus in jaundice a key word list would include the interactome of liver function.

Alternatively, the list of core pentamers located in BEPIs in the human proteome may be screened in its entirely to identify any protein in which a problematic mimic relationship may exist. This "all matches" approach allows the identification of B cell epitope mimics in proteins not identified by key word annotations in Uniprot. This is a particularly appropriate approach for any new biologic in development. It is also a desirable approach in comparing two exogenous proteins which differ only by one or two mutations, to determine what new mimics may have been created by mutation.

### Example 2: Ebola

Ebola is an infection characterized by hemorrhagic lesions in all major organs. We were interested to determine the possibility that antibody mimicry may be contributing to the pathogenesis of the clinical disease. Following the procedure laid out in Example 1 we computed the B cell epitope probabilities in the Ebola proteins of West Africa 2014, Mayinga, Bundibugyo and Musoke strains of Ebola Marbug virus. However, instead of searching for pentamer BEPI matches in the human proteome based on neurologic key words as illustrated in Example 1 we used a key word search comprising the terms shown in Table 2 below.

**Table 2**

| | |
|---|---|
| angio | plasmin |
| coag | plate |
| c-rea | throm |
| endoth | vasc |
| eryth | vaso |
| ferr | vwc2 |
| fibri | vwce |
| hema | vwde |
| heme | vwf |
| hemo | vwfa |
| plak | will |

This identified an array of pentamers in each of the key proteins that elicit the primary immune response which are indicative of antibody mediated mimicry which could contribute to the vascular and hemorrhagic signs. In Tables 3-6 we summarize those results for the 2014 West African isolates of Ebola virus and for the spike protein, small soluble glycoprotein, VP24 and VP40.

**Table 3. Predicted mimics in Ebola Spike protein. "Query pos" shows position in that protein.**

| In interests of space only one isoform of each protein is shown | | | | | |
|---|---|---|---|---|---|
| Proteome penta | SEQ ID NO: | query BEPI | BEPI intra protein | query pos | proteome curation |
| DPETN | 1 | -2.34 | -1.53 | 331 | DESP_HUMAN Desmoplakin OS_Homo sapiens GN_DSP PE 1 SV 3 |
| TPPAT | 2 | -2.31 | -2.77 | 422 | ATS18 _HUMAN A disintegrin and metalloproteinase with thrombospondin motifs 18 OS_Homo sapiens GN_ADAMTS 18 PE_1 SV_3 |
| TGPDN | 3 | -2.20 | -0.74 | 384 | NF2L1_HUMAN Isoform 2 of Nuclear factor erythroid 2-related factor 1 OS Homo sapiens GN_NFE2L1 |
| DSTAS | 4 | -2.20 | -0.34 | 416 | R4GMW7 HUMAN rRNA tRNA 2'-O-methyltransferase fibrillarin-like protein 1 OS_Homo sapiens GN_FBLL1 PE_3 SV_1 |
| TSSDP | 5 | -2.18 | -2.10 | 328 | EDRF1_HUMAN Erythroid differentiation-related factor 1 OS Homo sapiens GN_EDRF1 PE_1 SV_1 |
| ESASS | 6 | -2.09 | -0.85 | 474 | CC4L_HUMAN Isoform 10 of C-C motif chemokine 4-like OS Homo sapiens GN_CCL4L1 |
| SASSG | 7 | -1.81 | -1.70 | 475 | VEGFA_HUMAN Isoform L-VEGF 165 of Vascular endothelial growth factor A OS_Homo sapiens GN_VEGFA |
| TTTSP | 8 | -1.72 | -2.03 | 450 | A2A3C1_HUMAN Brain-specific angiogenesis inhibitor 2 OS Homo sapiens GN_BAI2 PE_2 SV_1 |
| ATTAA | 9 | -1.66 | -1.23 | 425 | E7ET36_HUMAN Transferrin receptor protein 2 OS Homo sapiens GN_TFR2 PE_2 SV_1 |
| NATED | 10 | -1.62 | -1.95 | 206 | ATS2_HUMAN A disintegrin and metalloproteinase with thrombospondin motifs 2 OS_Homo sapiens GN_ADAMTS2 PE_2 SV_2 |
| TTAAG | 11 | -1.53 | -0.63 | 426 | COX10_HUMAN Protoheme IX farnesyltransferase |
| ATTTS | 12 | -1.44 | -1.12 | 449 | ATS12_HUMAN A disintegrin and metalloproteinase with thrombospondin motifs 12 OS_Homo sapiens GN_ADAMTS_12 PE_1 SV_2 |
| TAAGP | 13 | -1.36 | -1.62 | 427 | M0QZE4_HUMAN A disintegrin and metalloproteinase with thrombospondin motifs 10 OS_Homo sapiens GN_ADAMTS 10 PE_2 SV_1 |
| VSNGP | 14 | -1.24 | -1.43 | 313 | TSP2_HUMAN Thrombospondin-2 OS_Homo sapiens GN_THBS2 PE_1 SV_2 |
| SADSL | 15 | -1.21 | -1.00 | 442 | C3AR_HUMAN C3a anaphylatoxin chemotactic receptor OS Homo sapiens GN_C3AR1 PE_1 SV_2 |
| AAGPL | 16 | -1.19 | -1.22 | 428 | BAI1_HUMAN Brain-specific angiogenesis inhibitor 1 OS_Homo sapiens GN_BAI1 PE_1 SV_2 |
| IKKPD | 17 | -1.14 | -1.08 | 115 | FRIH_HUMAN Ferritin heavy chain OS_Homo sapiens GN_FTH1 PE_1 SV_2 |
| GRRTR | 18 | -1.10 | -0.36 | 498 | ATS4_HUMAN A disintegrin and metalloproteinase with thrombospondin motifs 4 OS_Homo sapiens GN_ADAMTS4 PE_1 SV_3 |
| KLSST | 19 | -1.05 | -1.31 | 58 | D6RJI3_HUMAN Fibrillin-2 OS_Homo sapiens GN_FBN2 PE_2 SV_1 |
| SENSS | 20 | -0.97 | -0.45 | 346 | BI2L1_HUMAN Brain-specific angiogenesis inhibitor 1-associated protein 2-like protein 1 OS_Homo sapiens GN_BAIAP2L1 PE_1 SV_2 |
| TDVPS | 21 | -0.92 | -1.34 | 79 | BAI1_HUMAN Brain-specific angiogenesis inhibitor 1 OS_Homo sapiens GN_BAI1 PE_1 SV_2 |
| SEATQ | 22 | -0.91 | -1.63 | 401 | B4DDV6_HUMAN Nuclear factor erythroid 2-related factor 1 OS Homo sapiens GN_NRF1 PE_2 SV_1 |
| VATDV | 23 | -0.89 | -0.41 | 77 | B0QYF0_HUMAN Brain-specific angiogenesis inhibitor 1-associated protein 2-like protein 2 (Fragment) OS_Homo sapiens GN_BAIAP2L2 PE_2 SV_1 |
| LPAAP | 24 | -0.85 | -1.77 | 124 | ATS17_HUMAN A disintegrin and metalloproteinase with thrombospondin motifs 17 OS_Homo sapiens GN_ADAMTS17 PE_2 SV_2 |
| ISEAT | 25 | -0.80 | -1.97 | 400 | B4DF38_HUMAN Platelet-activating factor acetylhydrolase IB subunit alpha OS_Homo sapiens GN_PAFAH1B1 PE_2 SV_1 |
| ATQVG | 26 | -0.79 | -0.46 | 403 | K7EM16_HUMAN Vasodilator-stimulated phosphoprotein (Fragment) OS_Homo sapiens GN_VASP PE 4 SV 1 |
| QLANE | 27 | -0.62 | -1.16 | 562 | CCL20_HUMAN C-C motif chemokine 20 OS_Homo sapiens GN_CCL20 PE_1 SV_1 |

**Table 4: Predicted mimics in Ebola small soluble glycoprotein. "Query pos" shows position in that protein. In interests of space only one isoform of each protein is shown**

| proteome penta | SEQ ID NO: | query BEPI | proteome inv JSb predBEPI intra protein | query pos | proteome curation |
|---|---|---|---|---|---|
| NATED | 28 | -1.62 | -1.95 | 206 | ATS2_HUMAN A disintegrin and metalloproteinase with thrombospondin motifs 2 OS_Homo sapiens GN_ADAMTS2 PE_2 SV_2 |
| IKKPD | 29 | -1.14 | -1.08 | 115 | FRIH_HUMAN Ferritin heavy chain OS_Homo sapiens GN_FTH1 PE_1 SV_2 |
| KLSST | 30 | -1.05 | -1.31 | 58 | FBN2_HUMAN Isoform 2 of Fibrillin-2 OS_Homo sapiens GN FBN2 |
| TDVPS | 31 | -0.92 | -1.34 | 79 | BAI1_HUMAN Brain-specific angiogenesis inhibitor 1 OS_Homo sapiens GN_BAI1 PE_1 SV_2 |
| VATDV | 32 | -0.89 | -0.76 | 77 | BI2L2_HUMAN Isoform 2 of Brain-specific angiogenesis inhibitor 1-associated protein 2-like protein 2 OS_Homo sapiens GN_BAIAP2L2 |
| LPAAP | 33 | -0.85 | -1.77 | 124 | ATS17_HUMAN A disintegrin and metalloproteinase with thrombospondin motifs 17 OS_Homo sapiens GN_ADAMTS17 PE_2 SV_2 |

**Table 5: Predicted mimics in Ebola VP24 protein. "Query pos" shows position in that protein. In interests of space only one isoform of each protein is shown**

| proteome penta | SEQ ID NO: | query BEPI | proteome inv JSb predBEPI intra protein | query pos | proteome curation |
|---|---|---|---|---|---|
| KPGPA | 34 | -2.01 | -3.09 | 215 | G3V0F2_HUMAN Ferredoxin reductase |
| PGPAK | 35 | -1.70 | -0.53 | 216 | ATS7_HUMAN A disintegrin and metalloproteinase with thrombospondin motifs 7 OS_Homo sapiens GN_ADAMTS7 PE_1 SV_2 |
| GSSTR | 36 | -1.28 | -1.04 | 235 | VWF_HUMAN von Willebrand factor OS_Homo sapiens GN_VWF PE_1 SV_4 |
| STIES | 37 | -0.85 | 0.10 | 87 | VWA3A_HUMAN von Willebrand factor A domain-containing protein 3A OS Homo sapiens GN_VWA3A PE_2 SV_3 |
| TIESP | 38 | -0.64 | -0.41 | 88 | AGGF1_HUMAN Angiogenic factor with G patch and FHA domains 1 OS_Homo sapiens GN_AGGF1 PE_1 SV_2 |

**Table 6: Predicted mimics in Ebola VP40. "Query pos" shows position in that protein.**

| In interests of space only one isoform of each protein is shown | | | | | |
|---|---|---|---|---|---|
| proteome penta | SEQ ID NO: | query BEPI | proteome inv JSb predBEPI intra protein | query pos | proteome curation |
| SGKKG | 39 | -2.42 | -2.83 | 224 | FBN1_HUMAN Fibrillin-1 OS_Homo sapiens GN_FBN1 PE_1 SV_3 |
| TPTGS | 40 | -2.36 | -2.70 | 197 | VWA7_HUMAN von Willebrand factor A domain-containing protein 7 OS_Homo sapiens GN_VWA7 PE_2 SV_4 |
| KSGKK | 41 | -2.19 | -1.28 | 223 | K7EKI8_HUMAN Periplakin OS_Homo sapiens GN_PPL PE_2 SV_1 |
| VTSKN | 42 | -1.68 | 0.26 | 278 | PKP4_HUMAN Plakophilin-4 OS_Homo sapiens GN_PKP4 PE_1 SV_2 |
| IARGG | 43 | -1.49 | -0.63 | 28 | C5AR2_HUMAN C5a anaphylatoxin chemotactic receptor 2 OS_Homo sapiens GN_C5AR2 PE_1 SV 1 |
| GSNGA | 44 | -1.43 | -1.71 | 200 | VIPR1_HUMAN Vasoactive intestinal polypeptide receptor 1 OS Homo sapiens GN_VIPR1 PE_1 SV_1 |
| KNGQP | 45 | -1.33 | -2.52 | 281 | CCL16_HUMAN C-C motif chemokine 16 OS Homo sapiens GN_CCL16 PE_1 SV_1 |
| GKKVT | 46 | -1.17 | -0.91 | 275 | VWF_HUMAN von Willebrand factor OS_Homo sapiens GN_VWF PE_1 SV_4 |
| TCHSP | 47 | -0.84 | -1.10 | 315 | TSP3_HUMAN Thrombospondin-3 OS_Homo sapiens GN_THBS3 PE_2 SV_1 |
| RLGPG | 48 | -0.71 | 0.08 | 139 | B4DY31 HUMAN cDNA FLJ51386 OS Homo sapiens GN_VWCE PE_2 SV_1 |
| RLGPG | 49 | -0.71 | -1.26 | 139 | C9JCP7_HUMAN Vasoactive intestinal polypeptide receptor 2 OS Homo sapiens GN_PE 2_SV_1 |
| RLGPG | 50 | -0.71 | -0.14 | 139 | E9PJR7_HUMAN Plakophilin-3 (Fragment) OS Homo sapiens GN_PKP3 PE_2 SV_1 |
| RLGPG | 51 | -0.71 | -2.12 | 139 | K7EJK1_HUMAN Glial fibrillary acidic protein OS Homo sapiens GN_GFAP PE_2 SV_1 |
| RLGPG | 52 | -0.71 | -0.07 | 139 | VWCE_HUMAN von Willebrand factor C and EGF domain-containing protein OS_Homo sapiens GN_VWCE PE_2 SV_2 |

This provides an initial screening to identify the human proteome proteins of interest as potential targets of antibody mediated mimicry in Ebola virus.

### Example 3: Neurovirulence in mumps

It has been known for decades, since the beginning of development of cell culture attenuated mumps virus vaccines that certain strains of mumps virus retained their neurovirulence and that testing in animal models is not always a reliable detector of neuroattenuation (32). Neuroattenuation has been attributed to various of the mumps virus proteins and to specific single amino acid changes therein (33), (34), Cui et al PLOS One, 2013; Malik et al J Gen Virol, 2009; Lemon et al J Virol 2007); Shah et al J Med Virol 2009. We therefore selected several strains of mumps virus for which the characteristics of neurovirulence have been experimentally evaluated. These included the strains shown in Table 7.

**Table 7**

| Urabe SKB vaccine | vaccine | |
|---|---|---|
| Urabe Chiron | vaccine | |
| Urabe Biken | vaccine | |
| 87-1005 | clinical | neurovirulent |
| 87-1004 | clinical | neurovirulent |
| GW7 | lab | avirulent |
| JerylLynn | Vaccine | avirulent |

In this case the analysis as described in Example 1 failed to find any pentamer matches peculiar to the known neurovirulent strains as compared to the avirulent strains in Table 7. Jeryl Lynn did have a number of pentamer matches to the proteome that differed from the other strains, this may reflect its extensive *in vitro* passage history-

### Example 4: Evaluation of monoclonal antibodies

In order to evaluate the screening process on monoclonal antibody products we tapped a database of commercially developed monoclonal antibodies and downloaded sequences for brodalumab. Brodalumab, an anti-interleukin 17 receptor antibody was developed for treatment of psoriasis. It was effective in control of psoriasis but withdrawn from clinical trials because of an association with suicide and suicidal thoughts (Danesh MJ Kimball Ab J am Acad Dermatol, 2016; see also Wikipedia.org/wiki/brodalumab). We addressed two questions: what makes brodalumab different from other monoclonal antibody products and does it have any neurologic mimics which offer any indicators on behavioral changes In parallel, we evaluated Rituximab as an example of a monoclonal which is well tolerated.

In order to produce a clinical result differing from other monoclonal antibodies Brodalumab would have to contain a different set of pentamer motifs from other antibodies, or at least a rare set in a different context relative to B cell epitope characteristics and associated MHC II binding peptides. Necessarily such a motif would lie in the variable region or in any part of the constant region which has been engineered.

To examine this we looked at the entire sequences of heavy and light chain, and noted especially the variable region of both heavy and light chains of the product, comprising the N terminal 150 amino acids, to identify rare pentamer motifs. We set the threshold from a previously computed database of antibodies (see, e.g., PCT US2011/029192). Briefly this database comprises 45,000 heavy chain variable regions retrieved from NCBI Protein resource with a search argument "(immunoglobulin heavy chain variable region) AND (*Homo sapiens*)"*.* Various search arguments were used to extract non-redundant subsets (by Genbank accession number) that were either immunoglobulin class-defined, or to eliminate sequences for which the metadata attached to the accession indicated association with an immunopathology (lymphoma, leukemia, lupus, rheumatoid arthritis, multiple sclerosis). Manual curation was used to remove sequences that were obviously not immunoglobulins. The final dataset thus included 39,957 non-class-defined immunoglobulins, not associated with immunopathology. The resulting dataset comprises many different accession groups from studies carried out over a considerable period of time so can be considered a representative sample of "natural" human immunoglobulins. Accessions with signal peptides were identified and signal peptides removed using the combined signal peptide and transmembrane predictor Phobius (phobius.sbc.su.se). IGHV were included in the final set if they contained at least 80 amino acids, a value approximating the shortest germline equivalent sequence. All sequences longer than 130 amino acids were truncated at that point. The approximate positions of the three complementarity determining regions (CDR) have been indicated in Figure 1 relative to standard IGHV sequence landmarks. A further 16,000 light chain variable regions were also retrieved from Genbank and curated to remove those derived from immunopathologies, using the same criteria as described for the heavy chains. The final reference databases comprised approximately 6.4 × 10⁶ total TCEM, including 325,000 unique pentamer motifs. Using this database we identified motifs found at less than 1 in 1024 antibodies, less than 1 in 65000 (2¹⁶), and less than 1 in 1 million (2²⁰).

Secondly we computed the B cell epitope pentamers of brodalumab and rituximab and compared these to our precomputed database of human proteome pentamers (as described above). A key word search was conducted to identify protein with neurologic function, using the key words in Table A above. This identified 496 matches, inclusive of all isoforms. For Rituximab 560 pentamer matches were identified. When this was filtered to identify those wherein the predicted probability of B cell epitopes was in the top 25% for the brodalumab and in the top 40% of the proteome neurologic subset, 77 heavy chain and 69 light chain matches were identified for brodalumab, inclusive of multiple isoforms. For rituximab we identified 67 heavy chain and 69 light chain matches, inclusive of multiple isoforms.

**Table 8. The rare motif present in the two chains of the two monoclonals**

| proteome penta | SEQ ID NO: | N(brodalumab Homo sapiens H- | N(brodalumab Homo sapiens L- | N(rituximab Chimeric H- | N(rituximab Chimeric L- | Occurrence |
|---|---|---|---|---|---|---|
| ALPAP | 53 | | | X | | Rituximab |
| GLPAP | 54 | X | | | | Brodalumab |
| ISKAK | 55 | | | X | | Rituximab |
| KALPA | 56 | | | X | | Rituximab |
| KSTSG | 57 | | | X | | Rituximab |
| PAPPV | 58 | X | | | | Brodalumab |
| PPKPK | 59 | X | | X | | Both |
| PREEQ | 60 | X | | X | | Both |
| PSREE | 61 | X | | | | Brodalumab |
| RSTSE | 62 | X | | | | Brodalumab |
| SDEQL | 63 | | X | | X | Both |
| SRDEL | 64 | | | X | | Rituximab |
| SSPKP | 65 | | | | X | Rituximab |
| STSES | 66 | X | | | | Brodalumab |
| STYSL | 67 | | X | | X | Both |
| TKPRE | 68 | X | | X | | Both |

This focused our attention on five motif which are unique to brodalumab and all of which are in the heavy chain. Table 9 shows the affinity of these motifs in both brodalumab and the proteome as well as the position in the monoclonal.

**Table 9**

| query penta | SEQ ID NO: | proteome curation | proteome gi | Mab BEPI probability | Proteome BEPI probability | query pos |
|---|---|---|---|---|---|---|
| RSTSE | 62 | MYNN_HUMAN Myoneurin OS_Homo sapiens GN_MYNN PE 1 SV 1 | Q9NPC7 | -1.72 | -1.11 | 134 |
| RSTSE | 62 | MYNN_HUMAN Isoform 2 of Myoneurin OS_Homo sapiens GN MYNN | Q9NPC7-2 | -1.72 | -1.11 | 134 |
| RSTSE | 62 | MYNN_HUMAN Isoform 3 of Myoneurin OS_Homo sapiens GN MYNN | Q9NPC7-3 | -1.72 | -1.20 | 134 |
| RSTSE | 62 | MYNN_HUMAN Isoform 4 of Myoneurin OS_Homo sapiens GN MYNN | Q9NPC7-4 | -1.72 | -2.14 | 134 |
| STSES | 66 | MPZL1_HUMAN Myelin protein zero-like protein 1 OS_Homo sapiens GN_MPZL1 PE_1 SV_1 | O95297 | -1.71 | -0.84 | 135 |
| STSES | 66 | MPZL1_HUMAN Isoform 2 of Myelin protein zero-like protein 1 OS_Homo sapiens GN_MPZL1 | O95297-2 | -1.71 | -0.84 | 135 |
| STSES | 66 | MPZL1_HUMAN Isoform 4 of Myelin protein zero-like protein 1 OS_Homo sapiens GN_MPZL1 | O95297-4 | -1.71 | -0.70 | 135 |
| PAPPV | 58 | OPA3_HUMAN Isoform 2 of Optic atrophy 3 protein OS Homo sapiens GN_OPA3 | Q9H6K4-2 | -0.94 | -1.87 | 228 |
| GLPAP | 54 | Q5JUY5_HUMAN Myeloproliferative leukemia virus oncogene | Q5JUY5 | -0.96 | -1.18 | 324 |
| PSREE | 61 | MMTA2_HUMAN Multiple myeloma tumor-associated protein 2 OS_Homo sapiens GN_MMTAG2 PE_1 SV_1 | Q9BU76 | -0.88 | -0.38 | 350 |
| PSREE | 61 | MMTA2_HUMAN Isoform 2 of Multiple myeloma tumor-associated protein 2 OS_Homo sapiens GN_MMTAG2 | Q9BU76-2 | -0.88 | -0.95 | 350 |
| PSREE | 61 | MMTA2_HUMAN Isoform 3 of Multiple myeloma tumor-associated protein 2 OS_Homo sapiens GN_MMTAG2 | Q9BU76-3 | -0.88 | -0.93 | 350 |
| PSREE | 61 | MMTA2_HUMAN Isoform 4 of Multiple myeloma tumor-associated protein 2 OS_Homo sapiens GN_MMTAG2 | Q9BU76-4 | -0.88 | -0.68 | 350 |

Only two motifs RSTSE and overlapping STSES show high BEPI probability (<-1.4) and are located in the variable regions. Positions 134 and 135 are near the C terminus of the variable region and the motifs of interest may have been created as a function of the engineering of the variable region on to the constant region. As shown in Figure 1, the two overlapping motifs have a series of MHC II high binding peptides immediately adjacent to them.

In the case of Rituximab, as shown in table 10A, the BEPI probabilities are lower and the motifs are in the constant regions, except for one motif located at position 43 of the light chain.

**Table 10A**

| proteome penta | SEQ ID NO: | proteome curation | proteome gi | Mab BEPI | proteome BEPI | Mab pos |
|---|---|---|---|---|---|---|
| KALPA | 56 | H7BYZ3_HUMAN Calcineurin subunit B type 1 OS_Homo sapiens GN_PPP3R1 PE_2 SV 1 | H7BYZ3 | -0.86 | -0.87 | 332 |
| ALPAP | 53 | VWA1_HUMAN von Willebrand factor A domain-containing protein 1 OS_Homo sapiens GN_VWA1 PE_2 SV_1 | Q6PCB0 | -0.88 | -0.57 | 333 |
| ALPAP | 53 | VWA1_HUMAN Isoform 2 of von Willebrand factor A domain-containing protein 1 OS_Homo sapiens GN_VWA1 | Q6PCB0-2 | -0.88 | -0.41 | 333 |
| ISKAK | 55 | NPSR1_HUMAN Neuropeptide S receptor OS_Homo sapiens GN_NPSR1_PE_2 SV_1 | Q6W5P4 | -0.85 | -0.32 | 342 |
| ISKAK | 55 | NPSR1_HUMAN Isoform 3 of Neuropeptide S receptor OS_Homo sapiens GN_NPSR1 | Q6W5P4-3 | -0.85 | -0.33 | 342 |
| ISKAK | 55 | NPSR1_HUMAN Isoform 4 of Neuropeptide S receptor OS_Homo sapiens GN_NPSR1 | Q6W5P4-4 | -0.85 | -0.39 | 342 |
| ISKAK | 55 | NPSR1_HUMAN Isoform 5 of Neuropeptide S receptor OS_Homo sapiens GN_NPSR1 | Q6W5P4-5 | -0.85 | -0.39 | 342 |
| SRDEL | 64 | B4DFB8_HUMAN Synaptonemal complex protein 2-like OS_Homo sapiens GN_SYCP2L PE 2 SV 1 | B4DFB8 | -0.89 | -0.98 | 360 |
| SRDEL | 64 | SYC2L_HUMAN Synaptonemal complex protein 2-like OS_Homo sapiens GN_SYCP2L PE_1 SV_2 | Q5T4T6 | -0.89 | -0.55 | 360 |
| SRDEL | 64 | SYC2L_HUMAN Isoform 2 of Synaptonemal complex protein 2-like OS Homo sapiens GN_SYCP2L | Q5T4T6-2 | -0.89 | -0.97 | 360 |
| SSPKP | 65 | CEND_HUMAN Cell cycle exit and neuronal differentiation protein 1 OS_Homo sapiens GN_CEND1 PE_2 SV_1 | Q8N111 | -1.32 | -1.73 | 43 |
| PAPPV | 58 | OPA3_HUMAN Isoform 2 of Optic atrophy 3 protein OS_Homo sapiens GN_OPA3 | Q9H6K4-2 | -0.94 | -1.87 | 228 |

The two human proteins identified as unique matches in brodalumab, for Myoneurin and Myelin protein zero-like protein 1 are probable mimics and depending on the function of these two proteins would be candidates for investigation to determine their possible contribution to the neurologic changes seen in subjects.

When a search of all possible human proteome epitope mimics is conducted for the pentameric motifs that are high probability B cell epitopes in brodalumab but absent from rituximab, a further 344 possible proteins are identified which contain epitope mimics. Some have a function in neurologic pathways. These provide a second tier of proteins which should be examined for possible contributions to pathways leading to suicidal tendencies.

### Example 4: In utero infection with cytomegalovirus and rubella virus

The surface proteins of ten strains of rubella virus, E1 E2 and capsid protein were analyzed following the steps laid out in example 1. The same key word search pattern was used as described in example 1 to detect neurologic function proteins. Table 10B shows the results for one exemplary isolate (Br1). Where more than one isoform of the human protein exhibited a match, only one example is included in the table in the interests of space.

**Table 10B**

| **E1 protein** | | | | | |
|---|---|---|---|---|---|
| BEPI Motif | SEQ ID NO: | BEPI Virus | BEPI Proteome | query pos | proteome curation |
| APGGG | 69 | -1.60 | -2.24 | 206 | NAV1_HUMAN Neuron navigator 1 OS_Homo sapiens GN NAV1 PE_1 SV_2 |
| APGPG | 70 | -1.78 | -1.80 | 112 | NDF2 _HUMAN Neurogenic differentiation factor 2 OS Homo sapiens GN_NEUROD2 PE_2 SV 2 |
| FAPPR | 71 | -1.00 | -1.26 | 182 | NBAS_HUMAN Neuroblastoma-amplified sequence OS_Homo sapiens GN_NBAS PE_1 SV 2 |
| GLAPG | 72 | -1.31 | -0.39 | 204 | B4DIR1_HUMAN Glial fibrillary acidic protein OS Homo sapiens GN_GFAP PE_2 SV_1 |
| HTTSD | 73 | -0.74 | -0.87 | 154 | F5GXV7 _HUMAN Neurobeachin OS_Homo sapiens GN_NBEA PE_2 SV_1 |
| PGPGE | 74 | -1.47 | -2.41 | 113 | NRSN1_HUMAN Neurensin-1 OS_Homo sapiens GN_NRSN1 PE_2 SV_1 |
| PWHPP | 75 | -1.39 | -0.69 | 159 | MRF_HUMAN Myelin regulatory factor OS Homo sapiens GN_MYRF PE_1 SV_3 |
| QRHSP | 76 | -0.71 | -1.01 | 80 | CNTFR_HUMAN Ciliary neurotrophic factor receptor subunit alpha OS_Homo sapiens GN_CNTFR PE_1 SV_2 |
| WHPPG | 77 | -1.48 | -0.90 | 160 | MRF_HUMAN Myelin regulatory factor OS Homo sapiens GN MYRF PE 1 SV 3 |

| **E2 Protein** | | | | | |
|---|---|---|---|---|---|
| BEPI Motif | | BEPI Virus | BEPI Proteome | query pos | proteome curation |
| APPAP | 78 | -1.64 | -1.76 | 12 | NOTC2_HUMAN Neurogenic locus notch homolog protein 2 OS_Homo sapiens GN_NOTCH2 PE_1 SV_3 |
| ATPAT | 79 | -1.36 | -1.32 | 117 | Q5T6D8_HUMAN Neuropeptide FF receptor 1 (Fragment) OS_Homo sapiens GN_NPFFR1 PE 2 SV 1 |
| ATTPA | 80 | -1.01 | -0.43 | 120 | NEUM _HUMAN Neuromodulin OS_Homo sapiens GN_GAP43 PE_1 SV_1 |
| PPAPP | 81 | -1.68 | -1.71 | 13 | NAV1_HUMAN Neuron navigator 1 OS_Homo sapiens GN_NAV1 PE_1 SV_2 |
| TAANS | 82 | -0.72 | -0.61 | 109 | NAV2_HUMAN Isoform 12 of Neuron navigator 2 OS Homo sapiens GN NAV2 |
| TTPAP | 83 | -0.71 | -1.11 | 121 | NAV1_HUMAN Isoform 7 of Neuron navigator 1 OS Homo sapiens GN NAV1 |

| **Capsid protein** | | | | | |
|---|---|---|---|---|---|
| BEPI Motif | | BEPI Virus | BEPI Proteome | query pos | proteome curation |
| APLPP | 84 | -0.98 | -0.64 | 257 | VGF_HUMAN Neurosecretory protein VGF OS Homo sapiens GN_VGF PE_1 SV_2 |
| APPPP | 85 | -1.93 | -1.52 | 79 | F5GZS7_HUMAN Neuregulin-2 OS_Homo sapiens GN_NRG2 PE_2 SV_1 |
| CGPEP | 86 | -0.73 | -0.85 | 199 | F5GXV7_HUMAN Neurobeachin OS _Homo sapiens GN_NBEA PE_2 SV_1 |
| DSGGP | 87 | -1.55 | -1.85 | 57 | C9J4D3_HUMAN Neuroligin-1 (Fragment) OS Homo sapiens GN_NLGN1 PE_2 SV_1 |
| DSSTS | 88 | -1.50 | -1.47 | 46 | S6A16_HUMAN Orphan sodium- and chloride-dependent neurotransmitter transporter NTT5 OS Homo sapiens GN_SLC6A16 PE_2 SV_1 |
| GGTAP | 89 | -1.29 | -2.34 | 116 | NEU1A_HUMAN Neuralized-like protein 1A OS Homo sapiens GN_NEURL PE_2 SV_1 |
| GPRRR | 90 | -1.28 | -2.02 | 60 | NRTN_HUMAN Neurturin OS_Homo sapiens GN_NRTN PE_1 SV_1 |
| KAPPP | 91 | -1.69 | -1.85 | 78 | ACHA4_HUMAN Neuronal acetylcholine receptor subunit alpha-4 OS_Homo sapiens GN_CHRNA4 PE_1 SV_2 |
| PDTEA | 92 | -1.06 | -0.51 | 146 | H0Y465_HUMAN Neurofibromin truncated (Fragment) OS_Homo sapiens GN_NF1 PE_2 SV 1 |
| PPQPP | 93 | -1.74 | -1.77 | 102 | E7EUA9_HUMAN Neuron navigator 3 OS Homo sapiens GN_NAV3 PE_2 SV_2 |
| PPRAP | 94 | -1.57 | -1.68 | 98 | E5RHQ4_HUMAN Neuronal acetylcholine receptor subunit alpha-2 (Fragment) OS_Homo sapiens GN_CHRNA2 PE_2 SV_1 |
| PRPPR | 95 | -1.27 | -1.80 | 39 | NTR2_HUMAN Neurotensin receptor type 2 OS Homo sapiens GN_NTSR2 PE_1 SV_2 |
| PRRRR | 96 | -1.09 | -1.63 | 61 | NRTN_HUMAN Neurturin OS_Homo sapiens GN_NRTN PE_1 SV_1 |
| QPAGD | 97 | -0.62 | -1.39 | 213 | H7C408_HUMAN Neurobeachin-like protein 2 (Fragment) OS_Homo sapiens GN_NBEAL2 PE_2 SV_1 |
| RDSGG | 98 | -1.63 | -1.74 | 56 | C9J4D3_HUMAN Neuroligin-1 (Fragment) OS Homo sapiens GN_NLGN1 PE_2 SV_1 |
| RRRRG | 99 | -0.97 | -1.46 | 62 | S4R3K2_HUMAN Neuroblastoma breakpoint family member 1 OS_Homo sapiens GN_NBPF1 PE_4 SV_1 |
| SAPLP | 100 | -0.92 | -0.51 | 256 | NPDC1_HUMAN Neural proliferation differentiation and control protein 1 OS_Homo sapiens GN_NPDC1 PE_1 SV_2 |
| SSTSG | 101 | -1.65 | -1.50 | 47 | E7EUA9_HUMAN Neuron navigator 3 OS Homo sapiens GN_NAV3 PE_2 SV_2 |

Cytomegalovirus is a large virus comprising over 200 proteins of which over 130 are structural proteins. However, a large proportion of the virus by weight is comprised of the exposed surface membrane glycoproteins which are exposed to the host immune system and engender the majority of the antibody response. In secondary infections with cytomegalovirus antibody rise to glycoprotein B is particularly noted. While all proteins were analyzed, we report here on the results from the principal membrane glycoproteins. Further in the interests of space only results for glycoprotein B are shown in Table 11.

**Table 11**

| penta | SEQ ID NO: | query BEPI | proteome BEPI | query pos | proteome curation |
|---|---|---|---|---|---|
| AEQRA | 102 | -1.11 | -0.93 | 859 | NEUR2_HUMAN Sialidase-2 OS_Homo sapiens GN_NEU2 PE_1 SV_2 |
| AVSSS | 103 | -1.16 | -0.75 | 24 | NTR2_HUMAN Neurotensin receptor type 2 OS_Homo sapiens GN_NTSR2_PE_1 SV_2 |
| DFGRP | 104 | -0.83 | -0.75 | 310 | H3BUT1_HUMAN Ceroid-lipofuscinosis neuronal protein 6 OS_Homo sapiens GN_CLN6 PE_2 SV_1 |
| DGTTV | 105 | -1.43 | -1.10 | 796 | F5H025_HUMAN Neural cell adhesion molecule L1 OS_Homo sapiens GN_L1CAM PE_2 SV_1 |
| GPGPP | 106 | -2.95 | -2.07 | 827 | NOT3_HUMAN Neurogenic locus notch homolog protein 3 OS_Homo sapiens GN_NOTCH3 PE_1 SV_2 |
| GPPSS | 107 | -2.59 | -1.47 | 829 | PIANP_HUMAN Isoform 2 of PILR alpha-associated neural protein OS_Homo sapiens GN_PIANP |
| GRKGP | 108 | -2.00 | -0.91 | 824 | NEUG_HUMAN Neurogranin OS_Homo sapiens GN_NRGN PE_1 SV_1 |
| HNRTK | 109 | -0.93 | -1.19 | 457 | ZN274_HUMAN Neurotrophin receptor-interacting factor homolog OS_Homo sapiens GN_ZNF274 PE_1 SV_2 |
| KGPGP | 110 | -2.73 | -1.65 | 826 | GSCR1_HUMAN Isoform 2 of Glioma tumor suppressor candidate region gene 1 protein OS_Homo sapiens GN_GLTSCR1 |
| LGAAG | 111 | -0.82 | -0.59 | 721 | NTR2_HUMAN Neurotensin receptor type 2 OS_Homo sapiens GN_NTSR2 PE_1 SV_2 |
| NRTKR | 112 | -1.17 | -1.55 | 458 | ZN274_HUMAN Isoform 4 of Neurotrophin receptor-interacting factor homolog OS_Homo sapiens GN_ZNF274 |
| PGPPS | 113 | -2.87 | -1.31 | 828 | I3L2W2_HUMAN Neuralized-like protein 4 OS_Homo sapiens GN_NEURL4 PE_2 SV_1 |
| QLGED | 114 | -0.71 | -1.04 | 596 | H0Y764_HUMAN Neurobeachin-like protein 2 (Fragment) OS_Homo sapiens GN_NBEAL2 PE_4_SV_1 |
| RKGPG | 115 | -2.35 | -0.91 | 825 | NEUG_HUMAN Neurogranin OS_Homo sapiens GN_NRGN PE_1 SV_1 |
| SNTHS | 116 | -1.13 | -1.76 | 221 | NRX3A _HUMAN Isoform 4a of Neurexin-3 OS_Homo sapiens GN_NRXN3 |
| SQTVS | 117 | -0.95 | -0.44 | 62 | NAV2 _HUMAN Neuron navigator 2 OS_Homo sapiens GN_NAV2 PE_1 SV_3 |
| SQTVS | 118 | -0.95 | -0.42 | 62 | NAV2 _HUMAN Isoform 9 of Neuron navigator 2 OS_Homo sapiens GN_NAV2 |
| SRSGS | 119 | -1.46 | -0.90 | 50 | A8MZH3_HUMAN Myelin basic protein OS_Homo sapiens GN_MBP PE_2 SV_1 |
| SSQTV | 120 | -1.01 | -0.71 | 61 | NAV2_HUMAN Neuron navigator 2 OS_Homo sapiens GN_NAV2 PE_1 SV_3 |
| SSSST | 121 | -1.91 | -2.65 | 26 | MYT1L_HUMAN Isoform 4 of Myelin transcription factor 1-like protein OS_Homo sapiens GN_MYT1L |
| TAAPP | 122 | -1.92 | -1.34 | 837 | WASL_HUMAN Neural Wiskott-Aldrich syndrome protein OS_Homo sapiens GN_WASL PE_1 SV_2 |
| TDSLD | 123 | -1.37 | -0.59 | 868 | F8W7J9_HUMAN Neurabin-1 OS_Homo sapiens GN_PPP1R9A PE_2 SV_1 |
| THNRT | 124 | -0.67 | -1.25 | 456 | ZN274_HUMAN Neurotrophin receptor-interacting factor homolog OS_Homo sapiens GN_ZNF274 PE_1 SV_2 |
| VSSSS | 125 | -1.58 | -1.54 | 25 | B4DR69_HUMAN Neuronal PAS domain-containing protein 1 OS_Homo sapiens GN_NPAS1 PE_2 SV_1 |

### Example 5: Autoimmunity in Zika virus infection

The procedure described in Example 1 was followed in the case of Zika virus. Predicted antibody mimics were defined in each of the viral proteins. Table N shows the predicted mimics identified in the structural proteins of Zika virus as well as whether the motif is present in both African and American strains. The occurrence of mimic in proNPY and the NAV2 proteins is consistent with the appearance of Guillain Barre syndrome and other neurologic defeicits experienced by individuals infected. In addition, the interaction with NPY and with NAV2 at a critical point in fetal development may be the basis for the developmental failures the most obvious of which is microcephaly.

**Table 12. Predicted mimics arising from Anti-Zika antibody.**

| Pentamer | SEQ ID NO: | Zika AFR | Zika BR | BEPI Virus | BEPI Prote ome | UniProt ID | Annotation |
|---|---|---|---|---|---|---|---|
| Envelope | | | | | | | |
| PRAEA | 126 | Y | Y | -1.67 | -0.84 | OPTN | Optineurin |
| TESTE | 127 | Y | Y | -1.59 | -1.07 | F8WCE4 | Synaptogyrin-1 |
| ESTEN | 128 | Y | Y | -1.50 | -0.55 | NPY | Pro-neuropeptide Y |
| KGRLS | 129 | N | Y | -1.46 | -0.80 | NAV2 | Neuron navigator 2 |
| STENS | 130 | Y | Y | -1.29 | -1.22 | E7EP46 | Neurotrophin-4 |
| AGADT | 131 | Y | Y | -1.18 | -1.16 | NOTC3 | Neurogenic locus notch homolog protein 3 |
| QPENL | 132 | Y | Y | -0.95 | -1.32 | NOTC2 | Neurogenic locus notch homolog protein 2 |
| LSSGH | 133 | N | Y | -0.84 | -0.38 | NDF4 | Neurogenic differentiation factor 4 |
| PVITE | 134 | Y | Y | -0.76 | -0.41 | E9PHJ4 | Neural cell adhesion molecule L1 |
| GGALN | 135 | N | Y | -0.74 | -0.37 | NOTC 1 | Neurogenic locus notch homolog protein 1 |
| AKVEV | 136 | Y | N | -0.73 | -0.46 | HRSL4 | Retinoic acid receptor responder protein 3 |
| ATLGG | 137 | Y | Y | -0.70 | -1.13 | BRNP2 | BMP_retinoic acid-inducible neural-specific protein 2 |
| MSGGT | 138 | Y | Y | -0.66 | -0.52 | BDNF | Brain-derived neurotrophic factor |

| PrM | | | | | | | |
|---|---|---|---|---|---|---|---|
| ARRSR | 139 | Y | Y | -1.65 | -0.95 | NEUL2 | Neuralized-like protein 2 |
| SDAGK | 140 | Y | N | -1.46 | -1.55 | E7EUC6 | Neuron navigator 3 |
| GSSTS | 141 | Y | Y | -1.27 | -1.95 | SYPL2 | Synaptophysin-like protein 2 |
| STRKL | 142 | Y | Y | -1.15 | -0.59 | A2A341 | Synaptonemal complex protein 2 |
| SHSTR | 143 | Y | Y | -1.02 | -0.63 | F5GZS7 | Neuregulin-2 |
| RSRRA | 144 | Y | Y | -0.99 | -0.93 | ARHG8 | Neuroepithelial cell-transforming gene 1 protein |

| Capsid | | | | | | | |
|---|---|---|---|---|---|---|---|
| KKRRG | 145 | N | Y | -2.21 | -1.69 | H7BY68 | Putative neuroblastoma breakpoint family member 8 |
| RRGAD | 146 | Y | Y | -2.11 | -0.75 | NEUL4 | Neuralized-like protein 4 |
| EKKRR | 147 | N | Y | -2.05 | -1.55 | NPAS2 | Neuronal PAS domain-containing protein 2 |
| ERKRR | 148 | Y | N | -1.95 | -0.60 | NSMF | NMDA receptor synaptonuclear signaling and neuronal migration factor |
| SVGKK | 149 | Y | Y | -0.93 | -0.61 | ESYT3 | Extended synaptotagmin-3 |

In the case of Zika envelope protein, a feature conserved which is not seen in other flaviviruses is a band of high affinity MHC II binding immediately adjacent to the sequence which forms the domain II loop DE. This loop is the location of the sequence PVITESTENSK which encompasses several of the mimic peptides listed in the above table. The juxtaposition of high MHC II binding and hence T cell help favors the development of higher titers of antibody and class switch of the immunoglobulins which may accentuate the autoimmune consequences

### Example 6. NPY difference in species

As discussed in Example 5 above, the anti-Zika antibody mediated mimics which target proNeuropeptide Y through the motif ESTEN we were interested to know which species in addition to humans would be affected by this mimicry. We therefore searched UniProt to determine the sequence composition of proNPY for multiple species. Table 13 summarizes the findings for a subset of species.

**Table 13**

| Species | Mature peptide motif mimic for Dengue 3 | SEQ ID NO: | CPON motif mimic for Zika | SEQ ID NO: |
|---|---|---|---|---|
| Human | GEDAP | 150 | ESTEN | 151 |

| Corresponding motif in these positions in other species | | | | |
|---|---|---|---|---|
| *Sus scrofa* | GEDAP | 150 | EGTEN | 152 |
| *Oryctolagus cuniculus* | GEDAP | 150 | ENTEN | 153 |
| *Equus caballus* | GEDAP | 150 | ETTEN | 154 |
| *Felis catus* | GEDAP | 150 | ESTEN | 151 |
| *Macaca mulatta* | GEDAP | 150 | ESTEN | 151 |
| *Canis familiaris* | GEDAP | 150 | ESTEN | 151 |
| *Bos taurus* | GEDAP | 150 | ESTGN | 155 |
| *Ovis aries* | GEDAP | 150 | ESTGN | 155 |
| *Rattus norvegicus* | GEDAP | 150 | ESTEN | 151 |
| *Mus musculus* | GEDAP | 150 | ESTEN | 151 |

Among the species examined, only non-human primates and rats and mice carry the ESTEN motif which is predicted to be targeted by the anti-Zika envelope antibodies. Thus other animal species infected by Zika would not experience neurologic impacts due to binding of CPON. On the other hand the motif GEDAP found in dengue 3 is conserved across all the species evaluated.

The implication of this finding is that testing of a mimic in a species other than humans, non-human primates and certain rodents would result in experimental results which would not provide useful information relative to the impact of antibody mediated mimics in man. This underscored the importance of applying computational screening to select appropriate animal models for diseases or to test novel protein biopharmaceuticals and vaccines. The above example applies specifically to Zika but other species distributions of critical motifs would be expected for other proteome proteins which constitute the antibody mimic targets of antibodies elicited by other antigens.

### Example 7: Epitope mimics in Flavivirus NS1 corresponding to cardiovascular function human proteins

Dengue is well known as a hemorrhagic disease, with dengue hemorrhagic fever occurring most typically following a second infection with a different serotype from the first infection. While for many years the role of antibody dependent enhancement (ADE) has been cited as a cause for this (*35*), there is increasing evidence that dengue does evoke an autoimmune response (*36*), that von Willebrand factor may be depleted (*37*), and that other clotting factors may be affected (*38, 39*). Most recently the NS1 protein has been implicated as leading to vascular permeability in dengue (*40, 41*) and activating Toll receptor 4, and several possible direct viral pathogenic mechanisms have been described. However, the most serious vascular leakage in dengue hemorrhagic fever occurs after the peak of NS1 has declined, suggesting that a direct role of NS1 may not be the only factor (**42**). In particular embodiments of the present invention, a subset of the human proteome was selected to include those proteins which have a function in the cardiovascular system, including structural proteins found in endothelium, platelets, erythrocytes, and enzymes expressed by these cells, and coagulation cascade proteins. In the present invention, we describe the role of NS1 in dengue in eliciting auto antibodies to various proteins with cardiovascular function, including but not limited to coagulation factor V and VIII, prothrombin, von Willebrand factor, ADAMTS13 (A disintegrin and metalloproteinase with thrombospondin motifs 13), platelet glycoprotein Ib beta, vascular endothelial growth factor, vascular endothelial growth factor receptor and platelet endothelial aggregation receptor. Notably no such epitope matches in cardiovascular function proteins clearly linked to hemorrhage and thrombocytopenia occur in the corresponding proteins of West Nile virus. In particular embodiments we describe the precise B cell epitopes which are mimics, thereby enabling the mutation or removal of such epitopes to reduce adverse effects in a vaccine.

Infection with Zika virus has led to the development of deadly thrombocytopenia. (43, 44). In even mild cases of ZIKV, USUV, or dengue infection, an erythremic rash is a typical clinical sign. Epitope analysis of NS1 was conducted for an array of flaviviruses including four serotypes of dengue, yellow fever, Zika virus and Usutu virus, as well as St Louis encephalitis, West Nile, Japanese encephalitis, and Tick borne encephalitis. Particular attention was focused on the C terminal loop of NS1 lying between amino acids 280 and 329, bounded by cysteine residues, and more particularly between 290 and 311, likewise bounded by cysteine residues. This region in every flavivirus examined contains not only strong predicted B cell epitopes, but also a region of high MHC II binding for multiple alleles as shown in Table 14 below.

**Table 14: Predicted MHC II binding of sequential peptides across NS1 280-329 for multiple flaviviruses. Prediction is the permuted population average across 28 alleles of MHC II.**

| Index amino acid Position# | Permuted average MHC II binding across 28 MHC II alleles | | | | | | | |
|---|---|---|---|---|---|---|---|---|
| | DEN1 | DEN2 | DEN3 | DEN4 | YF | WNV | ZIKV | USUV |
| 280 | -0.55 | -0.76 | -0.74 | -0.05 | -0.56 | -1.14 | -0.60 | -1.25 |
| 281 | -0.38 | -0.40 | -0.67 | 0.05 | -0.51 | -0.90 | -0.74 | -1.02 |
| 282 | -0.11 | 0.05 | -0.63 | 0.10 | -0.39 | -0.44 | -0.78 | -0.71 |
| 283 | 0.10 | 0.40 | -0.55 | -0.04 | -0.31 | -0.04 | -0.71 | -0.49 |
| 284 | 0.06 | 0.43 | -0.55 | -0.28 | -0.32 | 0.04 | -0.75 | -0.44 |
| 285 | -0.17 | 0.28 | -0.57 | -0.39 | -0.27 | -0.08 | -0.74 | -0.50 |
| 286 | -0.39 | 0.16 | -0.63 | -0.36 | -0.13 | -0.04 | -0.80 | -0.52 |
| 287 | -0.39 | 0.19 | -0.58 | -0.40 | 0.16 | 0.05 | -0.73 | -0.44 |
| 288 | -0.31 | 0.19 | -0.44 | -0.42 | 0.54 | 0.29 | -0.59 | -0.34 |
| 289 | -0.38 | 0.04 | -0.33 | -0.47 | 0.85 | 0.41 | -0.52 | -0.31 |
| 290 | -0.52 | -0.24 | -0.36 | -0.56 | 0.98 | 0.35 | -0.52 | -0.40 |
| 291 | -0.69 | -0.56 | -0.54 | -0.67 | 1.01 | 0.17 | -0.58 | -0.54 |
| 292 | -0.84 | -0.82 | -0.77 | -0.76 | 0.89 | -0.09 | -0.65 | -0.66 |
| 293 | -0.88 | -0.84 | -0.82 | -0.81 | 0.79 | -0.26 | -0.59 | -0.64 |
| 294 | -0.88 | -0.87 | -0.83 | -0.83 | 0.52 | -0.34 | -0.59 | -0.66 |
| 295 | -0.91 | -0.86 | -0.84 | -0.83 | 0.19 | -0.38 | -0.61 | -0.68 |
| 296 | -0.95 | -0.88 | -0.86 | -0.85 | -0.11 | -0.49 | -0.61 | -0.70 |
| 297 | -0.98 | -0.84 | -0.87 | -0.84 | -0.17 | -0.52 | -0.62 | -0.69 |
| 298 | -1.02 | -0.87 | -0.90 | -0.86 | -0.22 | -0.56 | -0.57 | -0.71 |
| 299 | -1.03 | -0.93 | -0.94 | -0.83 | -0.36 | -0.64 | -0.57 | -0.76 |
| 300 | -1.10 | -1.02 | -1.02 | -0.88 | -0.73 | -0.84 | -0.67 | -0.82 |
| 301 | -1.25 | -1.16 | -1.17 | -1.03 | -1.09 | -1.08 | -0.84 | -0.93 |
| 302 | -1.36 | -1.17 | -1.29 | -1.10 | -1.24 | -1.14 | -0.94 | -0.88 |
| 303 | -1.43 | -1.21 | -1.36 | -1.19 | -1.26 | -1.19 | -1.05 | -0.93 |
| 304 | -1.59 | -1.47 | -1.52 | -1.43 | -1.40 | -1.48 | -1.21 | -1.27 |
| 305 | -1.81 | -1.81 | -1.73 | -1.70 | -1.58 | -1.88 | -1.50 | -1.73 |
| 306 | -2.03 | -2.13 | -1.96 | -2.01 | -1.77 | -2.26 | -1.76 | -2.14 |
| 307 | -2.14 | -2.25 | -2.09 | -2.13 | -1.82 | -2.42 | -1.86 | -2.31 |
| 308 | -2.12 | -2.19 | -2.08 | -2.07 | -1.77 | -2.36 | -1.85 | -2.22 |
| 309 | -2.11 | -2.20 | -2.05 | -2.07 | -1.77 | -2.33 | -1.91 | -2.22 |
| 310 | -2.11 | -2.19 | -2.04 | -2.08 | -1.74 | -2.33 | -1.97 | -2.22 |
| 311 | -2.11 | -2.20 | -2.06 | -2.13 | -1.77 | -2.36 | -2.04 | -2.26 |
| 312 | -2.15 | -2.23 | -2.12 | -2.19 | -1.78 | -2.44 | -2.08 | -2.34 |
| 313 | -2.06 | -2.10 | -2.04 | -2.14 | -1.62 | -2.35 | -1.98 | -2.26 |
| 314 | -1.88 | -1.85 | -1.83 | -2.05 | -1.38 | -2.10 | -1.83 | -2.06 |
| 315 | -1.67 | -1.57 | -1.59 | -1.95 | -1.16 | -1.80 | -1.66 | -1.80 |
| 316 | -1.56 | -1.40 | -1.47 | -1.93 | -1.13 | -1.62 | -1.62 | -1.65 |
| 317 | -1.56 | -1.40 | -1.49 | -1.99 | -1.26 | -1.62 | -1.65 | -1.66 |
| 318 | -1.57 | -1.44 | -1.55 | -1.99 | -1.38 | -1.69 | -1.63 | -1.72 |
| 319 | -1.49 | -1.36 | -1.49 | -1.93 | -1.32 | -1.63 | -1.51 | -1.63 |
| 320 | -1.44 | -1.33 | -1.49 | -1.91 | -1.32 | -1.57 | -1.45 | -1.64 |
| 321 | -1.48 | -1.42 | -1.54 | -1.89 | -1.46 | -1.58 | -1.51 | -1.79 |
| 322 | -1.53 | -1.56 | -1.58 | -1.86 | -1.70 | -1.62 | -1.64 | -1.99 |
| 323 | -1.50 | -1.64 | -1.56 | -1.76 | -1.87 | -1.66 | -1.70 | -2.11 |
| 324 | -1.45 | -1.65 | -1.52 | -1.68 | -1.92 | -1.67 | -1.70 | -2.12 |
| 325 | -1.38 | -1.61 | -1.49 | -1.66 | -1.84 | -1.61 | -1.65 | -2.05 |
| 326 | -1.37 | -1.61 | -1.53 | -1.70 | -1.84 | -1.60 | -1.64 | -2.08 |
| 327 | -1.39 | -1.64 | -1.55 | -1.73 | -1.82 | -1.61 | -1.62 | -2.08 |
| 328 | -1.43 | -1.67 | -1.59 | -1.77 | -1.84 | -1.63 | -1.65 | -2.15 |
| 329 | -1.43 | -1.66 | -1.58 | -1.76 | -1.87 | -1.64 | -1.67 | -2.13 |

Analysis was then conducted on the NS 1 proteins as described in Example 1 to compare predicted B cell linear epitopes to the predicted B cell linear epitopes in the proteins of the human proteome which have a function related to cardiovascular function. Human proteins were selected for inclusion in this comparison if they were annotated in UniProt with one of the key words shown in Table 15 indicative of a function in cardiovascular physiology or vascular endotheilial integrity.

**Table 15: Cardiovascular key words**

| | | | |
|---|---|---|---|
| acetyl-transferring | endoplasmin | heme-binding | thrombopoietin |
| alpha-2-antiplasmin | endoplasmin-like | hemochromatosis | thrombospondin |
| alpha-hemoglobin-stabilizing | endothelial | hemofiltrate | thrombospondin-1 |
| angio-associated | endothelin | hemogen | thrombospondin-2 |
| angiogenesis | endothelin-1 | hemoglobin | thrombospondin-3 |
| angiogenic | endothelin-2 | hemojuvelin | thrombospondin-4 |
| angiogenin | endothelin-3 | hemopexin | thrombospondin-type |
| angiomotin | endothelin-converting | lactotransferrin | thromboxane |
| angiomotin-like | envoplakin | lipoma-preferred | thromboxane-a |
| angiopoietin-1 | envoplakin-like | lvv-hemorphin-7 | transferrin |
| angiopoietin-2 | epiplakin | melanotransferrin | uroplakin-1a |
| angiopoietin-4 | erythroblast | microfibril-associated | uroplakin-1b |
| angiopoietin-like | erythrocyte | microfibrillar-associated | uroplakin-2 |
| angiopoietin-related | erythroid | mitoferrin-1 | uroplakin-3a |
| angiostatin | erythropoietic | mitoferrin-2 | uroplakin-3b |
| angiotensin | erythropoietin | neuferricin | uroplakin-3b-like |
| angiotensin-converting | ferredoxin | nucleoplasmin-2 | vascular |
| angiotensinogen | ferredoxin-fold | nucleoplasmin-3 | vasculin |
| antigen_chemokine | ferric-chelate | periplakin | vasculin-like |
| antithrombin-iii | ferritin | plakoglobin | vasoactive |
| ceruloplasmin | ferrochelatase | plakophilin-1 | vasodilator-stimulated |
| chemokine | fibrillarin | plakophilin-2 | vasohibin-1 |
| chemokine-like | fibrillarin-like | plakophilin-3 | vasohibin-2 |
| chemokine-related | fibrillary | plakophilin-4 | vasopressin |
| chemotactic | fibrillin-1 | plasminogen | vasopressin-induced |
| chemotaxin | fibrillin-2 | plasminogen-like | vasopressin-neurophysin |
| chemotaxin-2 | fibrillin-3 | platelet | vasorin |
| chemotaxis | fibrinogen | platelet-activating | vwf |
| coagulation | fibrinogen-like | platelet-derived | vwfa |
| c-reactive | gamma-glutamylcyclotransferas e | prothrombin | willebrand |
| cyclotransferase | hematological | protoheme | williams-beuren |
| cyclotransferase-like | hematopoietic | sarcoplasmic_endoplasmi c | |
| desmoplakin | hematopoietically-expressed | serotransferrin | |
| endoplasmic | heme | thrombomodulin | |

Peptide pentamer motifs were identified in flaviviruses which matched pentamer motifs in the cardiovascular protein set, where in both cases the pentamer occurred in a predicted linear B cell epitope. The resulting list was manually curated to exclude proteins which contained terms such as "domain containing" and to identify the proteins actually verified as related to or expressed in blood coagulation, platelets, endothelial cells and erythrocytes.

Accession numbers of viruses used in identifying these were as shown in Table 16. Additional strains/isolates of all were used to evaluate conservation. Table 17 shows peptides found in dengue, Zika, and Usutu virus NS1 which have mimics in the human cardiovascular set proteins and which fulfill the B cell epitope criteria.

**Table 16: Accession numbers of viruses analyzed**

| Flavivirus | | Polyprotein gi | Polyprotein accession | Nucleotide gi | DB Source accession |
|---|---|---|---|---|---|
| Zika | Brazil SPH2015 | 969945757 | ALU33341.1 | 969945756 | KU321639.1 |
| Zika | Senegal ArD158084 | 592746966 | AHL43504.1 | 592746965 | KF383119.1 |
| Dengue 1 | Nauru/West Pac/1974 | 1854039 | AAB70695.1 | 1854038 | U88536.1 |
| Dengue 1 | Brazil 12898/BR-PE/10 | 511782627 | AGN94866.1 | 5117826276 | JX669462.1 |
| Dengue 2 | Thailand/16681/84 | 323473 | AAA73185.1 | 323472 | M84727.1 |
| Dengue 2 | Brazil 9479BR-PE/10 | 511782661 | AGN94883.1 | 511782660 | JX669479.1 |
| Dengue 3 | Philippines 1956/ H87 | 961377532 | ALS05358.1 | 961377531 | KU050695.1 |
| Dengue 3 | Brazil 2009 D3BR/AL95/2009 | 389565793 | AFK83755.1 | 389565792 | JF808120.1 |
| Dengue 4 | Thailand/0476/1997 | 53653743 | AAU89375.1 | 53653742 | AY618988.1 |
| Dengue 4 | Brazil DENV-4/BEL83791 | 418715828 | AFX65871.1 | 418715827 | JQ513335.1 |
| Yellow fever | Live Attenuated Yellow Fever Vaccine 17D-204 | 564014615 | AHB63684.1 | 564014614 | KF769015.1 |
| Yellow fever | Peru 2007 "case #2" | 256274854 | ACU68590.1 | 256274853 | GQ379163.1 |
| West Nile | West Nile Virus 04-216CO | 90025138 | ABD85073.1 | 90025137 | DQ431702.1 |
| Japanese encephalitis | JEV SA-14 | 331332 | AAA46248.1 | 331331 | M55506.1 |
| Tick-borne encephalitis | TBEV Neudoerfl | 975238 | AAA86870.1 | 975237 | U27495.1 |
| Usutu | Usutu virus strain Italia 2009 | 339831600 | AEK21245.1 | 339831599 | JF266698 |

**Table 17: Epitope mimics in NS 1 proteins**

| Virus | Human protein annotation (short) | Virus B cell probability## | Proteome B cell probability## | query penta | SEQ ID NO: |
|---|---|---|---|---|---|
| DEN1 | A disintegrin and metalloproteinase with thrombospondin motifs 13 ADAMTS13 | -1.12 | -0.23 | SLRTT | 156 |
| DEN2 | A disintegrin and metalloproteinase with thrombospondin motifs 13 ADAMTS13 | -1.45 | -0.23 | SLRTT | 156 |
| DEN3 | A disintegrin and metalloproteinase with thrombospondin motifs 13 ADAMTS13 | -1.19 | -0.23 | SLRTT | 156 |
| DEN4 | A disintegrin and metalloproteinase with thrombospondin motifs 13 ADAMTS13 | -1.34 | -0.23 | SLRTT | 156 |
| DEN3 | Coagulation factor V | -0.26 | -1.01 | ASRAW | 157 |
| DEN3 | Coagulation factor VIII | -0.72 | -0.25 | IDGPS | 158 |
| DEN4 | Coagulation factor VIII | -0.50 | -0.57 | KGKRA | 159 |
| DEN4 | Plasminogen | -1.09 | -0.21 | IFTPE | 160 |
| DEN1 | Plasminogen | -0.94 | -1.03 | TTVTG | 161 |
| DEN3 | Platelet glycoprotein Ib beta chain | -0.84 | -1.34 | SLAGP | 162 |
| ZIKV | Platelet glycoprotein Ib beta chain | -0.79 | -1.34 | SLAGP | 162 |
| DEN3 | Vascular endothelial growth factor A | -0.62 | -1.19 | SASRA | 163 |
| ZIKV | Vascular endothelial growth factor B | -1.51 | -1.64 | PDSPR | 164 |
| DEN2 | Vascular endothelial growth factor receptor 1 | -0.67 | -0.80 | AGKRS | 165 |
| DEN3 | Vascular endothelial growth factor receptor 1 | -0.58 | -1.06 | LEQGK | 166 |
| DEN4 | Vascular endothelial growth factor receptor 2 | -0.52 | -0.43 | KNSTF | 167 |
| ZIKV | von Willebrand factor | -0.53 | -0.97 | EECPG | 168 |
| ZIKV | von Willebrand factor | -0.86 | -0.15 | EETCG | 169 |
| ZIKV | von Willebrand factor | -0.64 | -0.46 | VEETC | 170 |
| USUV | Platelet endothelial aggregation receptor 1 | -0.93 | -0.98 | SSGRL | 171 |
| USUV | Platelet glycoprotein Ib beta chain | -1.01 | -1.72 | LAGPR | 172 |

| | | | | | |
|---|---|---|---|---|---|
| ## B cell probabilities are shown in inverse standard deviation units. More negative scores are more likely B cell epitopes in the corresponding protein. | | | | | |

Some of these mimics may vary depending on the strain of dengue virus, and it will be clear to those skilled in the art that adjustments may be needed on a geographic basis or over time to adapt to changes in mimics which may affect clinical outcome. However, in particular it was noted that all dengue viruses contained a conserved motif SLRTT located in the stable C terminal loop of NS1 between two cysteine bonds (*45*) at positions 290-311 of the NS1 protein which corresponds to a motif in the C terminal region of ADAMTS13. ADAMTS13 is expressed in endothelial cells and is essential to cleavage to von Willebrand factor. A deficiency of ADAMTS13 is associated with accumulation of multimers of von Willebrand factor, intravascular platelet aggregation, and thrombocytopenia, both congenital and acquired (*46, 47*). ADAMTS is expressed in endothelial cells. Other motifs were found in coagulation factors V and VIII, von Willebrand factor and in platelet glycoprotein 1B beta which is also associated with acquired autoimmune thrombocytopenia (*48*) and is expressed in both platelets and endothelial cells. Notably these epitope mimic motifs for cardiovascular function proteins are not present in West Nile virus.

Development of transient autoimmunity to these motifs may arise on initial dengue infection but be exacerbated on re-exposure to a further dengue serotype, potentially further boosted by antibody dependent enhancement, thereby contributing to hemorrhagic signs characteristic of dengue hemorrhagic fever. It would be beneficial to remove such epitopes in a vaccine containing NS1 to preclude sensitization to an anamnestic autoimmune response on exposure to wildtype virus of any of the dengue serotypes.

### Example 8: Diagnosis of antibody mediated autoimmune diseases of unknown etiology

Diagnosing the basis of mimicry in an antibody mediated autoimmune disease where the initial exogenous driver of immunity and antibody development is not known is a complex task. As indicated in some of the preceding examples the challenge is to identify the commonality between B cell epitopes in an exogenous protein, which may be unknown at the time of patient presentation, and a B cell epitope in a human protein, dysfunction of which is leading to the clinical signs, directly or indirectly. In one approach to this challenge, a microarray is prepared which displays peptides to which antibodies from the subject will bind. As the total number of possible pentamers comprising core peptides of B cell linear epitopes is 3.2 million in an ideal situation all 3.2 million would be arrayed. This has practical limitations and therefore a subset may be selected based on the presenting clinical signs or an array of longer peptides, for instance 15mers or 20 mers can be used each of which comprises multiple pentamers which can be further dissected. Identification of binding to one or many peptides created a more limited set of motifs which can then be searched in both the human proteome B cell epitope database created (Example 1) and in a microbiome or virome of interest and further analyzed.

### Example 9: Epitope matches in the murine proteome

The B cell epitope peptides in the murine proteome were computed using the process described in Example 1. The analysis was based on the reference mouse proteome documented in Uniprot uniprot.org/proteomes/UP000000589 which is for the C57BL/6J mouse. This proteome, with isoforms, comprises 58,430 proteins. 75% of the mouse genes are in 1:1 orthologous relationships to human genes and have most likely maintained their ancestral function in both species; however, this does not imply the protein sequences and thus B cell epitopes are the same.

As an example of the differences in mimic matches in murine and human proteome we compared matches with B cell epitopes in the envelope protein of Zika virus. Table 18 shows the similarities and differences of epitope mimics between human and murine proteomes across just 9 amino acids of the Zika envelope (strain SPH2015), comprising 5 possible pentamer motifs. For clarity records for duplicate entries (as isoforms) are not shown in Table 18. Even allowing for differences in annotations of proteins there is clearly a wide difference between the two proteomes. This provides an illustration of how over a whole protein or microbial proteome the potential for divergence in mimic matches among species is vast and may have a significant impact on the clinical disease syndrome seen in each species.

**Table 18**

| Human proteome matches | | | | | |
|---|---|---|---|---|---|
| query BEPI | proteome SG15 JSb PredBEPI | query penta | SEQ ID NO: | protein annotation (short) | UniProt ID |
| -1.42 | -0.74 | ITEST | 173 | Contactin-5 | CNTN5_ HUMAN |
| -1.42 | -0.83 | ITEST | 173 | Dual specificity tyrosine-phosphorylation-regulated kinase 2 | DYRK2_ HUMAN |
| -1.42 | -0.71 | ITEST | 173 | Mucin-16 | MUC16_ HUMAN |
| -1.42 | -1.12 | ITEST | 173 | Peroxisomal multifunctional enzyme type 2 | E7EPL9_ HUMAN |
| | | | | | |
| -1.59 | -1.61 | TESTE | 127 | Ankyrin-2 | ANK2_H UMAN |
| -1.59 | -1.47 | TESTE | 127 | DENN domain-containing protein 2A | DEN2A_ HUMAN |
| -1.59 | -0.71 | TESTE | 127 | Diffuse panbronchiolitis critical region protein 1 | E9PEI6_ HUMAN |
| -1.59 | -0.86 | TESTE | 127 | Histone-lysine N-methyltransferase 2C | KMT2C_ HUMAN |
| -1.59 | -1.62 | TESTE | 127 | II,6ST nirs variant 6 | Q5FC02_ HUMAN |
| -1.59 | -1.41 | TESTE | 127 | Interphotoreceptor matrix proteoglycan 1 | IMPG1_ HUMAN |
| -1.59 | -1.33 | TESTE | 127 | Leucine-rich repeat-containing protein 53 | LRC53_H UMAN |
| -1.59 | -1.07 | TESTE | 127 | Synaptogyrin-1 | F8WCE4 _HUMA N |
| -1.59 | -2.15 | TESTE | 127 | TBC1 domain family member 8B | J3KN75_ HUMAN |
| -1.59 | -1.31 | TESTE | 127 | Uncharacterized protein C7orf65 | CG065_H UMAN |
| | | | | | |
| -1.50 | -1.05 | ESTEN | 128 | E3 ubiquitin-protein ligase TRIP12 | TRIPC_H UMAN |
| -1.50 | -0.52 | ESTEN | 128 | Leucine-rich repeat-containing protein 37A | L37A1_H UMAN |
| -1.50 | -0.52 | ESTEN | 128 | Leucine-rich repeat-containing protein 37A2 | L37A2_H UMAN |
| -1.50 | -0.53 | ESTEN | 128 | Leucine-rich repeat-containing protein 37A3 | L37A3_H UMAN |
| -1.50 | -0.55 | ESTEN | 128 | Pro-neuropeptide Y | NPY_HU MAN |
| -1.50 | -0.78 | ESTEN | 128 | Protein CBFA2T2 | MTG8R _HUMAN |
| -1.50 | -1.70 | ESTEN | 128 | Protein LAP2 | LAP2_H UMAN |
| -1.50 | -2.19 | ESTEN | 128 | Serine_threonine-protein kinase mTOR | MTOR_H UMAN |
| -1.50 | -1.59 | ESTEN | 128 | Titin | TITIN_H UMAN |
| -1.50 | -1.55 | ESTEN | 128 | Uncharacterized protein | M0QXV0 _HUMA N |
| -1.50 | -1.09 | ESTEN | 128 | Zinc finger protein 292 | ZN292_H UMAN |
| | | | | | |
| -1.29 | -1.23 | STENS | 130 | Apoptosis-stimulating of p53 protein 2 | ASPP2_H UMAN |
| -1.29 | -1.09 | STENS | 130 | Dentin matrix acidic phosphoprotein 1 | DMP1_H UMAN |
| -1.29 | -1.72 | STENS | 130 | DNA repair protein complementing XP-G cells | ERCC5_ HUMAN |
| -1.29 | -1.89 | STENS | 130 | Dual 3' | PDE11_H UMAN |
| -1.29 | -2.37 | STENS | 130 | Duffy antigen_chemokine receptor | ACKR1_ HUMAN |
| -1.29 | -1.10 | STENS | 130 | Msx2-interacting protein | MINT_H UMAN |
| -1.29 | -1.22 | STENS | 130 | Neurotrophin-4 | E7EP46_ HUMAN |
| -1.29 | -1.72 | STENS | 130 | Pancreatic secretory granule membrane major glycoprotein GP2 | GP2_HU MAN |
| -1.29 | -1.86 | STENS | 130 | Protein BIVM-ERCC5 (Fragment) | R4GMW 8_HUMA N |
| -1.29 | -0.55 | STENS | 130 | Protogenin | PRTG_H UMAN |
| -1.29 | -2.13 | STENS | 130 | Serine_threonine-protein kinase mTOR | B1AKP8_ HUMAN |
| -1.29 | -0.56 | STENS | 130 | Telomere-associated protein RIF 1 | RIF1_HU MAN |
| -1.29 | -2.00 | STENS | 130 | Uncharacterized protein C2orf71 | CB071_H UMAN |
| -1.29 | -1.50 | STENS | 130 | Voltage-dependent L-type calcium channel subunit beta-4 | F8WA06 _HUMA N |
| -1.29 | -1.49 | STENS | 130 | Zinc finger MYM-type protein 1 | ZMYM1_ HUMAN |
| | | | | | |
| -1.06 | -1.51 | TENSK | 174 | Disheveled-associated activator of morphogenesis 2 | DAAM2 _HUMAN |
| -1.06 | -2.28 | TENSK | 174 | Lysocardiolipin acyltransferase 1 | LCLT1_ HUMAN |
| -1.06 | -1.31 | TENSK | 174 | Misshapen-like kinase 1 | MINK1_ HUMAN |
| -1.06 | -1.94 | TENSK | 174 | Nicotinamide phosphoribosyltransferase | NAMPT _HUMAN |
| -1.06 | -1.91 | TENSK | 174 | Protein NAMPTL (Fragment) | Q5SYT8_ |
| | | | | | HUMAN |
| -1.06 | -0.63 | TENSK | 174 | von Willebrand factor A domain-containing protein 3A | VWA3A _HUMAN |

| Murine Proteome matches | | | | | |
|---|---|---|---|---|---|
| query BEPI | proteome SG15 JSb PredBEPI | query penta | | protein annotation (short) | UniProt ID |
| -1.42 | -1.52 | ITEST | 173 | Cohesin subunit SA-2 OS=Mus musculus GN=Stag2 PE=1 SV=3 | STAG2_ MOUSE |
| -1.42 | -0.73 | ITEST | 173 | Contactin-5 OS=Mus musculus GN=Cntn5 PE=1 SV=2 | CNTN5_ MOUSE |
| -1.42 | -0.93 | ITEST | 173 | Dedicator of cytokinesis protein 8 OS=Mus musculus GN=Dock8 PE=1 SV=4 | DOCK8_ MOUSE |
| -1.42 | -0.97 | ITEST | 173 | Protein inscuteable homolog OS=Mus musculus GN=Insc PE=1 SV=2 | INSC_M OUSE |
| | | | | | |
| -1.59 | -1.83 | TESTE | 127 | ADAMTS-like protein 2 OS=Mus musculus GN=Adamtsl2 PE=2 SV=1 | ATL2_M OUSE |
| -1.59 | -1.51 | TESTE | 127 | Ankyrin-2 OS=Mus musculus GN=Ank2 PE=1 SV=2 | ANK2_M OUSE |
| -1.59 | -2.09 | TESTE | 127 | FRAS 1-related extracellular matrix protein 2 OS=Mus musculus GN=Frem2 PE=1 SV=2 | FREM2_ MOUSE |
| -1.59 | -1.58 | TESTE | 127 | Huntingtin OS=Mus musculus GN=Htt PE=1 SV=2 | HD_MOU SE |
| -1.59 | -0.85 | TESTE | 127 | Lipoxygenase homology domain-containing protein 1 OS=Mus musculus GN=Loxhd1 PE=4 SV=1 | E9PVB2_ MOUSE |
| -1.59 | -1.59 | TESTE | 127 | Protein Tex15 OS=Mus musculus GN=Tex15 PE=4 SV=1 | F8VPN2_ MOUSE |
| -1.59 | -2.06 | TESTE | 127 | Ras-GEF domain-containing family member 1C OS=Mus musculus GN=Rasgef1c PE=2 SV=1 | RGF1C_ MOUSE |
| -1.59 | -1.04 | TESTE | 127 | TM2 domain-containing protein 3 OS=Mus musculus GN=Tm2d3 PE=2 SV=1 | TM2D3_ MOUSE |
| -1.59 | -1.13 | TESTE | 127 | Tubby-related protein 2 OS=Mus musculus GN=Tulp2 PE=1 SV=3 | TULP2_ MOUSE |
| -1.59 | -1.73 | TESTE | 127 | Voltage-dependent N-type calcium channel subunit alpha-1B OS=Mus musculus GN=Cacna1b PE=1 SV=1 | CAC1B_ MOUSE |
| | | | | | |
| -1.50 | -1.09 | ESTEN | 128 | E3 ubiquitin-protein ligase TRIP 12 OS=Mus musculus GN=Trip12 PE=1 SV=1 | TRIPC_M OUSE |
| -1.50 | -1.15 | ESTEN | 128 | Histone-lysine N-methyltransferase 2E OS=Mus musculus GN=Kmt2e | KMT2E_ MOUSE |
| | | | | PE=1 SV=2 | |
| -1.50 | -1.35 | ESTEN | 128 | Inhibitor of nuclear factor kappa-B kinase-interacting protein OS=Mus musculus GN=Ikbip PE=1 SV=2 | IKIP_MO USE |
| -1.50 | -1.31 | ESTEN | 128 | KN motif and ankyrin repeat domain-containing protein 2 OS=Mus musculus GN=Kank2 PE=1 SV=1 | KANK2_ MOUSE |
| -1.50 | -0.84 | ESTEN | 128 | Pro-neuropeptide Y OS=Mus musculus GN=Npy PE=1 SV=2 | NPY_MO USE |
| -1.50 | -1.62 | ESTEN | 128 | Protein 5330417C22Rik OS=Mus musculus GN=5330417C22Rik PE=1 SV=1 | A0A0A0 MQC6_M OUSE |
| -1.50 | -0.81 | ESTEN | 128 | Protein CBFA2T2 OS=Mus musculus GN=Cbfa2t2 PE=1 SV=3 | MTG8R_ MOUSE |
| -1.50 | -1.34 | ESTEN | 128 | Protein PRRC2C OS=Mus musculus GN=Prrc2c PE=1 SV=3 | PRC2C_ MOUSE |
| -1.50 | -1.35 | ESTEN | 128 | Telomere-associated protein RIF 1 OS=Mus musculus GN=Rif1 PE=1 SV=2 | RIF1_MO USE |
| -1.50 | -1.55 | ESTEN | 128 | Titin OS=Mus musculus GN=Ttn PE=1 SV=1 | TITIN_M OUSE |
| -1.50 | -1.62 | ESTEN | 128 | UPF0577 protein KIAA1324 OS=Mus musculus GN=Kiaa1324 PE=1 SV=1 | K1324_M OUSE |
| -1.50 | -0.76 | ESTEN | 128 | Zinc finger protein 106 OS=Mus musculus GN=Znf106 PE=1 SV=3 | ZN106_M OUSE |
| -1.50 | -1.02 | ESTEN | 128 | Zinc finger protein 292 OS=Mus musculus GN=Zfp292 PE=1 SV=2 | ZN292_M OUSE |
| | | | | | |
| -1.29 | -1.30 | STENS | 130 | Apoptosis-stimulating of p53 protein 2 OS=Mus musculus GN=Tp53bp2 PE=1 SV=3 | ASPP2_M OUSE |
| -1.29 | -1.79 | STENS | 130 | Dual 3' | PDE11_M OUSE |
| -1.29 | -0.90 | STENS | 130 | E3 ubiquitin-protein ligase RNF185 OS=Mus musculus GN=Rnf185 PE=2 SV=1 | RN185_M OUSE |
| -1.29 | -1.36 | STENS | 130 | Melanoma inhibitory activity protein 2 OS=Mus musculus GN=Mia2 PE=1 SV=2 | MIA2_M OUSE |
| -1.29 | -0.86 | STENS | 130 | Synphilin-1 OS=Mus musculus GN=Sncaip PE=2 SV=2 | SNCAP_ MOUSE |
| -1.29 | -1.21 | STENS | 130 | Telomere-associated protein RIF1 OS=Mus musculus GN=Rifl PE=1 SV=2 | RIF1_MO USE |
| -1.29 | -0.81 | STENS | 130 | Testis-expressed sequence 22 protein OS=Mus musculus GN=Tex22 PE=1 SV=1 | TEX22_ MOUSE |
| -1.29 | -1.20 | STENS | 130 | Ubiquilin-3 OS=Mus musculus GN=Ubqln3 PE=1 SV=1 | UBQL3_ MOUSE |
| -1.29 | -1.36 | STENS | 130 | Voltage-dependent L-type calcium channel subunit beta-4 OS=Mus musculus GN=Cacnb4 PE=1 SV=1 | J3QK20_ MOUSE |
| -1.29 | -1.35 | STENS | 130 | Voltage-dependent L-type calcium channel subunit beta-4 OS=Mus musculus GN=Cacnb4 PE=1 SV=2 | CACB4_ MOUSE |
| -1.29 | -0.82 | STENS | 130 | Zinc finger and BTB domain-containing protein 9 OS=Mus musculus GN=Zbtb9 PE=2 SV=1 | ZBTB9_ MOUSE |
| | | | | | |
| -1.06 | -1.20 | TENSK | 174 | Breast carcinoma-amplified sequence 1 homolog OS=Mus musculus GN=Bcas1 PE=1 SV=3 | BCAS1_ MOUSE |
| -1.06 | -1.44 | TENSK | 174 | Disheveled-associated activator of morphogenesis 2 OS=Mus musculus GN=Daam2 PE=1 SV=4 | DAAM2_ MOUSE |
| -1.06 | -1.37 | TENSK | 174 | Misshapen-like kinase 1 OS=Mus musculus GN=Mink1 PE=1 SV=3 | MINK1_ MOUSE |
| -1.06 | -2.05 | TENSK | 174 | Nicotinamide phosphoribosyltransferase OS=Mus musculus GN=Nampt PE=1 SV=1 | NAMPT_ MOUSE |
| -1.06 | -0.54 | TENSK | 174 | Testis anion transporter 1 OS=Mus musculus GN=Slc26a8 PE=2 SV=2 | S26A8_M OUSE |
| -1.06 | -0.65 | TENSK | 174 | von Willebrand factor A domain-containing protein 3A OS=Mus musculus GN=Vwa3a PE=2 SV=1 | VWA3A_ MOUSE |

### Example 10: Determination of epitopes in viruses that match a Parkinson's Disease proteome filter

Parkinson's disease is a chronic neurodegenerative disease characterized by the accumulation of aggregates of alpha synuclein as Lewy bodies, located in motor neurons of the midbrain. The mechanism leading to the alpha synuclein accumulation is not understood. A large number of other proteins have been examined for their association with the etiology of Parkinson's disease. In order to examine whether commonly occurring viruses may have any role in autoimmune mechanisms contributing to Parkinson's and related alpha synucleinopathies, we assembled a panel of the associated proteins in which the probable B cell epitope peptides were identified. The proteins included are shown in Table 19. These proteins were selected based on review of the literature and the Uniprot annotations indicating associations with Parkinson's disease. The epitopes in these human proteins were then compared to a set of potential candidate viromes, comprising common, non-arbovirus, causes of viral encephalitis, including herpes simplex 1 and 2, cytomegalovirus, and measles.

**Table 19: Parkinson's disease and other alphasynucleinopathy associated proteins**

| Uniprot identifier | Uniprot Name | Protein names | Gene names |
|---|---|---|---|
| O60733 | PLPL9_HUMAN | 85/88 kDa calcium-independent phospholipase A2 | PLA2G6 PLPLA9 |
| P37840 | SYUA_HUMAN | Alpha-synuclein | SNCA NACP PARK1 |
| Q9Y6H1 | CHCH2_HUMA N | Coiled-coil-helix-coiled-coil-helix domain-containing protein 2 | CHCHD2 C7orf17 AAG10 |
| 075165 | DJC13_HUMAN | DnaJ homolog subfamily C member | DNAJC13 KIAA0678 RME8 |
| O60260 | PRKN2_HUMAN | E3 ubiquitin-protein ligase parkin (Parkin) | PARK2 PRKN |
| B1AKC3 | B1AKC3_HUMA N | E3 ubiquitin-protein ligase parkin (Parkinson protein 2 E3 ubiquitin protein ligase isoform 2) | PARK2 |
| Q04637 | IF4G1_HUMAN | Eukaryotic translation initiation factor 4 gamma 1 | EIF4G1 EIF4F EIF4G EIF4GI |
| Q9Y3I1 | FBX7_HUMAN | F-box only protein 7 | FBXO7 FBX7 |
| Q9NP95 | FGF20_HUMAN | Fibroblast growth factor 20 | FGF20 |
| P04062 | GLCM_HUMAN | Glucosylceramidase | GBA GC GLUC |
| Q5S007 | LRRK2_HUMAN | Leucine-rich repeat serine/threonine-protein kinase 2 (Dardarin) | LRRK2 PARK8 |
| P10636 | TAU_HUMAN | Microtubule-associated protein tau (Neurofibrillary tangle protein) | MAPT MAPTL MTBT1 TAU |
| Q9NQ 11 | AT132_HUMAN | Probable cation-transporting ATPase 13A2 | ATP13A2 PARK9 |
| 075061 | AUXI_HUMAN | Putative tyrosine-protein phosphatase auxilin | DNAJC6 KIAA0473 |
| O43464 | HTRA2_HUMA N | Serine protease HTRA2, mitochondrial | HTRA2 OMI PRSS25 |
| Q9BXM 7 | PINK1_HUMAN | Serine/threonine-protein kinase PINK1, mitochondrial | PINK 1 |
| O43426 | SYNJ1_HUMAN | Synaptojanin-1 | SYNJ1 KIAA0910 |
| Q9BT88 | SYT11_HUMAN | Synaptotagmin-11 | SYT11 KIAA0080 |
| Q96A57 | TM230_HUMAN | Transmembrane protein 230 | TMEM230 C20orf30 HSPC274 UNQ2432/PRO499 2 |
| P09936 | UCHL1_HUMA N | Ubiquitin carboxyl-terminal hydrolase isozyme L1 | UCHL1 |
| Q709C8 | VP13C_HUMAN | Vacuolar protein sorting-associated protein 13C | VPS13C KIAA1421 |
| Q96QK1 | VPS35_HUMAN | Vacuolar protein sorting-associated protein 35 | VPS35 MEM3 TCCCTA00141 |
| 014874 | BCKD_HUMAN | [3-methyl-2-oxobutanoate dehydrogenase [lipoamide]] kinase, mitochondrial | BCKDK |
| Q8TDX5 | ACMSD_HUMA N | 2-amino-3-carboxymuconate-6-semialdehyde decarboxylase (Picolinate carboxylase) | ACMSD |
| Q96D46 | NMD3_HUMAN | 60S ribosomal export protein NMD3 | NMD3 CGI-07 |
| Q07912 | ACK1_HUMAN | Activated CDC42 kinase 1 (ACK-1) (Tyrosine kinase non-receptor protein 2) | TNK2 ACK1 |
| Q10588 | BST1_HUMAN | ADP-ribosyl cyclase/cyclic ADP-ribose hydrolase | BST1 |
| Q6P9F0 | CCD62_HUMAN | Coiled-coil domain-containing protein 62 (Protein TSP-NY) | CCDC62 |
| Q8NA47 | CCD63_HUMAN | Coiled-coil domain-containing protein 63 | CCDC63 |
| 014976 | GAK_HUMAN | Cyclin-G-associated kinase | GAK |
| P52824 | DGKQ_HUMAN | Diacylglycerol kinase theta (DAG kinase theta) (Diglyceride kinase theta) (DGK-theta) | DGKQ DAGK4 |
| Q15700 | DLG2_HUMAN | Disks large homolog 2 (Channel-associated protein of synapse-110) (Chapsyn-110) (Postsynaptic density protein PSD-93) | DLG2 |
| Q9BSA9 | TM175_HUMAN | Endosomal/lysomomal potassium channel TMEM175 (Transmembrane protein 175) | TMEM175 |
| P30793 | GCH1_HUMAN | GTP cyclohydrolase 1 (GTP cyclohydrolase I) (GTP-CH-I) | GCH1 DYT5 GCH |
| Q99578 | RIT2_HUMAN | GTP-binding protein Rit2 (Ras-like protein expressed in neurons) (Ras-like without CAAX protein 2) | RIT2 RIN ROC2 |
| Q9NR48 | ASH1L_HUMAN | Histone-lysine N-methyltransferase ASH1L (ASH1-like protein) (huASH1) (Absent small and homeotic disks protein 1 homolog) (Lysine N-methyltransferase 2H) | ASH1L KIAA1420 KMT2H |
| 075146 | HIP1R_HUMAN | Huntingtin-interacting protein 1-related protein (HIP1-related protein) (Huntingtin-interacting protein 12) (HIP-12) | HIP1R HIP12 KIAA0655 |
| Q01968 | OCRL_HUMAN | Inositol polyphosphate 5-phosphatase OCRL-1 (Lowe oculocerebrorenal syndrome protein) | OCRL INPP5F OCRL1 |
| P53708 | ITA8_HUMAN | Integrin alpha-8 [Cleaved into: Integrin alpha-8 heavy chain; Integrin alpha-8 light chain] | ITGA8 |
| Q14108 | SCRB2_HUMAN | Lysosome membrane protein 2 | SCARB2 CD36L2 LIMP2 LIMPII |
| Q9UQV 4 | LAMP3_HUMA N | Lysosome-associated membrane glycoprotein 3 (LAMP-3) | LAMP3 DCLAMP TSC403 |
| P51512 | MMP16_HUMA N | Matrix metalloproteinase-16 (MMP-16) | MMP 16 MMPX2 |
| Q96RQ3 | MCCA_HUMAN | Methylcrotonoyl-CoA carboxylase subunit alpha, mitochondrial (MCCase subunit alpha) | MCCC1 MCCA |
| Q6GTS8 | P20D1_HUMAN | N-fatty-acyl-amino acid synthase/hydrolase PM20D1 (Peptidase M20 domain-containing protein 1) | PM20D1 |
| Q9H1E3 | NUCKS_HUMA N | Nuclear ubiquitous casein and cyclin-dependent kinase substrate 1 | NUCKS1 NUCKS JC7 |
| Q6ZV65 | FA47E_HUMAN | Protein FAM47E | FAM47E |
| P57735 | RAB25_HUMAN | Ras-related protein Rab-25 (CATX-8) | RAB25 CATX8 |
| O75787 | RENR_HUMAN | Renin receptor (Renin/prorenin receptor) (Vacuolar ATP synthase membrane sector-associated protein M8-9) (ATP6M8-9) | ATP6AP2 ATP6IP2 CAPER ELDF 10 HT028 MSTP009 PSEC0072 |
| 094941 | RNF37_HUMAN | RING finger protein 37 (Ubiquitin-conjugating enzyme 7-interacting protein 5) | UBOX5 KIAA0860 RNF37 UBCE7IP5 UIP5 |
| Q8IWL8 | STH_HUMAN | Saitohin | STH |
| Q9P2F8 | SI1L2_HUMAN | Signal-induced proliferation-associated 1-like protein 2 (SIPA1-like protein 2) | SIPA1L2 KIAA1389 |
| Q9UEW 8 | STK39_HUMAN | STE20/SPS1-related proline-alanine-rich protein kinase (Ste-20-related kinase) (DCHT) (Serine/threonine-protein kinase 39) | STK39 SPAK |
| P36956 | SRBP1_HUMAN | Sterol regulatory element-binding protein 1 (SREBP-1 | SREBF1 BHLHD1 SREBP1 |
| Q92752 | TENR_HUMAN | Tenascin-R (TN-R) (Janusin) (Restrictin) | TNR |
| Q14956 | GPNMB_HUMA N | Transmembrane glycoprotein NMB (Transmembrane glycoprotein HGFIN) | GPNMB HGFIN NMB UNQ1725/PRO992 5 |
| Q7Z410 | TMPS9_HUMAN | Transmembrane protease serine 9 (Polyserase-I) | TMPRSS9 |
| Q8NBD 8 | T229B_HUMAN | Transmembrane protein 229B | TMEM229B C14orf83 |
| Q9UHP3 | UBP25_HUMAN | Ubiquitin carboxyl-terminal hydrolase 25 (Ubiquitin-specific-processing protease 25) | USP25 USP21 |

| *Additional proteins selected based on Uniprot annotations* | | | |
|---|---|---|---|
| Q9UGJ0 | AAKG2_HUMA N | 5'-AMP-activated protein kinase subunit gamma-2 (AMPK gamma2) | PRKAG2 |
| Q13155 | AIMP2_HUMAN | Aminoacyl tRNA synthase complex-interacting multifunctional protein 2 | AIMP2 JTV1 PRO0992 |
| P18859 | ATP5J_HUMAN | ATP synthase-coupling factor 6, mitochondrial (ATPase subunit F6) | ATP5J ATP5A ATPM |
| Q16143 | SYUB_HUMAN | Beta-synuclein | SNCB |
| P23560 | BDNF_HUMAN | Brain-derived neurotrophic factor (BDNF) (Abrineurin) | BDNF |
| Q6YNR 1 | Q6YNR1_HUMA N | Brain-derived neurotrophic factor BDNF7 | BDNF |
| Q03135 | CAV1_HUMAN | Caveolin-1 | CAV1 CAV |
| B7Z1J9 | B7Z1J9_HUMAN | cDNA FLJ53027, highly similar to Mus musculus Parkinson disease 7 domain containing 1 (Pddc1), mRNA | |
| Q9UQN3 | CHM2B_HUMAN | Charged multivesicular body protein 2b (CHMP2.5 | CHMP2B CGI-84 |
| O14810 | CPLX1_HUMAN | Complexin-1 (Complexin I) (CPX I) (Synaphin-2) | CPLX1 |
| Q96PZ7 | CSMD1_HUMAN | CUB and sushi domain-containing protein 1 (CUB and sushi multiple domains protein 1) | CSMD1 KIAA1890 UNQ5952/PRO198 63 |
| Q00535 | CDK5_HUMAN | Cyclin-dependent-like kinase 5 (Tau protein kinase II catalytic subunit) (TPKII catalytic subunit) | CDK5 CDKN5 |
| P11509 | CP2A6_HUMAN | Cytochrome P450 2A6 | CYP2A6 CYP2A3 |
| Q9H5Q 4 | TFB2M_HUMAN | Dimethyladenosine transferase 2, mitochondrial | TFB2M NS5ATP5 |
| P78352 | DLG4_HUMAN | Disks large homolog 4 (Postsynaptic density protein 95) (PSD-95) (Synapse-associated protein 90) (SAP-90) (SAP90) | DLG4 PSD95 |
| Q9NX0 9 | DDIT4_HUMAN | DNA damage-inducible transcript 4 protein (HIF-1 responsive protein RTP801) (Protein regulated in development and DNA damage response 1) (REDD-1) | DDIT4 REDD1 RTP801 |
| P54098 | DPOG1_HUMAN | DNA polymerase subunit gamma-1 | POLG MDP1 POLG1 POLGA |
| Q9NV5 8 | RN19A_HUMAN | E3 ubiquitin-protein ligase RNF19A (Dorfin) (RING finger protein 19A) (p38) | RNF19A RNF19 |
| Q8IUQ4 | SIAH1_HUMAN | E3 ubiquitin-protein ligase SIAH1 (Seven in absentia homolog 1) (Siah-1) (Siah-1a) | SIAH1 HUMSIAH |
| Q9C026 | TRIM9_HUMAN | E3 ubiquitin-protein ligase TRIM9 (RING finger protein 91) (Tripartite motif-containing protein 9) | TRIM9 KIAA0282 RNF91 |
| Q9Y371 | SHLB1_HUMAN | Endophilin-B1 (Bax-interacting factor 1) (Bif-1) (SH3 domain-containing GRB2-like protein B1) | SH3GLB1 KIAA0491 CGI-61 |
| P05305 | EDN1_HUMAN | Endothelin-1 (Preproendothelin-1) (PPET1 | EDN1 |
| Q96CU9 | FXRD1_HUMAN | FAD-dependent oxidoreductase domain-containing protein 1 | FOXRED1 FP634 |
| P58012 | FOXL2_HUMAN | Forkhead box protein L2 | FOXL2 |
| Q9H4Y 5 | GSTO2_HUMAN | Glutathione S-transferase omega-2 (GSTO-2) | GSTO2 |
| O95263 | PDE8B_HUMAN | High affinity cAMP-specific and IBMX-insensitive 3',5'-cyclic phosphodiesterase 8B | PDE8B PIG22 |
| P25021 | HRH2_HUMAN | Histamine H2 receptor (H2R) (HH2R) (Gastric receptor I) | HRH2 |
| P20702 | ITAX_HUMAN | Integrin alpha-X (CD11 antigen-like family member C) (Leu M5) (Leukocyte adhesion glycoprotein p150,95 alpha chain) | ITGAX CD11C |
| Q8TB37 | NUBPL_HUMAN | Iron-sulfur protein NUBPL (IND1 homolog) | NUBPL C14orf127 |
| Q92876 | KLK6_HUMAN | Kallikrein-6 (Neurosin) (Protease M) (SP59) (Serine protease 18) | KLK6 PRSS18 PRSS9 |
| Q96FE5 | LIGO1_HUMAN | Leucine-rich repeat and immunoglobulin-like domain-containing nogo receptor-interacting protein 1 (Leucine-rich repeat neuronal protein 6A) | LINGO 1 LERN1 LRRN6A UNQ201/PRO227 |
| Q8N183 | MIMIT_HUMAN | Mimitin, mitochondrial (B17.2-like) (B17.2L) (Myc-induced mitochondrial protein) (MMTN) (NADH dehydrogenase [ubiquinone] | NDUFAF2 NDUFA12L |
| Q8IWA 4 | MFN1_HUMAN | Mitofusin-1 (Fzo homolog) (Transmembrane GTPase MFN1) | MFN1 |
| Q9UBU 8 | MO4L1_HUMAN | Mortality factor 4-like protein 1 (MORF-related gene 15 protein) (Protein MSL3-1) (Transcription factor-like protein MRG15) | MORF4L1 MRG15 FWP006 HSPC008 HSPC061 PP368 |
| Q15014 | MO4L2_HUMAN | Mortality factor 4-like protein 2 (MORF-related gene X protein) | MORF4L2 KIAA0026 MRGX |
| Q330K2 | NDUF6_HUMAN | NADH dehydrogenase (ubiquinone) complex I, assembly factor 6 | NDUFAF6 C8orf38 |
| Q9BU61 | NDUF3_HUMAN | NADH dehydrogenase [ubiquinone] 1 alpha subcomplex assembly factor 3 | NDUFAF3 C3orf60 |
| Q9P032 | NDUF4_HUMAN | NADH dehydrogenase [ubiquinone] 1 alpha subcomplex assembly factor 4 | NDUFAF4 C6orf66 HRPAP20 HSPC125 My013 |
| Q5TEU 4 | NDUF5_HUMAN | NADH dehydrogenase [ubiquinone] 1 alpha subcomplex assembly factor 5 | NDUFAF5 C20orf7 |
| O15239 | NDUA1_HUMA N | NADH dehydrogenase [ubiquinone] 1 alpha subcomplex subunit 1 (Complex I-MWFE) | NDUFA1 |
| Q86Y39 | NDUAB_HUMA N | NADH dehydrogenase [ubiquinone] 1 alpha subcomplex subunit 11 | NDUFA11 |
| P03886 | NU1M_HUMAN | NADH-ubiquinone oxidoreductase chain 1 (NADH dehydrogenase subunit 1) | MT-ND1 MTND1 NADH1 ND1 |
| P03897 | NU3M_HUMAN | NADH-ubiquinone oxidoreductase chain 3 (NADH dehydrogenase subunit 3) | MT-ND3 MTND3 NADH3 ND3 |
| P03915 | NU5M_HUMAN | NADH-ubiquinone oxidoreductase chain 5 (NADH dehydrogenase subunit 5) | MT-ND5 MTND5 NADH5 ND5 |
| P03923 | NU6M_HUMAN | NADH-ubiquinone oxidoreductase chain 6 (NADH dehydrogenase subunit 6) | MT-ND6 MTND6 NADH6 ND6 |
| P16435 | NCPR_HUMAN | NADPH--cytochrome P450 reductase (CPR) | POR CYPOR |
| Q6ZNJ1 | NBEL2_HUMAN | Neurobeachin-like protein 2 | NBEAL2 KIAA0540 UNQ253/PRO290 |
| P35228 | NOS2_HUMAN | Nitric oxide synthase, inducible (Hepatocyte NOS) (HEP-NOS) (Inducible NO synthase) | NOS2 NOS2A |
| P78380 | OLR1_HUMAN | Oxidized low-density lipoprotein receptor 1 (Ox-LDL receptor 1) | OLR1 CLEC8A LOX1 |
| Q96M98 | PACRG_HUMAN | Parkin coregulated gene protein (Molecular chaperone/chaperonin-binding protein) (PARK2 coregulated gene protein) | PACRG GLUP |
| Q8NB37 | PDDC1_HUMAN | Parkinson disease 7 domain-containing protein 1 | PDDC1 |
| Q9UBK 2 | PRGC1_HUMAN | Peroxisome proliferator-activated receptor gamma coactivator 1-alpha (PGC-1-alpha) (PPAR-gamma coactivator 1-alpha) | PPARGC1A LEM6 PGC1 PGC1A PPARGC1 |
| Q6Y7W 6 | PERQ2_HUMAN | PERQ amino acid-rich with GYF domain-containing protein 2 (GRB10-interacting GYF protein 2) | GIGYF2 KIAA0642 PERQ2 TNRC15 |
| O00443 | P3C2A_HUMAN | Phosphatidylinositol 4-phosphate 3-kinase C2 domain-containing subunit alpha (PI3K-C2-alpha) | PIK3C2A |
| Q92508 | PIEZ1_HUMAN | Piezo-type mechanosensitive ion channel component 1 (Membrane protein induced by beta-amyloid treatment) (Mib) | PIEZO1 FAM38A KIAA0233 |
| Q96IZ0 | PAWR_HUMAN | PRKC apoptosis WT1 regulator protein (Prostate apoptosis response 4 protein) (Par-4) | PAWR PAR4 |
| Q16342 | PDCD2_HUMAN | Programmed cell death protein 2 (Zinc finger MYND domain-containing protein 7) (Zinc finger protein Rp-8) | PDCD2 RP8 ZMYND7 |
| O15354 | GPR37_HUMAN | Prosaposin receptor GPR37 (Endothelin B receptor-like protein 1) (ETBR-LP-1) (G-protein coupled receptor 37) (Parkin-associated endothelin receptor-like receptor) (PAELR) | GPR37 |
| Q99497 | PARK7_HUMAN | Protein deglycase DJ-1 (DJ-1) (EC 3.1.2.-) (EC 3.5.1.-) (Oncogene DJ1) (Parkinson disease protein 7) | PARK7 |
| J3KSC0 | CR064_HUMAN | Putative uncharacterized protein encoded by LINC01387 | LINC01387 C18orf64 |
| Q96DA 2 | RB39B_HUMAN | Ras-related protein Rab-39B | RAB39B |
| Q9BZI7 | REN3B_HUMAN | Regulator of nonsense transcripts 3B (Nonsense mRNA reducing factor 3B) | UPF3B RENT3B UPF3X |
| Q9Y3C5 | RNF11_HUMAN | RING finger protein 11 | RNF11 CGI-123 |
| Q99719 | SEPT5_HUMAN | Septin-5 (Cell division control-related protein 1) (CDCrel-1) (Peanut-like protein 1) | SEPT5 PNUTL1 |
| Q13501 | SQSTM_HUMAN | Sequestosome-1 (EBI3-associated protein of 60 kDa) (EBIAP) (p60) | SQSTM1 ORCA OSIL |
| P51955 | NEK2_HUMAN | Serine/threonine-protein kinase Nek2 (EC 2.7.11.1) (HSPK 21) | NEK2 NEK2A NLK1 |
| Q9Y6H5 | SNCAP_HUMAN | Synphilin-1 (Sph1) (Alpha-synuclein-interacting protein) | SNCAIP |
| Q8WVP 5 | TP8L1_HUMAN | Tumor necrosis factor alpha-induced protein 8-like protein 1 (TIPE1) (TNF alpha-induced protein 8-like protein 1 | TNFAIP8L1 |
| P07101 | TY3H_HUMAN | Tyrosine 3-monooxygenase (Tyrosine 3-hydroxylase) (TH) | TH TYH |
| Q93009 | UBP7_HUMAN | Ubiquitin carboxyl-terminal hydrolase 7 (Deubiquitinating enzyme 7) | USP7 HAUSP |
| P68036 | UB2L3_HUMAN | Ubiquitin-conjugating enzyme E2 L3 (Ubiquitin-protein ligase L3) | UBE2L3 UBCE7 UBCH7 |
| P49754 | VPS41_HUMAN | Vacuolar protein sorting-associated protein 41 homolog (S53) | VPS41 |
| Q9Y4E1 | FA21C_HUMAN | WASH complex subunit FAM21C (Vaccinia virus penetration factor) (VPEF) | FAM21C KIAA0592 |
| Q9P202 | WHRN_HUMAN | Whirlin (Autosomal recessive deafness type 31 protein) | DFNB31 KIAA1526 WHRN |
| Q6NUN 9 | ZN746_HUMAN | Zinc finger protein 746 (Parkin-interacting substrate) (PARIS) | ZNF746 PARIS |

As an example of the output of such analysis, Table 20 provides an example of the epitope mimics found in measles virus that match those found in the Parkinson's disease associated proteins. The analysis was based on a recent US wildtype isolate (MiV Arizona.USA/11.08/2). This information, used alongside HLA data from a patient which would determine which virus epitopes would be likely to generate high titers is indicative of how the present invention can enable further inquiry to focus on a few proteins in seeking causal associations. A further example is provided in Table 21, where the epitope mimics in the envelope proteins of a HSV1 isolate (Kos). This result would be used as for measles above.

The examples of measles and HSV1 envelope proteins were selected in this Example simply in the interests of space (i.e. by using small virus examples). It does not imply that measles or HSV1 are primary suspects in the eitology of Parkinsons disease, but rather demonstrates an analytical approach that should in no way be considered limiting. While this example shows the application to a virus of interest; it is also indicative of how the invention can be applied to other microbial proteins or environmental antigens.

**Table 20: High probability B cell epitopes in Measles virus matching B cell epitopes in Parkinson's related proteins. In both query (measles) and proteome protein the threshold applied was the top 15% probability B cell epitopes.**

| query BEPI | proteome BEPI | query pos | Measles protein | query penta | SEQ ID NO: | protein annotation (short) | UniProt ID |
|---|---|---|---|---|---|---|---|
| -1.50 | -1.26 | 244 | H_JN635406_hemagglutinin | KGSEL | 175 | Vacuolar protein sorting-associated protein 13C | VP13C_HUMAN |
| -1.50 | -1.86 | 589 | H_JN635406_hemagglutinin | DSESG | 176 | 5'-AMP-activated protein kinase subunit gamma-2 | AAKG2_HUMAN |
| -1.31 | -1.23 | 1203 | L_JN635406_large_polymerase | IDKET | 177 | Lysosome membrane protein 2 | SCRB2_HUMAN |
| -1.47 | -1.57 | 1204 | L_JN635406_large_polymerase | DKETS | 178 | E3 ubiquitin-protein ligase parkin | PRKN2_HUMAN |
| -2.22 | -1.72 | 1355 | L_JN635406_large_polymerase | DTGSS | 179 | Whirlin | WHRN_HUMAN |
| -1.62 | -1.20 | 1651 | L_JN635406_large_polymerase | RLSPA | 180 | NADH dehydrogenase ubiquinone 1 alpha subcomplex assembly factor 3 | NDUF3_HUMAN |
| -1.36 | -1.95 | 1821 | L_JN635406_large_polymerase | SGQRE | 181 | Inositol polyphosphate 5-phosphatase OCRL-1 | OCRL_HUMAN |
| -1.19 | -1.25 | 2077 | L_JN635406_large_polymerase | RSQQG | 182 | Eukaryotic translation initiation factor 4 gamma 1 | IF4G1_HUMAN |
| -1.27 | -1.06 | 42 | M_JN635406_matrix | PGLGD | 183 | Disks large homolog 2 | DLG2_HUMAN |
| -1.43 | -2.34 | 24 | N_JN63 5406_ nucleocapsid | SGSGG | 184 | Sterol regulatory element-binding protein 1 | SRBP1_HUMAN |
| -1.43 | -2.21 | 24 | N_JN63 5406_ nucleocapsid | SGSGG | 184 | Zinc finger protein 746 | ZN746_HUMAN |
| -1.53 | -1.59 | 65 | N_JN63 5406_ nucleocapsid | DVSGP | 185 | Signal-induced proliferation-associated 1-like protein 2 | SI1L2_HUMAN |
| -1.60 | -1.83 | 108 | N_JN63 5406_ nucleocapsid | QSDQS | 186 | Signal-induced proliferation-associated 1-like protein 2 | SI1L2_HUMAN |
| -1.88 | -1.64 | 185 | N_JN63 5406_ nucleocapsid | TAPDT | 187 | Piezo-type mechanosensitive ion channel component 1 | PIEZ1_HUMAN |
| -1.55 | -1.10 | 427 | N_JN635406_nucleocapsid | SENEL | 188 | Oxidized low-density lipoprotein receptor 1 | OLR1_HUMAN |
| -1.19 | -1.23 | 469 | N_JN635406_nucleocapsid | LPTGT | 189 | DNA polymerase subunit gamma-1 | DPOG1 _HUMAN |
| -1.19 | -2.99 | 469 | N_JN635406_nucleocapsid | LPTGT | 189 | E3 ubiquitin-protein ligase SIAH1 | SIAH1_HUMAN |
| -2.20 | -2.37 | 511 | N_JN635406_nucleocapsid | GSDTD | 190 | CUB and sushi domain-containing protein 1 | CSMD1_HUMAN |
| -1.45 | -1.37 | 76 | P_JN635406_phosphoprotein | GAPRI | 191 | 60S ribosomal export protein NMD3 | NMD3_HUMAN |
| -1.44 | -1.01 | 80 | P_JN635406_phosphoprotein | IRGQG | 192 | Brain-derived neurotrophic factor | BDNF_HUMAN |
| -2.06 | -1.05 | 144 | P_JN635406_phosphoprotein | SGGDD | 193 | Sequestosome-1 | SQSTM_HUMAN |
| -1.79 | -1.07 | 216 | P_JN635406_phosphoprotein | LPPNP | 194 | Whirlin | WHRN_HUMAN |
| -2.06 | -1.64 | 220 | P_JN635406_phosphoprotein | PSRAS | 195 | High affinity cAMP-specific and IBMX-insensitive 3' | PDE8B_HUMAN |
| -2.12 | -1.78 | 224 | P_JN635406_phosphoprotein | STSET | 196 | Mortality factor 4-like protein 1 | MO4L1_HUMAN |
| -1.88 | -1.85 | 225 | P_JN635406_phosphoprotein | TSETP | 197 | Mortality factor 4-like protein 1 | MO4L1_HUMAN |
| -1.59 | -1.12 | 258 | P_JN635406_phosphoprotein | RKSPS | 198 | Histone-lysine N-methyltransferase ASH1L | ASH1L_HUMAN |
| -2.14 | -2.42 | 265 | P_JN635406_phosphoprotein | SGPGA | 199 | NADH dehydrogenase (ubiquinone) complex I | NDUF6_HUMAN |
| -2.39 | -1.17 | 267 | P_JN635406_phosphoprotein | PGAPA | 200 | Serine_threonine-protein kinase PINK1 | PINK1_HUMAN |
| -1.46 | -1.85 | 288 | P_JN635406_phosphoprotein | TPESG | 201 | Diacylglycerol kinase theta | DGKQ_HUMAN |
| -1.53 | -1.45 | 292 | P_JN635406_phosphoprotein | GTTIS | 202 | Lysosome membrane protein 2 | SCRB2_HUMAN |
| -1.64 | -1.29 | 296 | P_JN635406_phosphoprotein | SPRSQ | 203 | Serine protease HTRA2 | HTRA2_HUMAN |
| -2.03 | -1.36 | 427 | P_JN635406_phosphoprotein | GRTSS | 204 | Mitofusin-1 | MFN1_HUMAN |
| -1.45 | -1.37 | 76 | V_JN635406_V | GAPRI | 191 | 60S ribosomal export protein NMD3 | NMD3_HUMAN |
| -1.44 | -1.01 | 80 | V_JN635406_V | IRGQG | 192 | Brain-derived neurotrophic factor | BDNF_HUMAN |
| -2.06 | -1.05 | 144 | V_JN635406_V | SGGDD | 193 | Sequestosome-1 | SQSTM_HUMAN |
| -1.79 | -1.07 | 216 | V_JN635406_V | LPPNP | 194 | Whirlin | WHRN_HUMAN |
| -2.06 | -1.64 | 220 | V_JN635406_V | PSRAS | 195 | High affinity cAMP-specific and IBMX-insensitive 3' | PDE8B_HUMAN |
| -2.16 | -1.78 | 224 | V_JN635406_V | STSET | 196 | Mortality factor 4-like protein 1 | MO4L1_HUMAN |
| -1.91 | -1.85 | 225 | V_JN635406_V | TSETP | 197 | Mortality factor 4-like protein 1 | MO4L1_HUMAN |

**Table 21: High probability B cell epitopes in envelope glycoproteins of HSV1 (Kos) virus matching B cell epitopes in Parkinson's related proteins. In both query (HSV) and proteome protein the threshold applied was the top 15% probability B cell epitopes.**

| query curation | query pos | query penta | SEQ ID NO: | protein annotation (short) | UniProt ID | query BEPI | proteome SG15 JSb PredBEPI |
|---|---|---|---|---|---|---|---|
| glycoprotein_B | 36 | SPGTP | 205 | Microtubule-associated protein tau | TAU_HUMAN | -2.30 | -2.34 |
| glycoprotein_B | 37 | PGTPG | 206 | Microtubule-associated protein tau | TAU_HUMAN | -1.86 | -2.03 |
| glycoprotein_B | 62 | GAAPT | 207 | 85_88 kDa calcium-independent phospholipase A2 | PLPL9_HUMAN | -1.27 | -1.54 |
| glycoprotein_B | 68 | DPKPK | 208 | Mortality factor 4-like protein 1 | MO4L1_HUMAN | -1.93 | -1.32 |
| glycoprotein_B | 76 | KPKNP | 209 | Ubiquitin carboxyl-terminal hydrolase 25 | UBP25_HUMAN | -2.14 | -1.39 |
| glycoprotein_B | 85 | PAGDN | 210 | Transmembrane glycoprotein NMB | GPNMB_HUMAN | -1.26 | -1.50 |
| glycoprotein_B | 339 | TAPTT | 211 | Mitofusin-1 | MFN1_HUMAN | -1.41 | -2.29 |
| glycoprotein_B | 482 | TPPPP | 212 | Probable cation-transporting ATPase 13A2 | AT132_HUMAN | -2.40 | -2.97 |
| glycoprotein_C | 28 | SETAS | 213 | E3 ubiquitin-protein ligase RNF19A | RN19A_HUMAN | -1.61 | -1.11 |
| glycoprotein_C | 51 | SGSPG | 214 | Huntingtin-interacting protein 1-related protein | HIP1R_HUMAN | -2.18 | -2.85 |
| glycoprotein_C | 53 | SPGSA | 215 | GTP-binding protein Rit2 | RIT2_HUMAN | -2.01 | -2.12 |
| glycoprotein_C | 53 | SPGSA | 215 | Sterol regulatory element-binding protein 1 | SRBP1_HUMAN | -2.01 | -2.30 |
| glycoprotein_C | 57 | AASPE | 216 | Integrin alpha-8 | ITA8_HUMAN | -1.43 | -2.22 |
| glycoprotein_C | 83 | PASPP | 217 | Activated CDC42 kinase 1 | ACK1_HUMAN | -2.06 | -1.34 |
| glycoprotein_C | 83 | PASPP | 217 | Aminoacyl tRNA synthase complex-interacting multifunctional protein 2 | AIMP2_HUMAN | -2.06 | -2.03 |
| glycoprotein_C | 86 | PPTTP | 218 | Peroxisome proliferator-activated receptor gamma coactivator 1-alpha | PRGC1_HUMAN | -2.14 | -1.61 |
| glycoprotein_C | 98 | SPPTS | 219 | Tenascin-R | TENR_HUMAN | -2.45 | -2.85 |
| glycoprotein_C | 103 | TPDPK | 220 | Synaptojanin-1 | SYNJ1_HUMAN | -2.04 | -1.04 |
| glycoprotein_C | 105 | DPKPK | 208 | Mortality factor 4-like protein 1 | MO4L1_HUMAN | -1.84 | -1.32 |
| glycoprotein_C | 119 | RPTKP | 221 | Parkin coregulated gene protein | PACRG_HUMAN | -2.02 | -2.09 |
| glycoprotein_C | 211 | AGPGA | 222 | Serine_threonine-protein kinase PINK1 | PINK1_HUMAN | -1.84 | -1.52 |
| glycoprotein_C | 406 | DPSPA | 223 | Probable cation-transporting ATPase 13A2 | AT132_HUMAN | -1.98 | -2.00 |
| glycoprotein_C | 463 | QPPPR | 224 | Synaptojanin-1 | SYNJ1_HUMAN | -2.05 | -2.12 |
| glycoprotein_D | 288 | PNATQ | 225 | Lysosome-associated membrane glycoprotein 3 | LAMP3_HUMAN | -1.42 | -1.22 |
| glycoprotein_D | 290 | ATQPE | 226 | Probable cation-transporting ATPase 13A2 | AT132_HUMAN | -1.09 | -1.66 |
| glycoprotein_E | 102 | APPAP | 78 | Forkhead box protein L2 | FOXL2_HUMAN | -1.62 | -1.44 |
| glycoprotein_E | 103 | PPAPS | 227 | Eukaryotic translation initiation factor 4 gamma 1 | IF4G1_HUMAN | -1.83 | -2.01 |
| glycoprotein_E | 105 | APSAT | 228 | Eukaryotic translation initiation factor 4 gamma 1 | IF4G1_HUMAN | -1.88 | -1.60 |
| glycoprotein_E | 167 | PVPTP | 229 | Synaptojanin-1 | SYNJ1_HUMAN | -1.55 | -1.69 |
| glycoprotein_E | 202 | LPPPP | 230 | Activated CDC42 kinase 1 | ACK1_HUMAN | -1.62 | -1.41 |
| glycoprotein_E | 203 | PPPPA | 231 | Activated CDC42 kinase 1 | ACK1_HUMAN | -1.76 | -1.28 |
| glycoprotein_E | 204 | PPPAP | 232 | Eukaryotic translation initiation factor 4 gamma 1 | IF4G1_HUMAN | -1.74 | -2.07 |
| glycoprotein_E | 204 | PPPAP | 232 | Forkhead box protein L2 | FOXL2_HUMAN | -1.74 | -1.48 |
| glycoprotein_E | 204 | PPPAP | 232 | Transmembrane protein 175 | TM175_HUMAN | -1.74 | -2.37 |
| glycoprotein_E | 205 | PPAPP | 81 | Forkhead box protein L2 | FOXL2_HUMAN | -1.70 | -1.30 |
| glycoprotein_E | 205 | PPAPP | 81 | Zinc finger protein 746 | ZN746_HUMAN | -1.70 | -1.54 |
| glycoprotein_E | 455 | KSRAS | 233 | Synaptojanin-1 | SYNJ1_HUMAN | -1.27 | -1.30 |
| glycoprotein_E | 456 | SRASG | 234 | High affinity cAMP-specific and IBMX-insensitive 3' | PDE8B_HUMAN | -1.59 | -1.82 |
| glycoprotein_E | 458 | ASGKG | 235 | Iron-sulfur protein NUBPL | NUBPL_HUMAN | -1.82 | -1.09 |
| glycoprotein_E | 479 | SDSEG | 236 | PERQ amino acid-rich with GYF domain-containing protein 2 | PERQ2_HUMAN | -1.70 | -1.16 |
| glycoprotein_G | 42 | TGRPS | 237 | Matrix metalloproteinase-16 | MMP16_HUMAN | -1.33 | -1.58 |
| glycoprotein_G | 81 | EEEEE | 238 | Eukaryotic translation initiation factor 4 gamma 1 | IF4G1_HUMAN | -1.09 | -1.33 |
| glycoprotein_G | 81 | EEEEE | 238 | Piezo-type mechanosensitive ion channel component 1 | PIEZ1_HUMAN | -1.09 | -1.75 |
| glycoprotein_G | 82 | EEEEE | 238 | Eukaryotic translation initiation factor 4 gamma 1 | IF4G1_HUMAN | -1.38 | -1.33 |
| glycoprotein_G | 82 | EEEEE | 238 | Piezo-type mechanosensitive ion channel component 1 | PIEZ1_HUMAN | -1.38 | -1.75 |
| glycoprotein_G | 83 | EEEEE | 238 | Eukaryotic translation initiation factor 4 gamma 1 | IF4G1_HUMAN | -1.50 | -1.33 |
| glycoprotein_G | 83 | EEEEE | 238 | Piezo-type mechanosensitive ion channel component 1 | PIEZ1_HUMAN | -1.50 | -1.75 |
| glycoprotein_G | 84 | EEEEE | 238 | Eukaryotic translation initiation factor 4 gamma 1 | IF4G1_HUMAN | -1.67 | -1.33 |
| glycoprotein_G | 84 | EEEEE | 238 | Piezo-type mechanosensitive ion channel component 1 | PIEZ1_HUMAN | -1.67 | -1.75 |
| glycoprotein_G | 85 | EEEEG | 239 | Eukaryotic translation initiation factor 4 gamma 1 | IF4G1_HUMAN | -1.75 | -1.42 |
| glycoprotein_G | 85 | EEEEG | 239 | Piezo-type mechanosensitive ion channel component 1 | PIEZ1_HUMAN | -1.75 | -2.13 |
| glycoprotein_G | 109 | SPGPA | 240 | PERQ amino acid-rich with GYF domain-containing protein 2 | PERQ2_HUMAN | -1.26 | -1.30 |
| glycoprotein_G | 121 | EKDKP | 241 | Vacuolar protein sorting-associated protein 13C | VP13C_HUMAN | -1.81 | -2.31 |
| glycoprotein_G | 147 | PKTPP | 242 | Microtubule-associated protein tau | TAU_HUMAN | -1.88 | -1.70 |
| glycoprotein_G | 148 | KTPPT | 243 | Mimitin | MIMIT_HUMAN | -1.90 | -1.64 |
| glycoprotein_H | 135 | AQPPP | 244 | 85_88 kDa calcium-independent phospholipase A2 | PLPL9_HUMAN | -1.66 | -1.63 |
| glycoprotein_H | 136 | QPPPA | 245 | CUB and sushi domain-containing protein 1 | CSMD1_HUMAN | -1.48 | -2.16 |
| glycoprotein_H | 137 | PPPAV | 246 | CUB and sushi domain-containing protein 1 | CSMD1_HUMAN | -1.26 | -2.02 |
| glycoprotein_H | 194 | TPPPR | 247 | Probable cation-transporting ATPase 13A2 | AT132_HUMAN | -2.07 | -1.94 |
| glycoprotein_H | 195 | PPPRP | 248 | Activated CDC42 kinase 1 | ACK1_HUMAN | -1.96 | -2.09 |
| glycoprotein_H | 195 | PPPRP | 248 | Probable cation-transporting ATPase 13A2 | AT132_HUMAN | -1.96 | -2.13 |
| glycoprotein_H | 195 | PPPRP | 248 | Synaptojanin-1 | SYNJ1_HUMAN | -1.96 | -1.70 |
| glycoprotein_H | 195 | PPPRP | 248 | Transmembrane glycoprotein NMB | GPNMB_HUMAN | -1.96 | -2.30 |
| glycoprotein_H | 196 | PPRPP | 249 | Activated CDC42 kinase 1 | ACK1_HUMAN | -1.80 | -1.82 |
| glycoprotein_H | 196 | PPRPP | 249 | Histone-lysine N-methyltransferase ASH1L | ASH1L_HUMAN | -1.80 | -2.56 |
| glycoprotein_H | 196 | PPRPP | 249 | Matrix metalloproteinase-16 | MMP16_HUMAN | -1.80 | -2.56 |
| glycoprotein_H | 196 | PPRPP | 249 | Probable cation-transporting ATPase 13A2 | AT132_HUMAN | -1.80 | -2.24 |
| glycoprotein_H | 316 | PGGPR | 250 | Probable G-protein coupled receptor 37 | GPR37_HUMAN | -1.30 | -1.55 |
| glycoprotein_H | 348 | PEEGT | 251 | Activated CDC42 kinase 1 | ACK1_HUMAN | -1.26 | -1.01 |
| glycoprotein_H | 371 | GAEQG | 252 | Saitohin | STH_HUMAN | -1.80 | -1.49 |
| glycoprotein_H | 761 | AAGPT | 253 | Putative tyrosine-protein phosphatase auxilin | AUXI_HUMAN | -1.30 | -1.82 |
| glycoprotein_I | 238 | PKPQP | 254 | Putative tyrosine-protein phosphatase auxilin | AUXI_HUMAN | -1.82 | -1.58 |
| glycoprotein_I | 240 | PQPHG | 255 | Whirlin | WHRN_HUMAN | -1.24 | -1.06 |
| glycoprotein_I | 249 | PPSNA | 256 | Ubiquitin carboxyl-terminal hydrolase 25 | UBP25_HUMAN | -1.50 | -1.79 |
| glycoprotein_I | 336 | TPPKS | 257 | Microtubule-associated protein tau | TAU_HUMAN | -1.83 | -1.12 |
| glycoprotein_I | 366 | GLPTP | 258 | Neurobeachin-like protein 2 | NBEL2_HUMAN | -1.19 | -1.37 |
| glycoprotein_I | 367 | LPTPP | 259 | Neurobeachin-like protein 2 | NBEL2_HUMAN | -1.21 | -1.40 |
| glycoprotein_I | 367 | LPTPP | 259 | Zinc finger protein 746 | ZN746_HUMAN | -1.21 | -1.17 |
| glycoprotein_I | 368 | PTPPV | 260 | Neurobeachin-like protein 2 | NBEL2_HUMAN | -1.22 | -1.41 |
| glycoprotein_K | 275 | RGPAP | 261 | Neurobeachin-like protein 2 | NBEL2_HUMAN | -1.22 | -1.55 |
| glycoprotein_K | 285 | AAAPG | 262 | Diacylglycerol kinase theta | DGKQ_HUMAN | -1.97 | -1.41 |
| glycoprotein_K | 285 | AAAPG | 262 | Transmembrane glycoprotein NMB | GPNMB_HUMAN | -1.97 | -1.33 |
| glycoprotein_K | 286 | AAPGR | 263 | Neurobeachin-like protein 2 | NBEL2_HUMAN | -1.97 | -1.25 |
| glycoprotein_K | 286 | AAPGR | 263 | Probable G-protein coupled receptor 37 | GPR37_HUMAN | -1.97 | -1.42 |
| glycoprotein_M | 392 | GSPPG | 264 | Sterol regulatory element-binding protein 1 | SRBP1_HUMAN | -1.88 | -2.05 |
| glycoprotein_M | 415 | RYGDS | 265 | CUB and sushi domain-containing protein 1 | CSMD1_HUMAN | -1.04 | -1.59 |
| glycoprotein_M | 418 | DSDGE | 266 | Transmembrane glycoprotein NMB | GPNMB_HUMAN | -1.23 | -2.03 |
| glycoprotein_N | 29 | PHGEP | 267 | Septin-5 | SEPT5_HUMAN | -1.86 | -1.70 |
| glycoprotein_N | 33 | PPGEE | 268 | Activated CDC42 kinase 1 | ACK1_HUMAN | -1.95 | -1.85 |

It will be evident to those skilled in the art that a list or proteins associated with other disease syndromes, particularly those of unknown or complex etiology, could be compiled and a similar analytical approach used to identify potential epitope mimics and autoimmune associations. Thus, the example of Parkinson's disease is not considered limiting.

### Reference List

1. M. P. Lefranc et al., IMGT, the international ImMunoGeneTics information system. Nucleic acids research 37, D1006-1012 (2009).
2. F. A. Rey, F. X. Heinz, C. Mandl, C. Kunz, S. C. Harrison, The envelope glycoprotein from tick-borne encephalitis virus at 2 A resolution. Nature 375, 291-298 (1995).
3. V. C. Luca, J. AbiMansour, C. A. Nelson, D. H. Fremont, Crystal structure of the Japanese encephalitis virus envelope protein. Journal of virology 86, 2337-2346 (2012).
4. D. Gubler, Kuno G., Markoff L., in Field's Virology, D. Knipe, Howley, PM, Ed. (Lippincott, Williams and Wilkins, Philadelphia, PA, 2007), vol. 2, pp. 1153-1252.
5. R. D. Bremel, J. Homan, Extensive T-cell epitope repertoire sharing among human proteome, gastrointestinal microbiome, and pathogenic bacteria: Implications for the definition of self. Frontiers in immunology 6, (2015).
6. R. D. Bremel, E. J. Homan, Recognition of higher order patterns in proteins: immunologic kernels. PloS one 8, e70115 (2013).
7. S. Weiss, B. Bogen, B-lymphoma cells process and present their endogenous immunoglobulin to major histocompatibility complex-restricted T cells. Proc Natl Acad Sci U S A 86, 282-286 (1989).
8. B. Bogen, S. Weiss, Processing and presentation of idiotypes to MHC-Restricted T cells. International Reviews Immunology 10, 337-355 (1993).
9. M. Greco, P. Cofano, G. Lobreglio, Seropositivity for West Nile Virus Antibodies in Patients Affected by Myasthenia Gravis. J Clin Med Res 8, 196-201 (2016).
10. S. Bhattacharya et al., Public health. The cholera crisis in Africa. Science 324, 885 (2009).
11. Y. C. Chuang, Y. S. Lin, H. S. Liu, T. M. Yeh, Molecular mimicry between dengue virus and coagulation factors induces antibodies to inhibit thrombin activity and enhance fibrinolysis. Journal of virology 88, 13759-13768 (2014).
12. P. Fan et al., Identification of a common epitope between enterovirus 71 and human MED25 proteins which may explain virus-associated neurological disease. Viruses 7, 1558-1577 (2015).
13. A. Loshaj-Shala et al., Guillain Barre syndrome (GBS): new insights in the molecular mimicry between C. jejuni and human peripheral nerve (HPN) proteins. Journal of neuroimmunology 289, 168-176 (2015).
14. V. Phongsisay, The immunobiology of Campylobacter jejuni: Innate immunity and autoimmune diseases. Immunobiology 221, 535-543 (2016).
15. T. T. Kuo et al., Neonatal Fc receptor: from immunity to therapeutics. Journal of clinical immunology 30, 777-789 (2010).
16. C. Kowal, A. Athanassiou, H. Chen, B. Diamond, Maternal antibodies and developing blood-brain barrier. Immunologic research 63, 18-25 (2015).
17. B. Diamond, P. T. Huerta, P. Mina-Osorio, C. Kowal, B. T. Volpe, Losing your nerves? Maybe it's the antibodies. Nature reviews. Immunology 9, 449-456 (2009).
18. N. R. Saunders, S. A. Liddelow, K. M. Dziegielewska, Barrier mechanisms in the developing brain. Frontiers in pharmacology 3, 46 (2012).
19. E. Fox, D. Amaral, J. Van de Water, Maternal and fetal antibrain antibodies in development and disease. Developmental neurobiology 72, 1327-1334 (2012).
20. E. Fox-Edmiston, J. Van de Water, Maternal Anti-Fetal Brain IgG Autoantibodies and Autism Spectrum Disorder: Current Knowledge and its Implications for Potential Therapeutics. CNS drugs 29, 715-724 (2015).
21. C. Perret et al., Dengue infection during pregnancy and transplacental antibody transfer in Thai mothers. The Journal of infection 51, 287-293 (2005).
22. R. C. Leite et al., Dengue infection in pregnancy and transplacental transfer of anti-dengue antibodies in Northeast, Brazil. Journal of clinical virology : the official publication of the Pan American Society for Clinical Virology 60, 16-21 (2014).
23. M. C. Cheeran, J. R. Lokensgard, M. R. Schleiss, Neuropathogenesis of congenital cytomegalovirus infection: disease mechanisms and prospects for intervention. Clinical microbiology reviews 22, 99-126, Table of Contents (2009).
24. A. E. Barskey, J. W. Glasser, C. W. LeBaron, Mumps resurgences in the United States: A historical perspective on unexpected elements. Vaccine 27, 6186-6195 (2009).
25. M. Clagett-Dame, E. M. McNeill, P. D. Muley, Role of all-trans retinoic acid in neurite outgrowth and axonal elongation. Journal of neurobiology 66, 739-756 (2006).
26. E. M. McNeill, K. P. Roos, D. Moechars, M. Clagett-Dame, Nav2 is necessary for cranial nerve development and blood pressure regulation. Neural development 5, 6 (2010).
27. S. B. Boppana, K. B. Fowler, W. J. Britt, S. Stagno, R. F. Pass, Symptomatic congenital cytomegalovirus infection in infants born to mothers with preexisting immunity to cytomegalovirus. Pediatrics 104, 55-60 (1999).
28. S. B. Boppana, J. Miller, W. J. Britt, Transplacentally acquired antiviral antibodies and outcome in congenital human cytomegalovirus infection. Viral immunology 9, 211-218 (1996).
29. S. B. Boppana, R. F. Pass, W. J. Britt, Virus-specific antibody responses in mothers and their newborn infants with asymptomatic congenital cytomegalovirus infections. J Infect Dis 167, 72-77 (1993).
30. C. UniProt, UniProt: a hub for protein information. Nucleic acids research 43, D204-212 (2015).
31. G. Robin et al., Restricted diversity of antigen binding residues of antibodies revealed by computational alanine scanning of 227 antibody-antigen complexes. J Mol Biol 426, 3729-3743 (2014).
32. S. A. Rubin, M. A. Afzal, Neurovirulence safety testing of mumps vaccines--historical perspective and current status. Vaccine 29, 2850-2855 (2011).
33. S. A. Rubin et al., Changes in mumps virus gene sequence associated with variability in neurovirulent phenotype. Journal of virology 77, 11616-11624 (2003).
34. G. Amexis, S. Rubin, N. Chatterjee, K. Carbone, K. Chumakov, Identification of a new genotype H wild-type mumps virus strain and its molecular relatedness to other virulent and attenuated strains. Journal of medical virology 70, 284-286 (2003).
35. S. B. Halstead, Dengue Antibody-Dependent Enhancement: Knowns and Unknowns. Microbiology spectrum 2, (2014).
36. A. K. Falconar, The dengue virus nonstructural-1 protein (NS1) generates antibodies to common epitopes on human blood clotting, integrin/adhesin proteins and binds to human endothelial cells: potential implications in haemorrhagic fever pathogenesis. Arch.Virol. 142, 897-916 (1997).
37. K. Djamiatun et al., Severe dengue is associated with consumption of von Willebrand factor and its cleaving enzyme ADAMTS-13. PLoS neglected tropical diseases 6, e1628 (2012).
38. Y. C. Chuang, J. Lin, Y. S. Lin, S. Wang, T. M. Yeh, Dengue Virus Nonstructural Protein 1-Induced Antibodies Cross-React with Human Plasminogen and Enhance Its Activation. J Immunol 196, 1218-1226 (2016).
39. H. J. Cheng et al., Correlation between serum levels of anti-endothelial cell autoantigen and anti-dengue virus nonstructural protein 1 antibodies in dengue patients. The American journal of tropical medicine and hygiene 92, 989-995 (2015).
40. P. R. Beatty et al., Dengue virus NS1 triggers endothelial permeability and vascular leak that is prevented by NS1 vaccination. Science translational medicine 7, 304ra141 (2015).
41. H. Puerta-Guardo, D. R. Glasner, E. Harris, Dengue Virus NS1 Disrupts the Endothelial Glycocalyx, Leading to Hyperpermeability. PLoS pathogens 12, e1005738 (2016).
42. S. J. Thomas, NS1: A corner piece in the dengue pathogenesis puzzle? Science translational medicine 7, 304fs337 (2015).
43. O. Karimi et al., Thrombocytopenia and subcutaneous bleedings in a patient with Zika virus infection. Lancet, (2016).
44. T. M. Sharp et al., Zika Virus Infection Associated with Severe Thrombocytopenia. Clinical infectious diseases : an official publication of the Infectious Diseases Society of America, (2016).
45. M. A. Edeling, M. S. Diamond, D. H. Fremont, Structural basis of Flavivirus NS1 assembly and antibody recognition. Proc Natl Acad Sci U S A 111, 4285-4290 (2014).
46. H. J. Rogers, C. Allen, A. E. Lichtin, Thrombotic thrombocytopenic purpura: The role of ADAMTS13. Cleveland Clinic journal of medicine 83, 597-603 (2016).
47. X. L. Zheng, ADAMTS13 and von Willebrand factor in thrombotic thrombocytopenic purpura. Annu Rev Med 66, 211-225 (2015).
48. D. B. Cines, V. S. Blanchette, Immune thrombocytopenic purpura. The New England journal of medicine 346, 995-1008 (2002).

## Claims

1. A method for identifying B-cell epitopes in a protein of interest that mimic B-cell epitopes in a host proteome, comprising:
assembling a database of all proteins in the host proteome;
computing the probable B cell epitopes in each protein of said host proteome database;
identifying the core pentamers of said probable B cell epitopes in each protein of the host proteome;
assembling a database of said core pentamers of said probable B cell epitopes from each protein of the host proteome in a computer readable medium;
entering a sequence of a protein of interest into a computer with access to said database;
computing probable B cell epitopes in the protein of interest;
identifying the core pentamers of said probable B cell epitopes in said protein of interest;
identifying probable B cell epitopes in the protein of interest which are adjacent to a peptide with predicted high binding affinity for one or more MHC II molecule;
comparing said core pentamers of said probable B cell epitope in a protein of interest to the core pentamers contained in said database of peptides from the host proteome;
identifying those probable B cell epitopes in proteins of the host proteome which comprise a core pentamer identical to the core pentamers of a probable B cell epitope in the protein of interest that is adjacent to a peptide of predicted high binding affinity for one or more MHC II molecules in the protein of interest wherein said peptide with predicted high binding affinity to one or more MHC II molecules is within about 20 amino acids of said probable B cell epitope.

2. A method of producing a mutated biopharmaceutical protein comprising:
obtaining one or more gene or amino acid sequences encoding a biopharmaceutical protein of interest that has been mutated to remove one or more epitope mimics or alter one or more epitope mimics to non-mimics as compared to corresponding target biopharmaceutical protein; and
synthesizing the mutated biopharmaceutical protein by expressing the biopharmaceutical that has been mutated to remove one or more B-cell epitope mimics or alter one or more B-cell epitope mimics to non-mimics as compared to the corresponding target biopharmaceutical protein;
wherein the one or more epitope mimics are identified by a process comprising the steps of:
computing probable B cell epitopes in each protein of the human proteome in a human proteome database wherein the proteins of the human proteome are curated by function;
identifying the core pentamer of said probable B cell epitopes in each protein of human proteome, wherein said core pentamers are the central 5 amino acid peptide in a predicted B cell epitope;
assembling a database of the core pentamers of said probable B cell epitopes from each protein of the human proteome in a computer readable medium;
entering sequences encoding the target biopharmaceutical protein into a computer with access to said database;
computing probable B cell epitopes in the sequences encoding the target biopharmaceutical protein;
identifying the core pentamer of said probable B cell epitopes in the sequences encoding the target biopharmaceutical protein;
identifying those probable B cell epitopes in the target biopharmaceutical protein which are located within 10 to 20 amino acids of a peptide with predicted high binding affinity for one or more MHC II molecules;
comparing said core pentamer of said probable B cell epitope in the sequences encoding the target biopharmaceutical protein to the core pentamers contained in said database of peptides from the human proteome;
identifying those predicted B cell epitopes in one or more proteins of the human proteome which comprise a core pentamer identical to the core pentamer of a probable B cell epitope that is located within 10 to 20 amino acids of a peptide of predicted high binding affinity for one or more MHC II molecules in that target biopharmaceutical protein..

3. A method of producing a vaccine comprising one or more components that have been mutated, said method comprising:
obtaining one or more gene or amino acid sequences encoding one or more components of vaccine that have been mutated to remove one or more epitope mimics or alter one or more epitope mimics to non-mimics as compared to the corresponding wild type sequences; and
synthesizing components for a vaccine by a method selected from the group consisting of a) expressing the one more sequences encoding one or more components of vaccine that have been mutated to remove one or more epitope mimics or alter one or more epitope mimics to non-mimics as compared to the corresponding wild type sequences in a host cell to produce mutated proteins, and b) synthesizing nucleic acid segments encoding the one or more recombinant sequences encoding one or more components of vaccine that have been mutated to remove one or more epitope mimics or alter one or more epitope mimics to non-mimics as compared to the corresponding wild type sequences of the one or more components of the vaccine;
wherein the one or more epitope mimics are identified by a process comprising the steps of:
computing probable B cell epitopes in each protein of the human proteome in a human proteome database wherein the proteins of the human proteome are curated by function;
identifying the core pentamer of said probable B cell epitopes in each protein of human proteome, wherein said core pentamers are the central 5 amino acid peptide in a predicted B cell epitope;
assembling a database of the core pentamers of said probable B cell epitopes from each protein of the human proteome in a computer readable medium;
entering sequences encoding one or more components of vaccine into a computer with access to said database;
computing probable B cell epitopes in the sequences encoding the one or more components of vaccine;
identifying the core pentamer of said probable B cell epitopes in the sequences encoding one or more components of vaccine;
identifying those probable B cell epitopes in the one or more components of vaccine which are located within 10 to 20 amino acids of a peptide with predicted high binding affinity for one or more MHC II molecules;
comparing said core pentamer of said probable B cell epitope in the sequences encoding the one or more components of vaccine to the core pentamers contained in said database of peptides from the human proteome;
identifying those predicted B cell epitopes in one or more proteins of the human proteome which comprise a core pentamer identical to the core pentamer of a probable B cell epitope that is located within 10 to 20 amino acids of a peptide of predicted high binding affinity for one or more MHC II molecules in that one or more components of vaccine.

4. The method of any of claims 1 or 3, wherein the probable B cell epitope in said protein of interest is in the top 25% most probable B cell epitopes in the protein of interest, mutated biopharmaceutical protein or vaccine.

5. The method of any of claims 1-4, wherein said probable B cell epitope in said protein of interest is in the top 10% most probable B cell epitopes in the protein of interest, mutated biopharmaceutical protein or vaccine.

6. The method of any one of claims 1-5, further comprising identifying which MHC II alleles are predicted to be have high binding affinity to a peptide adjacent to the B cell epitope in the protein of interest, mutated biopharmaceutical protein or vaccine.

7. The method of claim 6, further comprising identifying a subpopulation of host subjects which carry the MHC II alleles identified in claim 6 and thus which subpopulation is most at risk of adverse effects arising from antibodies elicited by the mutated biopharmaceutical protein or vaccine also binding to the matching B cell epitope in the host proteome.

8. The method of any of claims 2-6, wherein the mutated biopharmaceutical protein or vaccine is an antigen binding protein.

9. The method of any of claims 2 and 4-7, wherein the mutated biopharmaceutical protein is a microbial protein, preferably wherein said microbial protein is selected from the group consisting of a virus protein, a bacterial protein, a parasite protein, a fungus protein, and a microbial toxin.

10. The method of any of claims 3 - 7, wherein the sequences encoding one or more components of vaccine are microbial protein sequences, preferably wherein said microbial protein sequences are selected from the group consisting of virus, bacteria, parasite, fungus, and microbial toxin sequences.

11. The method of any of claims 2 - 10, further comprising the step of synthesizing a mutant version of the mutated biopharmaceutical protein or vaccine, wherein said core pentamer in a B cell epitope in said protein of interest is mutated to abrogate the match to a core pentamer in a B cell epitope in the human proteome.

12. The mutated biopharmaceutical protein or vaccine obtained by the method according to any one of the claims 2 - 11 for use in the treatment of an antibody mediated autoimmune disease.
